(19) Europäisches Patentamt
European Patent Office
Office européen des brevets

(11) EP 4 269 404 A1

(12) **EUROPEAN PATENT APPLICATION**
published in accordance with Art. 153(4) EPC

(43) Date of publication:
**01.11.2023 Bulletin 2023/44**

(21) Application number: **21909386.1**

(22) Date of filing: **21.12.2021**

(51) International Patent Classification (IPC):
***C07D 401/14*** (2006.01) ***C07D 401/04*** (2006.01)
***C07D 403/14*** (2006.01) ***C07D 403/04*** (2006.01)
***A61K 31/4439*** (2006.01) ***A61K 31/506*** (2006.01)
***A61P 1/16*** (2006.01)

(52) Cooperative Patent Classification (CPC):
**A61K 31/4439; A61K 31/506; A61P 1/16; C07D 401/04; C07D 401/14; C07D 403/04; C07D 403/14**

(86) International application number:
**PCT/CN2021/140067**

(87) International publication number:
**WO 2022/135390 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 CN 202011565850**
**07.02.2021 CN 202110166978**
**19.03.2021 CN 202110296088**
**11.05.2021 CN 202110509335**
**11.06.2021 CN 202110655424**

(71) Applicant: **Sichuan Haisco Pharmaceutical Co., Ltd.**
**Sichuan 611130 (CN)**

(72) Inventors:
- **LI, Yao**
  **Chengdu, Sichuan 611130 (CN)**
- **WANG, Wenjing**
  **Chengdu, Sichuan 611130 (CN)**
- **CHEN, Lei**
  **Chengdu, Sichuan 611130 (CN)**
- **ZHANG, Guobiao**
  **Chengdu, Sichuan 611130 (CN)**
- **ZHANG, Xiaobo**
  **Chengdu, Sichuan 611130 (CN)**
- **HU, Gang**
  **Chengdu, Sichuan 611130 (CN)**
- **WANG, Yajun**
  **Chengdu, Sichuan 611130 (CN)**
- **WANG, Haodong**
  **Chengdu, Sichuan 611130 (CN)**
- **TANG, Pingming**
  **Chengdu, Sichuan 611130 (CN)**
- **YU, Yan**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **Sonnenhauser, Thomas Martin**
**Wuesthoff & Wuesthoff**
**Patentanwälte PartG mbB**
**Schweigerstraße 2**
**81541 München (DE)**

# (54) KETOHEXOKINASE INHIBITOR AND USE THEREOF

(57) Provided are a compound of formula (I), a stereoisomer, a deuterated compound, a solvate, a prodrug, a metabolite, a pharmaceutically acceptable salt or a eutectic thereof, or a pharmaceutical composition comprising same, and use thereof as a ketohexokinase inhibitor in the preparation of drugs for treating related diseases. Each group in formula (I) is as defined in the description.

EP 4 269 404 A1

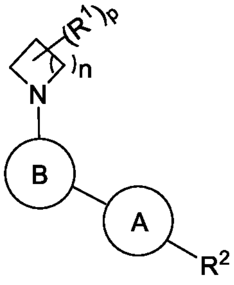

(I)

## Description

### Technical Field

The present invention belongs to the field of drugs, and in particular relates to a derivative of a ketohexokinase inhibitor, or a stereoisomer, a pharmaceutically acceptable salt, a solvate, a eutectic or a deuterated compound thereof, and the use thereof in the preparation of a drug for treating related diseases mediated by ketohexokinase.

### Background Art

Ketohexokinase (KHK) is a basic enzyme involved in fructose metabolism in the body, plays a very important role in fructose metabolism, and catalyses the reaction of fructose with ATP to form fructose-1-phosphate (F1P). Ketohexokinase has two main subtypes in humans, ketohexokinase A (KHKa) and ketohexokinase C (KHKc). KHKa is expressed more widely in the body; however, KHKc is expressed more highly in major metabolic organs (such as liver, kidney and intestines) of the human body (Ishimoto, Lanaspa et al. PNAS 109, 4320-4325, 2012), and therefore KHKc has a more significant role in regulating fructose metabolism. Epidemiological studies have shown that dietary sugar consumption has a clear correlation with the incidence of metabolic syndrome and obesity. Experiments have shown that administration of fructose to rats can induce metabolic syndrome, weight gain and increased body fat characteristics.

Metabolic syndrome and obesity seriously affect people's quality of life. The data released by the WHO showed that the number of obese people around the world has almost tripled since 1975. In 2016, more than 1.9 billion adults over the age of 18 were overweight, of which more than 650 million were obese (https://www.who.int/en/news-room/fact-sheets/detail/obesityand-overweight). Obesity is also called "four major killers" together with hypertension, hyperlipidaemia and hyperglycaemia, and may become the first killer in the 21st century. At least 2.8 million people die each year from being overweight or obese.

Diabetes is one of metabolic syndromes with a wide distribution of patients, and it is estimated that 463 million people between the ages of 20 and 79 suffered from diabetes worldwide, the vast majority of whom are type 2 diabetes patients (IDF Diabetes Atlas Ninth Edition (9th edition) 2019 released by the International Diabetes Federation). Although there are many drugs for treating diabetes available in the market, they still have not met the clinical needs.

Metabolic dysfunction-associated fatty liver disease (MAFLD) has received extensive attention in recent years with a global incidence of about 25%, and further development of MAFLD will cause inflammation, which may further deteriorate and lead to liver fibrosis, or even liver cancer. At present, metabolic dysfunction-associated fatty liver disease has become an increasingly common chronic liver disease worldwide, and is currently the first leading cause of liver transplantation in the United States. Unfortunately, there is currently no drug officially approved for metabolic dysfunction-associated fatty liver disease, and therefore, great clinical needs cannot be met.

Ketohexokinase (KHK) is the basic enzyme in fructose metabolism and catalyses the conversion of fructose to fructose-1-phosphate (F1P). KHK is expressed as two alternative mRNA splice variants (denoted KHKa and KHKc) resulting from alternative splicing of the third exon. The affinity and capacity of KHKc for fructose phosphorylation is much greater than KHKa as evidenced by a much lower Km (Ishimoto, Lanaspa et al., PNAS 109, 4320-4325, 2012). Although KHKa is widely expressed, the expression of KHKc is highest in the liver, kidney and intestines, the primary sites of fructose metabolism in the body (Diggle CP et al. (2009) J HistochemCytochem 57: 763-774; Ishimoto, Lanaspa et al., PNAS 109, 4320-4325, 2012). Additionally, loss of function mutations has been reported in humans with no adverse effects except the appearance of fructose in the urine after ingestion of the sugar.

A more severe condition involved in fructose metabolism is hereditary fructose intolerance (HFI, OMIM#229600) which is caused by defects in aldolase B (GENE: ALDOB), and the aldolase B is the enzyme responsible for breaking down F1P and is immediately downstream of the KHK step in the pathway (Bouteldja N et al., J. Inherit. Metab. Dis. Apr. 2010; 33(2): 105-12; Tolan, DR, HumMutat. 1995; 6(3): 210-8; http://www.omim.org/entry/229600). It is a rare disorder which affects an estimated 1 in 20,000 people, and mutations result in accumulation of F1P, depletion of ATP and increase in uric acid, the combination of which causes hypoglycaemia, hyperuricaemia and lactic acidosis, as well as other metabolic disorders. HFI impairs the body's ability to metabolize dietary fructose, thereby resulting in acute symptoms such as vomiting, severe hypoglycaemia, diarrhoea and abdominal distress, then leading to long term growth defects, liver and kidney damage and potentially death (Ali M et al., J.Med.Genet. May 1998: 35(5): 353-65). Patients generally suffer through a year prior to diagnosis, and the only course of treatment is avoiding fructose in the diet, which is made challenging by the presence of this macronutrient in a majority of foods. In addition to physical symptoms, many patients experience emotional and social isolation as a consequence of their unusual diet, and constantly struggle to adhere to strict dietary limitations (HFI-INFO Discussion Board, http://hfiinfo.proboards.com. Accessed 14 Dec. 2015). Even when they appear non-symptomatic, some patients develop NAFLD and kidney disease, which underscores the inadequacy of self-imposed dietary restriction as the only treatment option, and the high unmet medical need for this condition.

In hyperglycaemic conditions, endogenous fructose production occurs through the polyol pathway, a pathway by which glucose is converted to fructose with sorbitol as an intermediate. The activity of this pathway increases with hyperglycaemia. In these studies, the authors demonstrated that the KHK-null mice were protected from glucose-induced weight gain, insulin resistance and hepatic steatosis, suggesting that under hyperglycaemic conditions, endogenously produced fructose may contribute to insulin resistance and hepatic steatosis (Lanaspa, M.A., et al., NatureComm. 4, 2434, 2013). Therefore, the inhibition of KHK is anticipated to benefit many diseases where alterations of endogenous fructose and/or ingested fructose are involved.

As for compounds of ketohexokinase inhibitors, the Johnson & Johnson has published the utility of pyrimidopyrimidine compounds in inhibiting ketohexokinase activity (ACS Med. Chem. Lett. 2011, 2, 538-543). WO 2017/115205 discloses a compound that can be used as a ketohexokinase inhibitor and discloses the use of the compound in the treatment of obesity, type II diabetes, metabolic dysfunction-associated fatty liver disease, etc. However, there has been no KHK inhibitor available in the market so far, and therefore there is still an unmet clinical need for KHK inhibitors with high inhibitory activity and low toxicity.

## Summary of the Invention

The present invention aims to provide a KHK inhibitor compound, which has a good inhibitory activity against KHK, excellent pharmacokinetic parameters and high bioavailability, basically has no inhibition effect on hERG potassium channel and CYP3A4 enzyme, has low toxic and side effects and has the potential of druggability.

Embodiment one of the present invention provides a compound of formula (I), or a stereoisomer, a deuterated compound, a solvate, a prodrug, a metabolite, a pharmaceutically acceptable salt or a eutectic thereof,

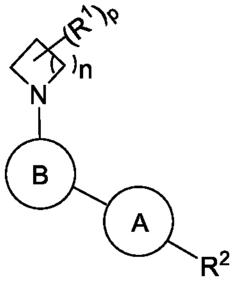
(I)

wherein

each $R^1$ is independently selected from deuterium, $C_{1-6}$ alkyl, $C_{2-6}$ alkenyl, $C_{2-6}$ alkynyl, hydroxyl, halogen, amino, nitro, cyano, carboxyl, $C_{1-6}$ alkoxy, $C_{1-6}$ alkylamino, and di($C_{1-6}$ alkyl)amino, wherein the alkyl and alkoxy are optionally substituted with 1 to 5 groups selected from halogen, deuterium, hydroxyl, amino, cyano, and $C_{1-6}$ alkoxy;
p is an integer selected from 1-8;
n is selected from 1, 2, or 3;
ring B is selected from groups B1, B2, B3, B4, B5, B6, and B7, or ring B is selected from B8 and B9, wherein # represents a connection site of ring B to ring A;

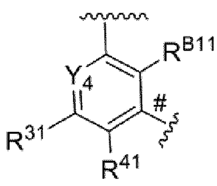 , 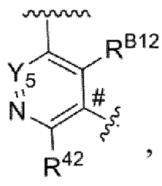 , 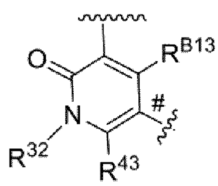 ,

B1 B2 B3

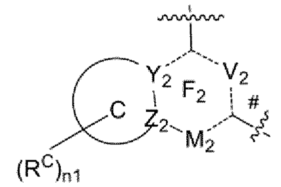,
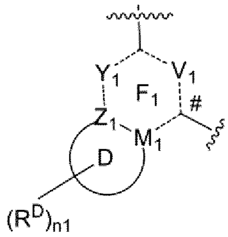,
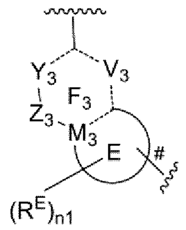,
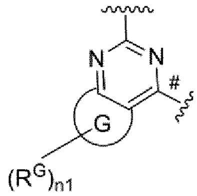

B4 B5 B6 B7

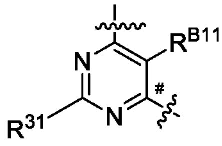
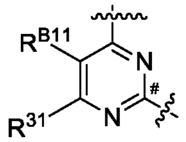

B8 B9 ;

----- represents a double bond or a single bond;

rings $F_1$, $F_2$, and $F_3$ are aryl or heteroaryl;

ring C is selected from 3-12-membered cycloalkyl, 3-14-membered heterocycloalkyl, 6-12-membered aryl, or 5-12-membered heteroaryl;

ring D is selected from 3-12-membered cycloalkyl, 3-14-membered heterocycloalkyl, 6-12-membered aryl, or 5-12-membered heteroaryl;

ring E is selected from 3-12-membered cycloalkyl, 3-14-membered heterocycloalkyl, 6-12-membered aryl, or 5-12-membered heteroaryl;

ring G is selected from 6-12-membered aryl, 5-12-membered heteroaryl, 3-14-membered heterocycloalkyl, or 3-12-membered cycloalkyl;

$Y_4$ and $Y_5$ are each independently selected from $-CR^{54}-$ or -N-;

$V_1$ and $Y_1$ are each independently selected from $-CR^{51}-$ or -N-;

$Z_1$ and $M_1$ are each independently selected from -C-, $-CR^{51}-$ or -N-;

$V_2$ and $M_2$ are each independently selected from $-CR^{52}-$ or -N-;

$Y_2$ and $Z_2$ are each independently selected from -C-, $-CR^{52}-$ or -N-;

$V_3$, $Y_3$, and $Z_3$ are each independently selected from $-CR^{53}-$ or -N-;

each $M_3$ is independently selected from -C-, $-CR^{53}-$, or -N-;

provided that when $Z_1$ and $M_1$ are both selected from -C-, $V_1$ and $Y_1$ are not simultaneously -N-;

n1 is selected from 0, 1, 2, and 3;

$R^C$, $R^D$, $R^E$, $R^G$, and $R^{B11}$ are each independently selected from deuterium, halogen, nitro, cyano, amino, hydroxyl, =O, $-SF_5$, di($C_{1-6}$ alkyl)phosphonyl, $C_{1-6}$ alkyl, $C_{1-6}$ alkoxy, $C_{2-6}$ alkenyl, $C_{2-6}$ alkynyl, $-S-C_{1-6}$ alkyl, $-S(O)-C_{1-6}$ alkyl, $-S(O)_2-C_{1-6}$ alkyl, $-(CH_2)_r-C_{3-12}$ cycloalkyl, $-(CH_2)_r-C_{3-12}$ heterocycloalkyl, $-O-C_{3-12}$ cycloalkyl, $-O-C_{3-12}$ heterocycloalkyl, $-NH-C_{3-12}$ cycloalkyl, $-NH-C_{3-12}$ heterocycloalkyl, $-S-C_{3-12}$ cycloalkyl, $-S-C_{3-12}$ heterocycloalkyl, 5- to 12-membered heteroaryl, 6- to 12-membered aryl, $-NHC_{1-6}$ alkyl, $-N(C_{1-6}\ alkyl)_2$, $-C(=O)NR^{31a}R^{41a}$, $-NR^{31a}C(=O)-R^{41a}$, $-NR^{31a}R^{41a}$, and $-C(=O)-R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-6}$ alkyl, $C_{1-6}$ alkoxy, halo $C_{1-6}$ alkyl, halo $C_{1-6}$ alkoxy, deuterated $C_{1-6}$ alkyl, and deuterated $C_{1-6}$ alkoxy;

$R^{B12}$, $R^{B13}$, $R^{31}$, $R^{32}$, $R^{41}$, $R^{42}$, $R^{43}$, $R^{51}$, $R^{52}$, $R^{53}$, and $R^{54}$ are each independently selected from hydrogen, deuterium, halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $-S-C_{1-6}$ alkyl, $-S(O)-C_{1-6}$ alkyl, $-S(O)_2-C_{1-6}$ alkyl, $-(CH_2)_r-C_{3-12}$ cycloalkyl, $-(CH_2)_r-C_{3-12}$ heterocycloalkyl, $-O-C_{3-12}$ cycloalkyl, $-O-C_{3-12}$ heterocycloalkyl, $-NH-C_{3-12}$ cycloalkyl, $-NH-C_{3-12}$ heterocycloalkyl, $-S-C_{3-12}$ cycloalkyl, $-S-C_{3-12}$ heterocycloalkyl, 5- to 12-membered heteroaryl, 6- to 12-membered aryl, $-NHC_{1-6}$ alkyl, $-N(C_{1-6}\ alkyl)_2$, $-C(=O)NR^{31a}R^{41a}$, $-NR^{31a}C(=O)-R^{41a}$, $-NR^{31a}R^{41a}$, and $-C(=O)-R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-6}$ alkyl, $C_{1-6}$ alkoxy, halo $C_{1-6}$ alkyl, halo $C_{1-6}$ alkoxy, deuterated $C_{1-6}$ alkyl, and deuterated $C_{1-6}$ alkoxy;

each r is independently selected from 0, 1, 2, 3, or 4;

$R^{31a}$ and $R^{41a}$ are each independently substituted with a group selected from hydrogen, halogen, deuterium, nitro, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, and deuterated

$C_{1-6}$ alkoxy;
ring A is selected from the following groups, wherein * represents a connection site of ring A to $R^2$:

(1) 4-7-membered monocyclic heterocycloalkyl and 4-7-membered monocyclic cycloalkyl;
(2) a 5-12-membered spiro ring;
(3)

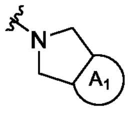, 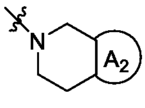, or 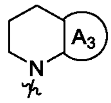,

wherein the connection site of ring A to $R_2$ is $A_1$, $A_2$, or $A_3$ ring atom;
(4)

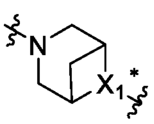, 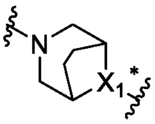, 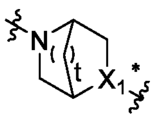, 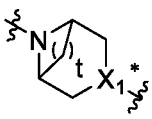, or 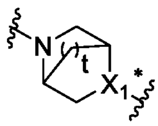;

(5)

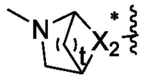 or 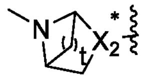;

(6)

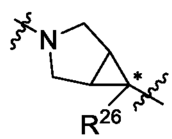 or 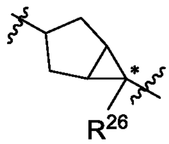;

(7) 7-12-membered aryl;
(8) 5-12-membered heteroaryl;

the ring A is optionally further substituted with 1 to 5 $R^A$;
each $R^A$ is independently selected from deuterium, halogen, cyano, hydroxyl, amino, =O, $C_{1-6}$ alkyl, $C_{1-6}$ alkoxy, halo $C_{1-6}$ alkyl, halo $C_{1-6}$ alkoxy, deuterated $C_{1-6}$ alkyl, and deuterated $C_{1-6}$ alkoxy, or two $R^A$ on the same atom together form 3-5-membered monocyclic alkyl;
each t is independently selected from 1, 2, and 3;
ring $A_1$ is selected from 4-6-membered monocyclic cycloalkyl, 4-6-membered monocyclic heterocycloalkyl, 5-6-membered heteroaryl, and phenyl;
rings $A_2$ and $A_3$ are each independently selected from 3-6-membered monocyclic cycloalkyl, 5-6-membered heteroaryl, and phenyl;
$X_1$ and $X_2$ are each independently selected from -CH-, $-CR^x-$, and -N-;
$R^x$ is selected from deuterium, F, Cl, $C_{1-6}$ alkyl, halo $C_{1-6}$ alkyl, and deuterated $C_{1-6}$ alkyl;
$R^{26}$ is selected from hydrogen, deuterium, F, Cl, and $C_{1-6}$ alkyl, wherein the alkyl is optionally further substituted with 1 to 5 groups selected from deuterium, halogen, cyano, hydroxyl, amino, and $C_{1-6}$ alkoxy;
$R_2$ is selected from $-(CR^{2a}R^{2b})_m-C(O)NR^{21}R^{22}$, $-(CR^{2a}R^{2b})_m-COOR^{23}$, - $(CR^{2a}R^{2b})_m-S(O)R^{24}$, $-(CR^{2a}R^{2b})_m-S(O)_2R^{24}$, $-(CR^{2a}R^{2b})_m-C(O)R^{25}$, $-(CR^{2a}R^{2b})_m-P(O)_2R^{24}$, and $-(CR^{2a}R^{2b})_m$-tetrazol-5-yl;
$R^{2a}$ and $R^{2b}$ are each independently selected from hydrogen, deuterium, F, Cl, $C_{1-6}$ alkyl, and halo $C_{1-6}$ alkyl, or $R^{2a}$ and $R^{2b}$ together with the carbon atom to which they are attached form 3-4-membered cycloalkyl or 4-membered heterocycloalkyl;
$R^{21}$ and $R^{22}$ are each independently selected from hydrogen, deuterium, and $C_{1-6}$ alkyl, wherein the alkyl is optionally further substituted with deuterium;

$R^{23}$ and $R^{25}$ are each selected from hydrogen, deuterium, $C_{1-6}$ alkyl, and halo $C_{1-6}$ alkyl, wherein the alkyl is optionally further substituted with deuterium;

each $R^{24}$ is independently selected from hydrogen, deuterium, hydroxyl, $C_{1-6}$ alkyl, and -NH$C_{1-6}$ alkyl, wherein the alkyl is optionally further substituted with deuterium; and

each m is independently selected from 0, 1, 2, 3, or 4.

In the compound of formula (I), or the stereoisomer, deuterated compound, solvate, prodrug, metabolite, pharmaceutically acceptable salt or eutectic thereof according to the present invention,

provided that:

(1) when B is selected from B7 structure, and A is selected from

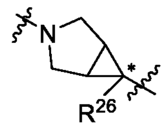

,

$R^2$ is not selected from selected from $-CH_2-COOR^{23}$;

(2) when n is selected from 2, and B is selected from B1, ring A is not selected from

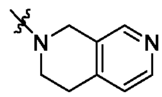

;

(3) when n is selected from 2 and 3, and B is selected from B4, ring A is not selected from piperazinyl; and

(4) when B is selected from B8 or B9 structure, n is only selected from 1, and $R^1$ is not selected from hydroxyl.

Embodiment two of the present invention relates to the compound of formula (I), or the stereoisomer, deuterated compound, solvate, prodrug, metabolite, pharmaceutically acceptable salt or eutectic thereof, wherein the compound of formula (I) has a structure of formula (I-a) or (I-b):

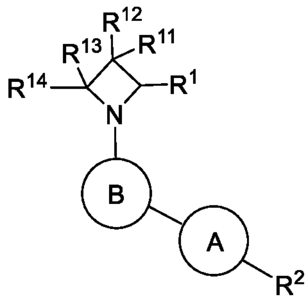

(I-a)

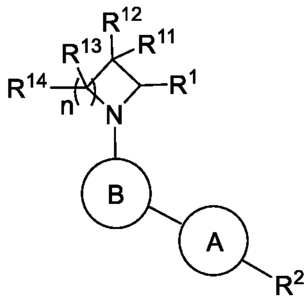

(I-b)

each $R^1$ is independently selected from $C_{1-3}$ alkyl, $C_{2-4}$ alkenyl, and $C_{2-4}$ alkynyl, wherein the alkyl is optionally substituted with 1 to 3 groups selected from halogen, deuterium, hydroxyl, and amino;

$R^{11}$, $R^{12}$, $R^{13}$, and $R^{14}$ are each independently selected from H, deuterium, $C_{1-3}$ alkyl, F, and Cl, wherein alkyl is optionally substituted with 1 to 3 groups selected from F, Cl, deuterium, hydroxyl, and amino;

other groups have the same definitions as those in embodiment one.

Embodiment three of the present invention relates to the compound, or the stereoisomer, deuterated compound, solvate, prodrug, metabolite, pharmaceutically acceptable salt or eutectic thereof according to the present invention, wherein

ring A is selected from the following groups, wherein * represents a connection site of ring A to $R^2$:

(1) 4-membered monocyclic heterocycloalkyl containing 1 to 3 N heteroatoms and containing 0 to 1 heteroatom

selected from O and S, 5-membered monocyclic heterocycloalkyl containing 1 to 3 N heteroatoms and containing 0 to 1 heteroatom selected from O and S, 7-membered monocyclic heterocycloalkyl containing 1 to 3 N heteroatoms and containing 0 to 1 heteroatom selected from O and S, 4-membered monocyclic cycloalkyl, 5-membered monocyclic cycloalkyl, and 6-membered monocyclic cycloalkyl, wherein the N atom is a connection site of ring A to ring B;

(2) azetidinyl spiro 3-membered cycloalkyl, azetidinyl spiro 4-membered cycloalkyl, azetidinyl spiro 5-membered cycloalkyl, azetidinyl spiro 6-membered cycloalkyl, azacyclopentyl spiro 3-membered cycloalkyl, azacyclopentyl spiro 4-membered cycloalkyl, azacyclopentyl spiro 5-membered cycloalkyl, azacyclopentyl spiro 6-membered cycloalkyl, azacyclohexyl spiro 3-membered cycloalkyl, azacyclohexyl spiro 4-membered cycloalkyl, azacyclohexyl spiro 5-membered cycloalkyl, azacyclohexyl spiro 6-membered cycloalkyl, azetidinyl spiro 3-membered heterocycloalkyl, azetidinyl spiro 4-membered heterocycloalkyl, azetidinyl spiro 5-membered heterocycloalkyl, azetidinyl spiro 6-membered heterocycloalkyl, azacyclopentyl spiro 3-membered heterocycloalkyl, azacyclopentyl spiro 4-membered heterocycloalkyl, azacyclopentyl spiro 5-membered heterocycloalkyl, azacyclopentyl spiro 6-membered heterocycloalkyl, azacyclohexyl spiro 3-membered heterocycloalkyl, azacyclohexyl spiro 4-membered heterocycloalkyl, azacyclohexyl spiro 5-membered heterocycloalkyl, or azacyclohexyl spiro 6-membered heterocycloalkyl, wherein the heterocycloalkyl is a saturated monocyclic heterocycloalkyl containing 1 to 2 heteroatoms selected from N, O, and S, the connection site of ring A to $R^2$ is cycloalkyl or heterocycloalkyl, and ring B and $R^2$ are connected in different rings of ring A;

(3)

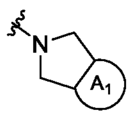, 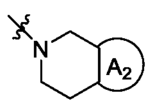, or 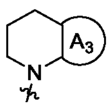,

wherein the connection site of ring A to $R_2$ is $A_1$, $A_2$, or $A_3$ ring atom;

(4)

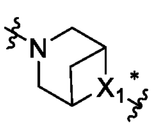, 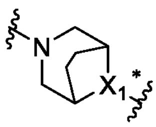, 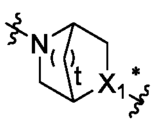, 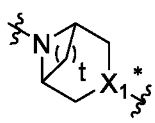, or 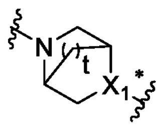;

(5)

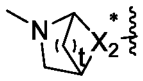 or 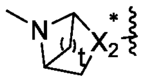;

(6)

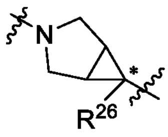 or 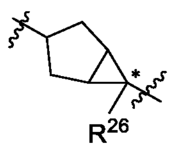;

the ring A is optionally further substituted with 1 to 3 $R^A$;

each $R^A$ is independently selected from deuterium, halogen, cyano, hydroxyl, amino, =O, $C_{1-4}$ alkyl, $C_{1-4}$ alkoxy, halo $C_{1-4}$ alkyl, halo $C_{1-4}$ alkoxy, deuterated $C_{1-4}$ alkyl, and deuterated $C_{1-4}$ alkoxy, or two $R^A$ on the same atom together form 3-4-membered monocyclic cycloalkyl;

$R^{26}$ is selected from hydrogen, deuterium, F, Cl, and $C_{1-4}$ alkyl, wherein the alkyl is optionally further substituted with 1 to 3 groups selected from deuterium, halogen, cyano, hydroxyl, amino, and $C_{1-3}$ alkoxy;

each t is independently selected from 1 or 2;

ring $A_1$ is selected from 4-membered monocyclic cycloalkyl, 5-membered monocyclic cycloalkyl, 6-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, and phenyl;
ring $A_2$ is selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, and phenyl;
each ring $A_3$ is independently selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, and phenyl;
$X_1$ and $X_2$ are each independently selected from -CH-, -CR$^x$-, and -N-;
$R^x$ is selected from deuterium, F, Cl, $C_{1-3}$ alkyl, halo $C_{1-3}$ alkyl, and deuterated $C_{1-3}$ alkyl;
other groups have the same definitions as those in either embodiment one or embodiment two described herein.

Embodiment four of the present invention relates to the compound, or the stereoisomer, deuterated compound, solvate, prodrug, metabolite, pharmaceutically acceptable salt or eutectic thereof described herein, wherein

ring A is selected from cyclobutyl, cyclopentyl, cyclohexyl,

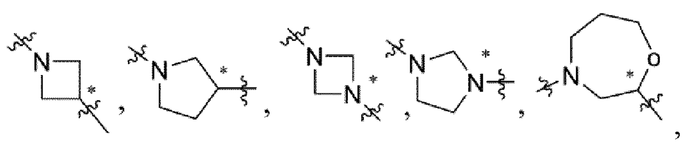

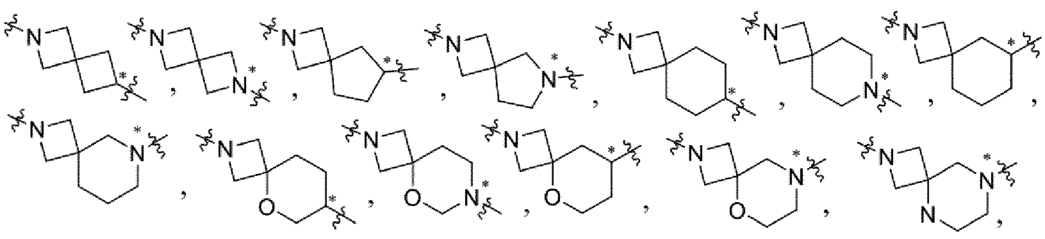

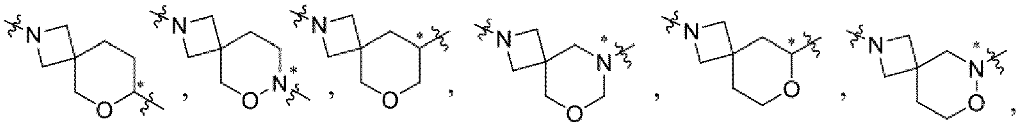

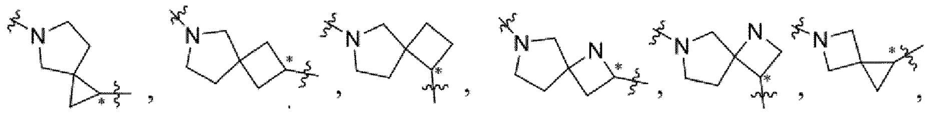

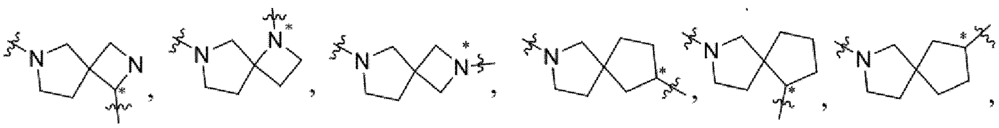

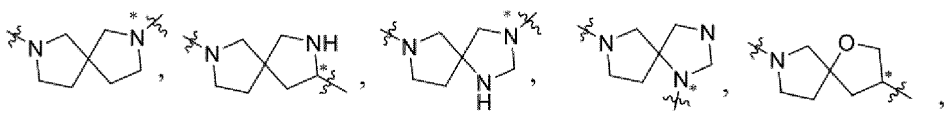

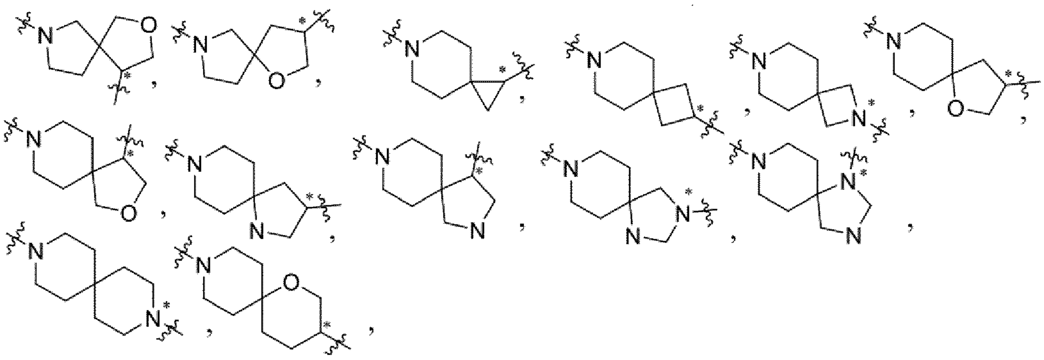
, , , , , ,
, , , , ,
, ,

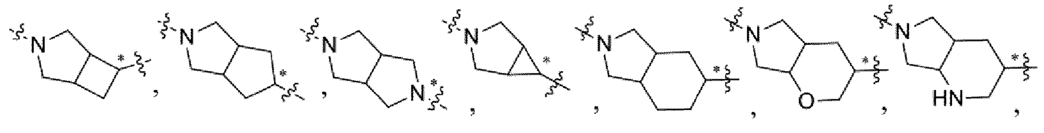
, , , , , , ,

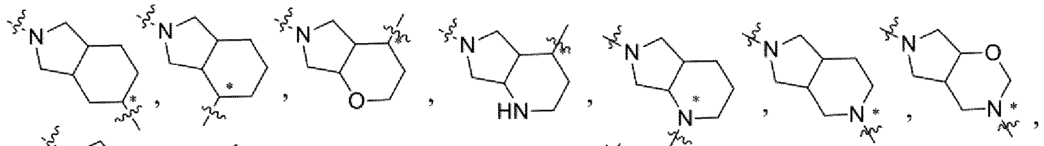
, , , , , , ,

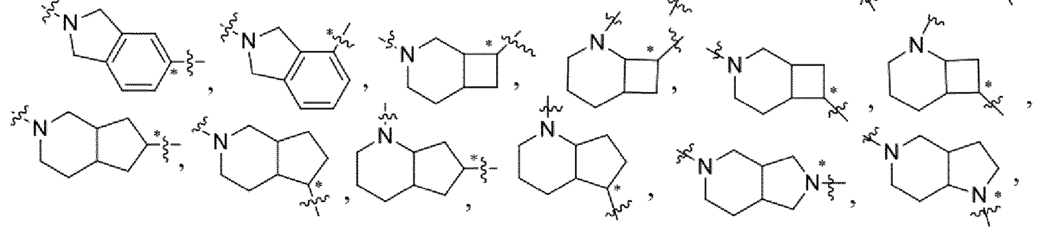
, , , , , ,
, , , , , ,

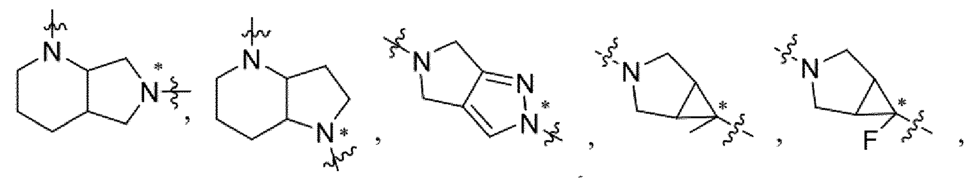
, , , , ,

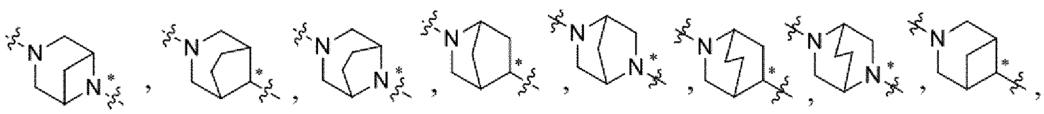
, , , , , , , ,

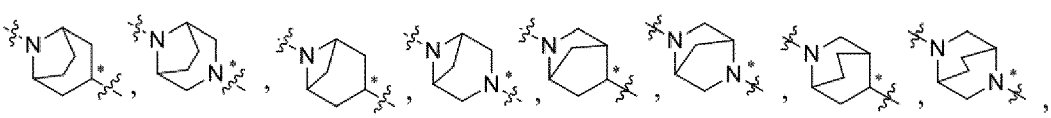
, , , , , , , ,

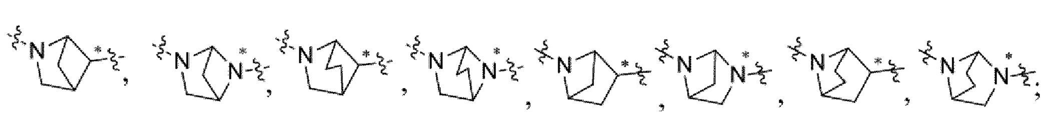
, , , , , , , ;

wherein * represents a connection site of ring A to $R^2$;
or ring A is optionally further substituted with 1 to 3 $R^A$;
each $R^A$ is independently selected from deuterium, F, cyano, hydroxyl, amino, methyl, ethyl, methoxy, ethoxy, $-CH_2F$, $-CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$;
other groups have the same definitions as those in either embodiment one or embodiment two.

Embodiment five of the present invention relates to the compound, or the stereoisomer, deuterated compound, solvate, prodrug, metabolite, pharmaceutically acceptable salt or eutectic thereof described herein, wherein

ring A is selected from 5-membered heteroaryl containing 1 to 2 heteroatoms selected from N, O, and S, 8-membered heteroaryl containing 1 to 2 heteroatoms selected from N, O, and S, 9-membered heteroaryl containing 1 to 2 heteroatoms selected from N, O, and S, and 10-membered heteroaryl containing 1 to 2 heteroatoms selected from N, O, and S;
other groups have the same definitions as those in either embodiment one or embodiment two.

Embodiment six of the present invention relates to the compound, or the stereoisomer, deuterated compound, solvate, prodrug, metabolite, pharmaceutically acceptable salt or eutectic thereof described herein, wherein

each $R^1$ is independently selected from $C_{1-3}$ alkyl, $C_{2-4}$ alkenyl, and $C_{2-4}$ alkynyl, wherein the alkyl is optionally substituted with 1 to 3 groups selected from F, Cl, deuterium, and hydroxyl; or
each $R^1$ is independently selected from $C_{1-3}$ alkyl and $C_{2-4}$ alkynyl, wherein the alkyl is optionally substituted with 1 to 3 groups selected from F, Cl, deuterium, and hydroxyl; or
each $R^1$ is independently selected from methyl and ethynyl, wherein the methyl is optionally substituted with 1 to 3 groups selected from halogen, deuterium, and hydroxyl;
other groups have the same definitions as those in any of the preceding embodiments described herein.

Embodiment seven of the present invention relates to the compound, or the stereoisomer, deuterated compound, solvate, prodrug, metabolite, pharmaceutically acceptable salt or eutectic thereof described herein, wherein

$R^{11}$, $R^{12}$, $R^{13}$, and $R^{14}$ are each independently selected from H, deuterium, $C_{1-3}$ alkyl, F, and Cl, wherein alkyl is optionally substituted with 1 to 3 groups selected from F, Cl, deuterium, hydroxyl, and amino; or
$R^{11}$, $R^{12}$, $R^{13}$, and $R^{14}$ are each independently selected from H, deuterium, methyl, ethyl, F, and Cl;
other groups have the same definitions as those in any of the preceding embodiments described herein.

Embodiment eight of the present invention relates to the (I) compound, or the stereoisomer, deuterated compound, solvate, prodrug, metabolite, pharmaceutically acceptable salt or eutectic thereof described herein, wherein

$R_2$ is selected from $-(CR^{2a}R^{2b})_m-C(O)NR^{21}R^{22}$ and $-(CR^{2a}R^{2b})_m-COOR^{23}$, and $R_2$ is further selected from$-(CR^{2a}R^{2b})_m-COOR^{23}$;
$R^{2a}$ and $R^{2b}$ are each independently selected from hydrogen, deuterium, F, Cl, methyl, and ethyl, or $R^{2a}$ and $R^{2b}$ together with the carbon atom to which they are attached form cyclopropyl or cyclobutyl;
$R^{21}$ and $R^{22}$ are each independently selected from hydrogen, deuterium, methyl, ethyl, propyl, or tert-butyl, wherein the methyl, ethyl, propyl, or tert-butyl is optionally further substituted with deuterium;
$R^{23}$ is selected from hydrogen, deuterium, methyl, ethyl, propyl, or tert-butyl, and the $R^{23}$ is optionally further substituted with deuterium;
m is selected from 0 or 1;
other groups have the same definitions as those in any of the preceding embodiments described herein.

Embodiment nine of the present invention relates to the compound, or the stereoisomer, deuterated compound, solvate, prodrug, metabolite, pharmaceutically acceptable salt or eutectic thereof described herein, wherein

$R^C$, $R^D$, $R^E$, $R^G$, and $R^{B11}$ are each independently selected from deuterium, F, Cl, nitro, cyano, amino, hydroxyl, =O, $-SF_5$, di($C_{1-3}$ alkyl)phosphonyl, $C_{1-4}$ alkyl, $C_{1-4}$ alkoxy, $-(CH_2)_r-C_{3-6}$ cycloalkyl, and $-(CH_2)_r-C_{3-6}$ heterocycloalkyl, wherein the $CH_2$, alkyl, alkoxy, cycloalkyl, and heterocycloalkyl are optionally further substituted with 1-3 groups selected from F, Cl, deuterium, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy;
$R^{B12}$, $R^{B13}$, $R^{31}$, $R^{32}$, $R^{41}$, $R^{42}$ $R^{43}$, $R^{51}$, $R^{52}$, and $R^{53}$ are each independently selected from hydrogen, deuterium, F, Cl, cyano, $-SF_5$, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, and phenyl, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy; or
$R^C$, $R^D$, $R^E$, $R^G$, and $R^{B11}$ are each independently selected from deuterium, F, Cl, cyano, hydroxyl, =O, $-SF_5$, di(methyl)phosphonyl, methyl, ethyl, propyl, isopropyl, tert-butyl, 2-methylpropyl, methoxy, ethoxy, propoxy, tert-butoxy, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, $-CH_2$-azacyclohexyl, oxetanyl, oxacyclopentyl, oxacyclohexyl, $-CH_2$-oxetanyl, $-CH_2$-oxacyclopentyl, - $CH_2$-oxacyclohexyl, thietanyl, thiolanyl, thianyl, $-CH_2$-thietanyl, $-CH_2$-thiolanyl, and $-CH_2$-thianyl, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, $-CHF_2$, $-CF_3$, $-OCH_2F$, - $OCHF_2$, $-OCF_3$, $-CH_2D$, $-CHD_2$, $-CD_3$, $-OCH_2D$, $-OCHD_2$, and $-OCD_3$;

$R^{B12}$, $R^{B13}$, $R^{31}$, $R^{32}$, $R^{41}$, $R^{42}$ $R^{43}$, $R^{51}$, $R^{52}$, and $R^{53}$ are each independently selected from hydrogen, deuterium, F, Cl, cyano, $-SF_5$, methyl, ethyl, propyl, isopropyl, tert-butyl, 2-methylpropyl, methoxy, ethoxy, propoxy, tert-butoxy, -S-methyl, -S-ethyl, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, $-CH_2$-azacyclohexyl, oxetanyl, oxacyclopentyl, oxacyclohexyl, $-CH_2$-oxetanyl, $-CH_2$-oxacyclopentyl, $-CH_2$-oxacyclohexyl, thietanyl, thiolanyl, thianyl, $-CH_2$-thietanyl, - $CH_2$-thiolanyl, $-CH_2$-thianyl, -O-cyclopropyl, -O-cyclobutyl, -O-cyclopentyl, -O-cyclohexyl, -O-azetidinyl, -O-azacyclopentyl, and -O-azacyclohexyl, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, $-CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$;

other groups have the same definitions as those in any of the preceding embodiments described herein.

Embodiment ten of the present invention relates to the compound, or the stereoisomer, deuterated compound, solvate, prodrug, metabolite, pharmaceutically acceptable salt or eutectic thereof described herein, wherein

ring B is selected from the following groups, wherein # represents a connection site of ring B to ring A;

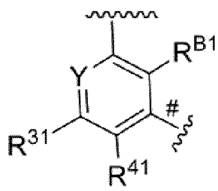 , 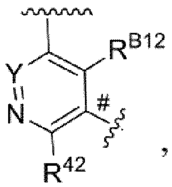 , 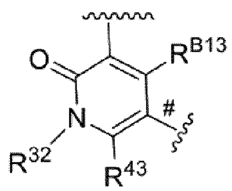 ,

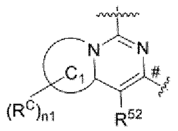 , 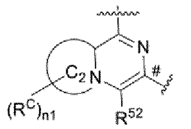 , 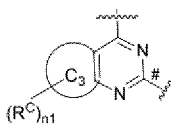 , 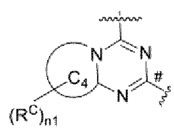 , 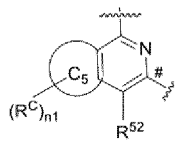 ,

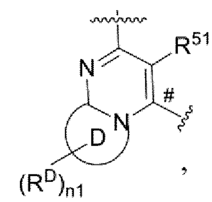 ,
B5-1

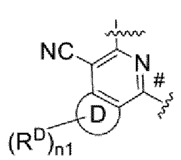
B5-2

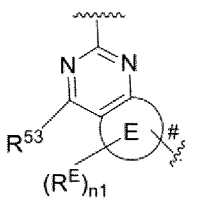 ,
B6-1

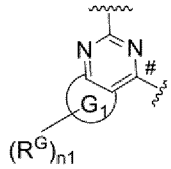 ,
B7-1

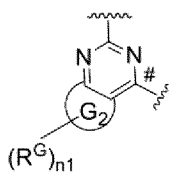
B7-2

or ring B is selected from the following groups:

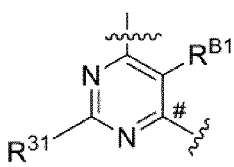 ,
B8

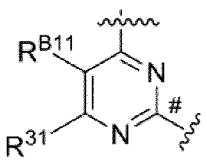
B9 ;

rings $C_1$, $C_2$, $C_3$, and $C_4$ are selected from 5-membered heteroaryl and 6-membered heteroaryl;

$C_5$ is selected from 5-membered cycloalkyl, 6-membered cycloalkyl, 5-membered heterocycloalkyl, 6-membered heterocycloalkyl, 5-membered heteroaryl, and 6-membered heteroaryl;
ring D is selected from 5-membered heteroaryl, 6-membered heteroaryl, 5-membered cycloalkyl, and 6-membered cycloalkyl;
ring $G_1$ is selected from phenyl, 5-membered heteroaryl, and 6-membered heteroaryl;
$G_2$ is selected from 5-membered cycloalkyl and 6-membered cycloalkyl;
ring E is selected from 5-membered heterocycloalkyl and 6-membered heterocycloalkyl;
n1 is selected from 0, 1, 2, or 3;
$R^C$, $R^D$, $R^E$, $R^G$, and $R^{B11}$ are each independently selected from deuterium, F, Cl, cyano, $-SF_5$, di($C_{1-2}$ alkyl)phosphonyl, $C_{1-4}$ alkyl, and $C_{1-4}$ alkoxy, wherein the alkyl and alkoxy are optionally further substituted with 1, 2, or 3 groups selected from F, Cl, deuterium, hydroxyl, =O, methyl, ethyl, methoxy, ethoxy, $-CF_3$, $-CHF_2$, $-CH_2F$, $-OCF_3$, $-OCHF_2$, and $-OCH_2F$;
$R^{B12}$, $R^{B13}$, $R^{31}$, $R^{32}$, $R^{41}$, $R^{42}$ $R^{43}$, $R^{51}$, $R^{52}$, and $R^{53}$ are each independently selected from hydrogen, deuterium, F, Cl, cyano, $-SF_5$, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, phenyl, $-NHC_{1-3}$ alkyl, and $-N(C_{1-3}$ alkyl$)_2$, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, methyl, ethyl, methoxy, ethoxy, $-CF_3$, $-CHF_2$, $-CH_2F$, $-OCF_3$, $-OCHF_2$, and $-OCH_2F$;
provided that: (1) when ring B is selected from B7-1 and B7-2, A-$R^2$ is not

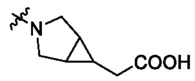 or 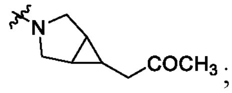;

and
(2) when B is selected from B8 or B9 structure, n is only selected from 1, and $R^1$ is not selected from hydroxyl.
other groups have the same definitions as those in any of the preceding embodiments described herein.

Embodiment eleven of the present invention relates to the compound, or the stereoisomer, deuterated compound, solvate, prodrug, metabolite, pharmaceutically acceptable salt or eutectic thereof described herein, wherein

ring B is selected from the following groups:

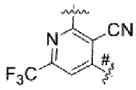, 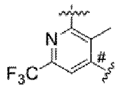, 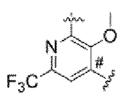, 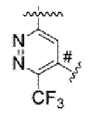, 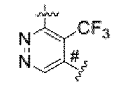, 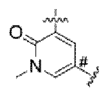, 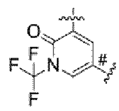, 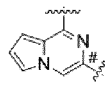,

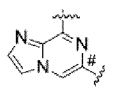, 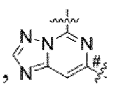, 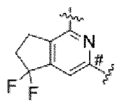, 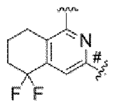, 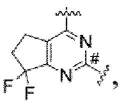, 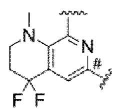, 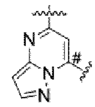, 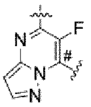,

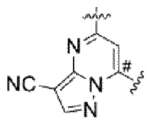, 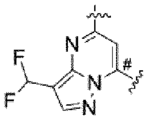, 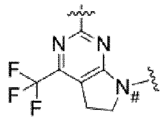 or 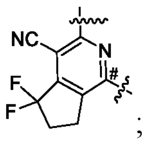;

or
ring B is selected from the following groups:

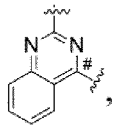, 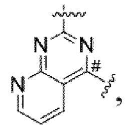, 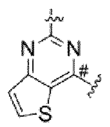, 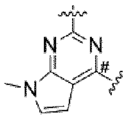, 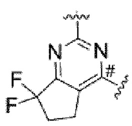, 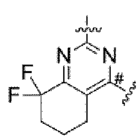,

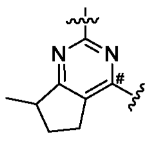, 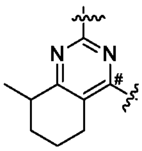, 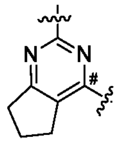, or 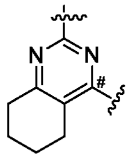,

provided that A-$R^2$ is not

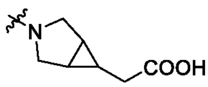 or 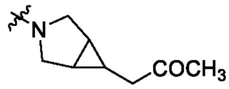;

or

ring B is selected from the following groups:

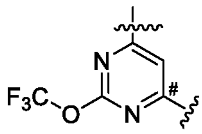, 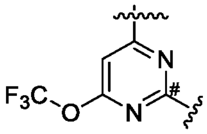;

wherein # represents a connection site of ring B to ring A;

other groups have the same definitions as those in any of the preceding embodiments described herein.

Embodiment twelve of the present invention relates to a compound of formula (I-a), or a stereoisomer, a deuterated compound, a solvate, a prodrug, a metabolite, a pharmaceutically acceptable salt or a eutectic thereof,

wherein $R^1$ is D, methyl, ethyl, or propyl, wherein the methyl, ethyl, or propyl is optionally substituted with 1 to 3 groups selected from halogen and deuterium;

$R^{11}$, $R^{12}$, $R^{13}$, and $R^{14}$ are each independently selected from H, deuterium, F, and Cl;

provided that when $R^1$ is methyl, $R^{11}$, $R^{12}$, $R^{13}$, and $R^{14}$ are not simultaneously H;

ring B is selected from the following groups:

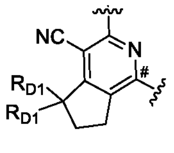 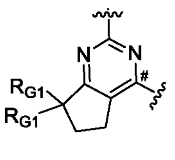 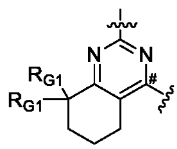;

$R_{D1}$ and $R_{G1}$ are each independently selected from D, H, F, Cl, Br, I, methyl, ethyl, or propyl, wherein the methyl, ethyl, or propyl is optionally substituted with 1-3 substituents selected from D, F, Cl, Br, and I;

ring A is selected from the following groups:

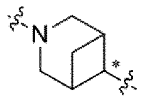, 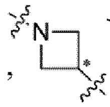, 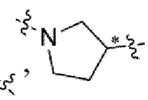;

$R^2$ is $-CR^{2a}R^{2b}-COOR^{23}$;

$R^{2a}$ and $R^{2b}$ are each independently selected from hydrogen, deuterium, F, Cl, methyl, and ethyl;

$R^{23}$ is selected from hydrogen, deuterium, methyl, ethyl, propyl, or tert-butyl;

other groups are as described in embodiment two.

Embodiment thirteen of the present invention relates to the compound, or the stereoisomer, deuterated compound, solvate, prodrug, metabolite, pharmaceutically acceptable salt or eutectic thereof according to embodiment

twelve, wherein

$R^1$ is D, methyl, ethyl, or propyl, wherein the methyl, ethyl, or propyl is substituted with 1 to 3 groups selected from halogen and deuterium; or
at least one of $R^{11}$, $R^{12}$, $R^{13}$, and $R^{14}$ is selected from deuterium, F, and Cl.

Embodiment fourteen of the present invention relates to the compound, or the stereoisomer, deuterated compound, solvate, prodrug, metabolite, pharmaceutically acceptable salt or eutectic thereof described herein, wherein

ring B is selected from B1, B2, B3, B1, B2, B3, B4, B5, B6, and B7, wherein # represents a connection site of ring B to ring A;

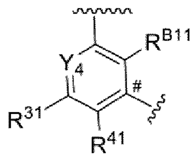 , 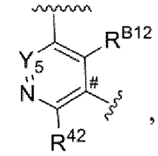 , 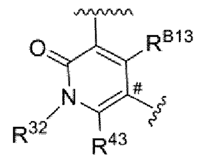 ,

B1 B2 B3

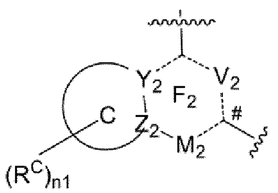 , 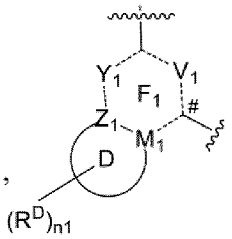 , 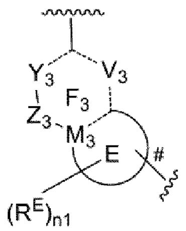 , 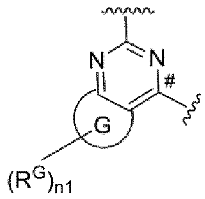

B4 B5 B6 B7

or ring B is selected from:

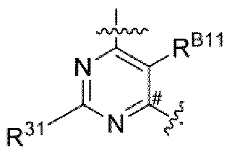 , 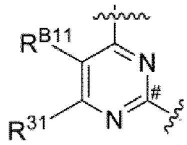

B8 B9 ;

ring C is selected from 5-membered cycloalkyl, 6-membered cycloalkyl, 5-membered heterocycloalkyl, 6-membered heterocycloalkyl, 5-membered heteroaryl, and 6-membered heteroaryl;
ring D is selected from 5-membered cycloalkyl, 6-membered cycloalkyl, 5-membered heterocycloalkyl, 6-membered heterocycloalkyl, 5-membered heteroaryl, and 6-membered heteroaryl;
ring E is selected from 5-membered heterocycloalkyl;
ring G is selected from phenyl, 5-membered heteroaryl, and 6-membered heteroaryl;
ring A is selected from the following groups, and the ring A is optionally further substituted with 1, 2, 3, 4, or 5 $R^A$, wherein * represents a connection site of ring A to $R^2$:

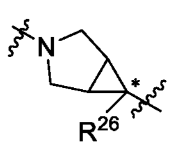 , 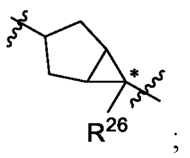 ;

each $R^A$ is independently selected from deuterium, halogen, cyano, hydroxyl, amino, =O, $C_{1-4}$ alkyl, $C_{1-6}$ alkoxy, halo $C_{1-4}$ alkyl, halo $C_{1-4}$ alkoxy, deuterated $C_{1-4}$ alkyl, and deuterated $C_{1-4}$ alkoxy, or two $R^A$ on the same atom

together form 3-membered monocyclic alkyl;

provided that: when B is selected from B8 or B9 structure, n is only selected from 1, and $R^1$ is not selected from hydroxyl;

other groups have the same definitions as those in any of the preceding embodiments described herein.

Embodiment fifteen of the present invention relates to the compound, or the stereoisomer, deuterated compound, solvate, prodrug, metabolite, pharmaceutically acceptable salt or eutectic thereof described herein, wherein

ring B is selected from the following groups, wherein # represents a connection site of ring B to ring A;

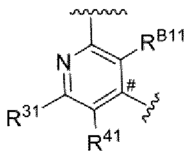
B1-1
,
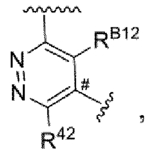
B2-1
,
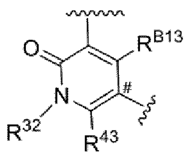
B3-1
,

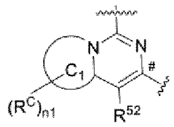
B4-1
,
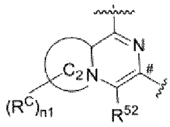
B4-2
,
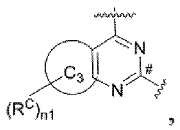
B4-3
,
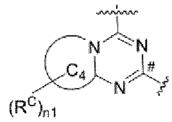
B4-4
,
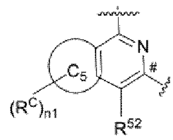
B4-5
,

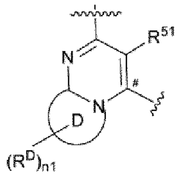
B5-1
,
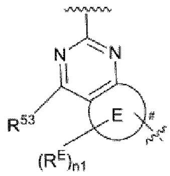
B6-1
,
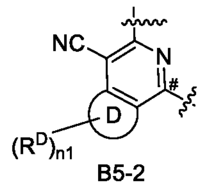
B5-2

or ring B is selected from:

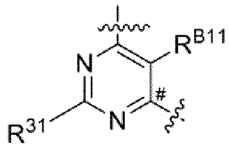
B8
,
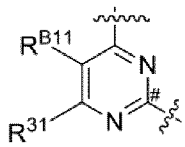
B9
;

rings $C_1$, $C_2$, $C_3$, and $C_4$ are selected from 5-membered heteroaryl and 6-membered heteroaryl;

$C_5$ is selected from 5-membered cycloalkyl, 6-membered cycloalkyl, 5-membered heterocycloalkyl, 6-membered heterocycloalkyl, 5-membered heteroaryl, and 6-membered heteroaryl;

ring D is selected from 5-membered heteroaryl, 6-membered heteroaryl, 5-membered cycloalkyl, and 6-membered cycloalkyl;

ring E is selected from 5-membered heterocycloalkyl and 6-membered heterocycloalkyl;

n1 is selected from 0, 1, 2, and 3;

ring A is selected from the following groups, wherein * represents a connection site of ring A to $R^2$:

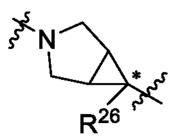
,
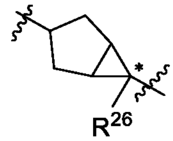
;

$R^2$ is selected from $-(CR^{2a}R^{2b})_m-C(O)NR^{21}R^{22}$ and $-(CR^{2a}R^{2b})_m-COOR^{23}$;
$R^{2a}$ and $R^{2b}$ are each independently selected from hydrogen, deuterium, F, Cl, methyl, and ethyl, or $R^{2a}$ and $R^{2b}$ together with the carbon atom to which they are attached form cyclopropyl or cyclobutyl;
$R^{21}$ and $R^{22}$ are each independently selected from hydrogen, deuterium, methyl, ethyl, propyl, or tert-butyl, wherein the methyl, ethyl, propyl, or tert-butyl is optionally further substituted with deuterium;
$R^{23}$ is selected from hydrogen, deuterium, methyl, ethyl, propyl, or tert-butyl, and the $R^{23}$ is optionally further substituted with deuterium;
m is selected from 0 or 1;
$R^C$, $R^D$, $R^E$, $R^G$, and $R^{B11}$ are each independently selected from deuterium, F, Cl, nitro, cyano, amino, hydroxyl, =O, $-SF_5$, di($C_{1-3}$ alkyl)phosphonyl, $C_{1-4}$ alkyl, $C_{1-4}$ alkoxy, $-(CH_2)_r-C_{3-6}$ cycloalkyl, and $-(CH_2)_r-C_{3-6}$ heterocycloalkyl, wherein the $CH_2$, alkyl, alkoxy, cycloalkyl, and heterocycloalkyl are optionally further substituted with 1-3 groups selected from F, Cl, deuterium, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy;
$R^{B12}$, $R^{B13}$, $R^{31}$, $R^{32}$, $R^{41}$, $R^{42}$ $R^{43}$, $R^{51}$, $R^{52}$, and $R^{53}$ are each independently selected from hydrogen, deuterium, F, Cl, cyano, $-SF_5$, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, and phenyl, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy;
each r is independently selected from 0, 1, or 2;
provided that: when B is selected from B8 or B9 structure, n is only selected from 1, and $R^1$ is not selected from hydroxyl.
Embodiment sixteen of the present invention relates to the compound, or the stereoisomer, deuterated compound, solvate, prodrug, metabolite, pharmaceutically acceptable salt or eutectic thereof described herein, wherein ring B is selected from the following groups, wherein # represents a connection site of ring B to ring A;

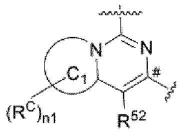
B4-1 ,
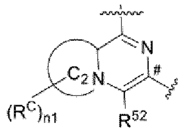
B4-2 ,
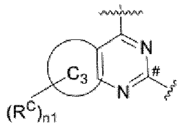
B4-3 ,
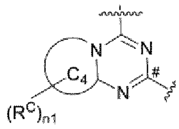
B4-4 ,
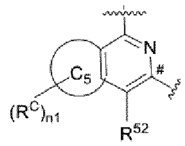
B4-5 ,

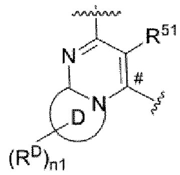
B5-1 ,
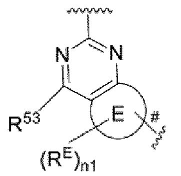
B6-1 ,
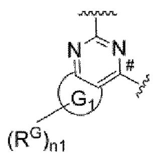
B7-1 ,
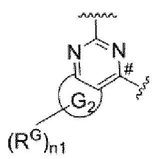
B7-2 ,
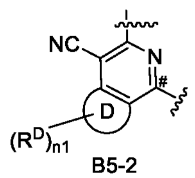
B5-2

rings $C_1$, $C_2$, $C_3$, and $C_4$ are selected from 5-membered heteroaryl and 6-membered heteroaryl;
$C_5$ is selected from 5-membered cycloalkyl, 6-membered cycloalkyl, 5-membered heterocycloalkyl, 6-membered heterocycloalkyl, 5-membered heteroaryl, and 6-membered heteroaryl;
ring D is selected from 5-membered heteroaryl, 6-membered heteroaryl, 5-membered cycloalkyl, and 6-membered cycloalkyl;
ring $G_1$ is selected from phenyl, 5-membered heteroaryl, and 6-membered heteroaryl;
$G_2$ is selected from 5-membered cycloalkyl and 6-membered cycloalkyl;
ring E is selected from 5-membered heterocycloalkyl and 6-membered heterocycloalkyl;
n1 is selected from 0, 1, 2, and 3;
ring A is selected from the following groups, wherein * represents a connection site of ring A to $R^2$:

(1) 4-membered monocyclic heterocycloalkyl, 5-membered monocyclic heterocycloalkyl, 7-membered monocyclic heterocycloalkyl, 4-membered monocyclic cycloalkyl, and 5-membered monocyclic cycloalkyl;

(2)

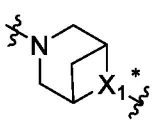, 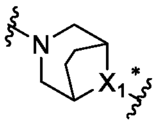, 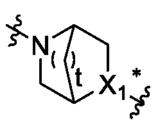, 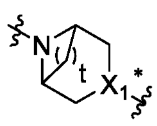, or 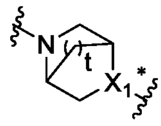,

and t is selected from 1 or 2;

the ring A is optionally further substituted with 1, 2, or 3 $R^A$;
each $R^A$ is independently selected from deuterium, halogen, cyano, hydroxyl, amino, =O, $C_{1-2}$ alkyl, halo $C_{1-2}$ alkyl, halo $C_{1-2}$ alkoxy, deuterated $C_{1-2}$ alkyl, and deuterated $C_{1-2}$ alkoxy, or two $R^A$ on the same atom together form 3- or 4-membered monocyclic cycloalkyl;
$R^2$ is selected from $-(CR^{2a}R^{2b})_m-C(O)NR^{21}R^{22}$ and $-(CR^{2a}R^{2b})_m-COOR^{23}$;
$R^{2a}$ and $R^{2b}$ are each independently selected from hydrogen, deuterium, F, Cl, methyl, and ethyl, or $R^{2a}$ and $R^{2b}$ together with the carbon atom to which they are attached form cyclopropyl or cyclobutyl;
$R^{21}$ and $R^{22}$ are each independently selected from hydrogen, deuterium, methyl, ethyl, propyl, or tert-butyl, wherein the methyl, ethyl, propyl, or tert-butyl is optionally further substituted with deuterium;
$R^{23}$ is selected from hydrogen, deuterium, methyl, ethyl, propyl, or tert-butyl, and the $R^{23}$ is optionally further substituted with deuterium;
m is selected from 0 or 1;
$R^C$, $R^D$, $R^E$, $R^G$, and $R^{B11}$ are each independently selected from deuterium, F, Cl, nitro, cyano, amino, hydroxyl, =O, $-SF_5$, di($C_{1-3}$ alkyl)phosphonyl, $C_{1-4}$ alkyl, $C_{1-4}$ alkoxy, $-(CH_2)_r-C_{3-6}$ cycloalkyl, and $-(CH_2)_r-C_{3-6}$ heterocycloalkyl, wherein the $CH_2$, alkyl, alkoxy, cycloalkyl, and heterocycloalkyl are optionally further substituted with 1-3 groups selected from F, Cl, deuterium, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy;
$R^{B12}$, $R^{B13}$, $R^{31}$, $R^{32}$ $R^{41}$, $R^{42}$ $R^{43}$, $R^{51}$, $R^{52}$, and $R^{53}$ are each independently selected from hydrogen, deuterium, F, Cl, cyano, $-SF_5$, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, and phenyl, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy;
and each r is independently selected from 0, 1, or 2.

In some specific embodiments, ring A is selected from the following groups, wherein * represents a connection site of ring A to $R^2$:

(1) 4-membered monocyclic heterocycloalkyl containing 1 to 2 N heteroatoms and containing 0 to 1 heteroatom selected from O and S, 5-membered monocyclic heterocycloalkyl containing 1 to 2 N heteroatoms and containing 0 to 1 heteroatom selected from O and S, 7-membered monocyclic heterocycloalkyl containing 1 to 2 N heteroatoms and containing 0 to 1 heteroatom selected from O and S, 4-membered monocyclic cycloalkyl, 5-membered monocyclic cycloalkyl, and 6-membered monocyclic cycloalkyl, wherein the N atom is a connection site of ring A to ring B;

(2)

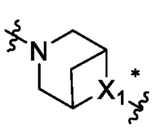, 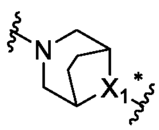, 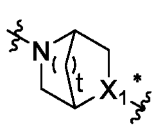, 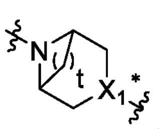, or 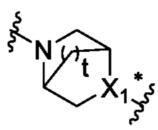;

(3)

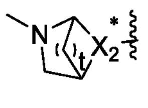 or 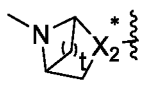;

(4)

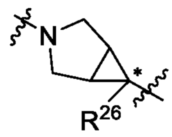 or 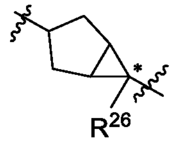 ;

the ring A is optionally further substituted with 1, 2, or 3 $R^A$;
each $R^A$ is independently selected from deuterium, halogen, cyano, hydroxyl, amino, =O, $C_{1-4}$ alkyl, $C_{1-4}$ alkoxy, halo $C_{1-4}$ alkyl, halo $C_{1-4}$ alkoxy, deuterated $C_{1-4}$ alkyl, and deuterated $C_{1-4}$ alkoxy, or two $R^A$ on the same atom together form 3-4-membered monocyclic cycloalkyl;
each t is independently selected from 1 or 2;
$X_1$ and $X_2$ are each independently selected from -CH-, -$CR^x$-, and -N-;
$R^{26}$ is selected from hydrogen, deuterium, F, Cl, methyl, and ethyl; and
$R^x$ is selected from deuterium, F, Cl, $C_{1-3}$ alkyl, halo $C_{1-3}$ alkyl, and deuterated $C_{1-3}$ alkyl.

In some specific embodiments, ring A is selected from the following groups, wherein * represents a connection site of ring A to $R^2$:

(1)

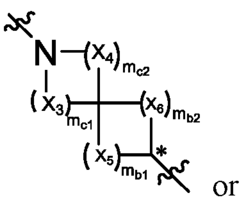 or 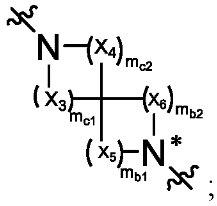 ;

(2)

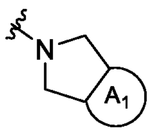 , 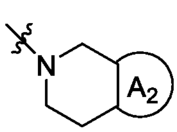 , or 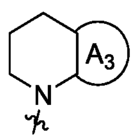 ,

wherein the connection site of ring A to $R_2$ is $A_1$, $A_2$, or $A_3$ ring atom;

the ring A is optionally further substituted with 1, 2, or 3 $R^A$;
each $R^A$ is independently selected from deuterium, halogen, cyano, hydroxyl, amino, =O, $C_{1-4}$ alkyl, $C_{1-4}$ alkoxy, halo $C_{1-4}$ alkyl, halo $C_{1-4}$ alkoxy, deuterated $C_{1-4}$ alkyl, and deuterated $C_{1-4}$ alkoxy, or two $R^A$ on the same atom together form 3-4-membered monocyclic cycloalkyl;
$m_{c1}$ and $m_{c2}$ are each independently selected from 0, 1, 2, 3, and 4, with $1 \leq M_{c1}+M_{c2} \leq 8$;
$m_{b1}$ and $m_{b2}$ are each independently selected from 0, 1, 2, 3, and 4, with $1 \leq m_{b1}+m_{b2} \leq 8$, and $2 \leq m_{b1}+m_{b2}+m_{c1}+m_{c2} \leq 9$;
ring $A_1$ is selected from 4-membered monocyclic cycloalkyl, 5-membered monocyclic cycloalkyl, 6-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, and phenyl;
ring $A_2$ is selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, and phenyl;
and each ring $A_3$ is independently selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, and phenyl.

Embodiment seventeen of the present invention relates to the compound of formula (I-a), or the stereoisomer, deuterated compound, solvate, prodrug, metabolite, pharmaceutically acceptable salt or eutectic thereof,

wherein ring B is selected from the following groups:

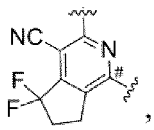, 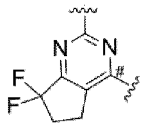, 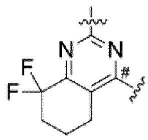;

ring A is selected from the following groups:

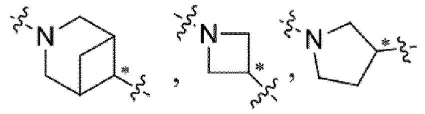;

$R^2$ is $-CR^{2a}R^{2b}-COOR^{23}$;

$R^{2a}$ and $R^{2b}$ are each independently selected from hydrogen, deuterium, F, Cl, methyl, and ethyl;

$R^{23}$ is selected from hydrogen, deuterium, methyl, ethyl, propyl, or tert-butyl.

In some specific embodiments, ring $A_1$ is selected from 4-membered monocyclic cycloalkyl, 5-membered monocyclic cycloalkyl, 6-membered monocyclic cycloalkyl, 5-membered monocyclic heterocycloalkyl, 6-membered monocyclic heterocycloalkyl, 5-membered heteroaryl, and 6-membered heteroaryl; in some specific embodiments, ring $A_1$ is selected from 4-membered monocyclic cycloalkyl, 5-membered monocyclic cycloalkyl, 5-membered monocyclic heterocycloalkyl, and 5-membered heteroaryl.

in some specific embodiments, ring $A_2$ is selected from 5-membered monocyclic heterocycloalkyl, 6-membered monocyclic heterocycloalkyl, and 5-membered heteroaryl; and in some specific embodiments, ring $A_2$ is selected from 5-membered monocyclic heterocycloalkyl and 6-membered monocyclic heterocycloalkyl.

In some specific embodiments, each ring $A_3$ is independently selected from 5-membered monocyclic heterocycloalkyl, 6-membered monocyclic heterocycloalkyl, and 5-membered heteroaryl.

In some specific embodiments, each $R^1$ is independently selected from $C_{1-3}$ alkyl, $C_{2-4}$ alkenyl, $C_{2-4}$ alkynyl, hydroxyl, halogen, amino, nitro, cyano, carboxyl, $C_{1-3}$ alkoxy, $C_{1-3}$ alkylamino, and di($C_{1-3}$ alkyl)amino, wherein the alkyl and alkoxy are optionally substituted with 1, 2, 3, 4, or 5 groups selected from F, Cl, deuterium, hydroxyl, amino, cyano, and $C_{1-3}$ alkoxy; in some specific embodiments, each $R^1$ is independently selected from $C_{1-3}$ alkyl, $C_{2-4}$ alkenyl, $C_{2-4}$ alkynyl, hydroxyl, F, Cl, cyano, and $C_{1-3}$ alkoxy, wherein the alkyl and alkoxy are optionally substituted with 1, 2, or 3 groups selected from F, Cl, deuterium, hydroxyl, amino, cyano, and $C_{1-2}$ alkoxy; in some specific embodiments, each $R^1$ is independently selected from $C_{1-3}$ alkyl, $C_{2-4}$ alkenyl, and $C_{2-4}$ alkynyl, wherein the alkyl is optionally substituted with 1, 2, or 3 groups selected from F, Cl, deuterium, and hydroxyl; in some specific embodiments, each $R^1$ is independently selected from $C_{1-3}$ alkyl and $C_{2-4}$ alkynyl, wherein the alkyl is optionally substituted with 1, 2, or 3 groups selected from F, Cl, deuterium, and hydroxyl; in some specific embodiments, $R^1$ is D, methyl, ethyl, or propyl, wherein the methyl, ethyl, or propyl is optionally substituted with 1 to 3 groups selected from halogen and deuterium; in some specific embodiments, each $R^1$ is independently selected from methyl and ethynyl, wherein the alkyl is optionally substituted with 1, 2, or 3 groups selected from halogen, deuterium, and hydroxyl; in some specific embodiments, each $R^1$ is independently selected from methyl, wherein the alkyl is optionally substituted with 1, 2, or 3 groups selected from halogen, deuterium, and hydroxyl; in some specific embodiments, each $R^1$ is independently selected from methyl; in some specific embodiments, each $R^1$ is independently selected from ethynyl; and in some specific embodiments, each $R^1$ is independently selected from cyano.

In some specific embodiments, p is an integer selected from 1, 2, 3, 4, 5, 6, 7, and 8; in some specific embodiments, p is selected from 1, 2, or 3; and in some specific embodiments, p is selected from 1 or 2.

In some specific embodiments, n is selected from 1 or 2; and in some specific embodiments, n is selected from 1.

In some specific embodiments, $R^C$, $R^D$, $R^E$, $R^G$, and $R^{B11}$ are each independently selected from deuterium, halogen, nitro, cyano, amino, hydroxyl, - $SF_5$, di($C_{1-4}$ alkyl)phosphonyl, $C_{1-4}$ alkyl, $C_{1-4}$ alkoxy, $C_{2-4}$ alkenyl, $C_{2-4}$ alkynyl, - S-$C_{1-4}$ alkyl, -S(O)-$C_{1-4}$ alkyl, -$S(O)_2$-$C_{1-4}$ alkyl, -$(CH_2)_r$-$C_{3-6}$ cycloalkyl, -$(CH_2)_r$-$C_{3-6}$ heterocycloalkyl, -O-$C_{3-6}$ cycloalkyl, -O-$C_{3-6}$ heterocycloalkyl, -NH-$C_{3-6}$ cycloalkyl, -NH-$C_{3-6}$ heterocycloalkyl, -S-$C_{3-6}$ cycloalkyl,-S-$C_{3-6}$ heterocycloalkyl, 5- to 6-membered heteroaryl, phenyl, -NH$C_{1-3}$ alkyl, -N($C_{1-3}$ alkyl$)_2$, - C(=O)$NR^{31a}R^{41a}$, -$NR^{31a}$C(=O)-$R^{41a}$, -$NR^{31a}R^{41a}$, and -C(=O)-$R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy.

In some specific embodiments, $R^C$, $R^D$, $R^E$, $R^G$, and $R^{B11}$ are each independently selected from deuterium, F,

Cl, cyano, hydroxyl, $-SF_5$, methyl, ethyl, propyl, isopropyl, tert-butyl, 2-methylpropyl, methoxy, ethoxy, propoxy, tert-butoxy, -S-methyl, -S-ethyl, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, - $CH_2$-azacyclohexyl, oxetanyl, oxacyclopentyl, oxacyclohexyl, $-CH_2$-oxetanyl, - $CH_2$-oxacyclopentyl, $-CH_2$-oxacyclohexyl, thietanyl, thiolanyl, thianyl, $-CH_2$-thietanyl, $-CH_2$-thiolanyl, $-CH_2$-thianyl, -O-cyclopropyl, -O-cyclobutyl, -O-cyclopentyl, -O-cyclohexyl, -O-azetidinyl, -O-azacyclopentyl, -O-azacyclohexyl, - O-oxetanyl, -O-oxacyclopentyl, -O-oxacyclohexyl, pyrrolyl, furyl, thienyl, imidazolyl, oxazolyl, thiazolyl, pyrazolyl, triazolyl, tetrazolyl, isoxazolyl, pyridyl, pyridazinyl, pyrimidinyl, pyrazinyl, phenyl, $-NHCH_3$, $-NHCH_2CH_3$, $-N(CH_3)CH_3$, $-N(CH_2CH_3)CH_2CH_3$, $-C(=O)NHCH_3$, and $-C(=O)N(CH_3)CH_3$, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, $-CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$; further, $R^C$, $R^D$, $R^E$, and $R^G$ are each independently selected from cyano, $-SF_5$, di(methyl)phosphonyl, methyl, F, $-CH_2F$, $-CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$; in some specific embodiments, $R_D$ is $R_{D1}$, $R_G$ is $R_{G1}$, $R_{D1}$ and $R_{G1}$ are independently selected from D, H, F, Cl, Br, I, methyl, ethyl, or propyl, wherein the methyl, ethyl, or propyl is optionally substituted with 1 to 3 substituents selected from D, F, Cl, Br, and I; and

in some specific embodiments, $R^{B12}$, $R^{B13}$, $R^{31}$, $R^{32}$, $R^{41}$, $R^{42}$, $R^{43}$, $R^{51}$, $R^{52}$, $R^{53}$, and $R^{54}$ are each independently selected from hydrogen, deuterium, halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $-S-C_{1-4}$ alkyl, $-S(O)-C_{1-4}$ alkyl, $-S(O)_2-C_{1-4}$ alkyl, $-(CH_2)_r-C_{3-6}$ cycloalkyl, - $(CH_2)_r-C_{3-6}$ heterocycloalkyl, $-O-C_{3-6}$ cycloalkyl, $-O-C_{3-6}$ heterocycloalkyl, $-NH-C_{3-6}$ cycloalkyl, $-NH-C_{3-6}$ heterocycloalkyl, $-S-C_{3-6}$ cycloalkyl, $-S-C_{3-6}$ heterocycloalkyl, 5- to 6-membered heteroaryl, phenyl, $-NHC_{1-3}$ alkyl, $-N(C_{1-3}$ alkyl)2, $-C(=O)NR^{31a}R^{41a}$, $-NR^{31a}C(=O)-R^{41a}$, $-NR^{31a}R^{41a}$, and $-C(=O)-R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, and deuterated $C_{1-2}$ alkoxy.

In some specific embodiments, $R^{B12}$, $R^{B13}$, $R^{31}$, $R^{32}$, $R^{41}$, $R^{42}$, $R^{43}$, $R^{51}$, $R^{52}$, $R^{53}$, and $R^{54}$ are each independently selected from hydrogen, deuterium, F, Cl, cyano, hydroxyl, $-SF_5$, methyl, ethyl, propyl, isopropyl, tert-butyl, 2-methylpropyl, methoxy, ethoxy, propoxy, tert-butoxy, -S-methyl, -S-ethyl, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, $-CH_2$-azacyclohexyl, oxetanyl, oxacyclopentyl, oxacyclohexyl, $-CH_2$-oxetanyl, $-CH_2$-oxacyclopentyl, $-CH_2$-oxacyclohexyl, thietanyl, thiolanyl, thianyl, $-CH_2$-thietanyl, $-CH_2$-thiolanyl, $-CH_2$-thianyl, -O-cyclopropyl, -O-cyclobutyl, -O-cyclopentyl, -O-cyclohexyl, -O-azetidinyl, -O-azacyclopentyl, -O-azacyclohexyl, -O-oxetanyl, -O-oxacyclopentyl, -O-oxacyclohexyl, pyrrolyl, furyl, thienyl, imidazolyl, oxazolyl, thiazolyl, pyrazolyl, triazolyl, tetrazolyl, isoxazolyl, pyridyl, pyridazinyl, pyrimidinyl, pyrazinyl, phenyl, $-NHCH_3$, $-NHCH_2CH_3$, $-N(CH_3)CH_3$, $-N(CH_2CH_3)CH_2CH_3$, - $C(=O)NHCH_3$, and $-C(=O)N(CH_3)CH_3$, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, - $CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$; and further, $R^{B12}$, $R^{B13}$, $R^{31}$, $R^{32}$ $R^{41}$, $R^{42}$, $R^{43}$, $R^{51}$, $R^{52}$, $R^{53}$, and $R^{54}$ are each independently selected from hydrogen, deuterium, cyano, $-SF_5$, methyl, ethyl, methoxy, ethoxy, $-CH_2F$, $-CHF_2$, $-CF_3$, $-OCH_2F$, $-OCHF_2$, $-OCF_3$, $-CH_2D$, - $CHD_2$, $-CD_3$, $-OCH_2D$, $-OCHD_2$, and $-OCD_3$.

In some specific embodiments, $R^{11}$, $R^{12}$, $R^{13}$, and $R^{14}$ are each independently selected from H, deuterium, F, and Cl.

In some specific embodiments, when $R^1$ is methyl, $R^{11}$, $R^{12}$, $R^{13}$, and $R^{14}$ are not simultaneously H.

In some specific embodiments, compounds satisfy that $R^1$ is D, methyl, ethyl, or propyl, wherein the methyl, ethyl, or propyl is substituted with 1 to 3 groups selected from halogen and deuterium; or at least one of $R^{11}$, $R^{12}$, $R^{13}$, and $R^{14}$ is selected from deuterium, F, and Cl.

In some specific embodiments, each r is independently selected from 0, 1, 2, or 3; in some specific embodiments, each r is independently selected from 0, 1, or 2; and in some specific embodiments, each r is independently selected from 0 or 1.

In some specific embodiments, $R^{31a}$ and $R^{41a}$ are each independently substituted with a group selected from hydrogen, deuterium, amino, hydroxyl, $C_{1-3}$ alkyl, halo $C_{1-3}$ alkyl, deuterated $C_{1-3}$ alkyl, $C_{1-3}$ alkoxy, halo $C_{1-3}$ alkoxy, and deuterated $C_{1-3}$ alkoxy; and in some specific embodiments, $R^{31a}$ and $R^{41a}$ are each independently substituted with a group selected from hydrogen, deuterium, amino, hydroxyl, methyl, ethyl, methoxy, ethoxy, $-CH_2F$, $-CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$.

In some specific embodiments, $R_2$ is selected from $-(CR^{2a}R^{2b})_m-C(O)NR^{21}R^{22}$, $-(CR^{2a}R^{2b})_m-COOR^{23}$, $-(CR^{2a}R^{2b})_m-S(O)_2R^{24}$, $-(CR^{2a}R^{2b})_m-P(O)_2R^{24}$, and - $(CR^{2a}R^{2b})_m$-tetrazol-5-yl; in some specific embodiments, $R_2$ is selected from - $(CR^{2a}R^{2b})_m-C(O)NR^{21}R^{22}$ and $-(CR^{2a}R^{2b})_m-COOR^{23}$; in some specific embodiments, $R_2$ is selected from $-(CR^{2a}R^{2b})_m-COOR^{23}$; in some specific embodiments, $R_2$ is $-CR^{2a}R^{2b}-COOR^{23}$;

$R^{2a}$ and $R^{2b}$ are each independently selected from hydrogen, deuterium, F, Cl, methyl, and ethyl;

$R^{23}$ is selected from hydrogen, deuterium, methyl, ethyl, propyl, or tert-butyl.

In some specific embodiments, $R^{2a}$ and $R^{2b}$ are each independently selected from hydrogen, deuterium, F, Cl, $C_{1-3}$ alkyl, halo $C_{1-3}$ alkyl, and deuterated $C_{1-3}$ alkyl, or $R^{2a}$ and $R^{2b}$ together with the carbon atom to which they are attached form 3-membered cycloalkyl, 4-membered cycloalkyl, or 4-membered heterocycloalkyl; in some specific embodiments, $R^{2a}$ and $R^{2b}$ are each independently selected from hydrogen, deuterium, F, Cl, methyl, ethyl, $-CH_2F$, $-CHF_2$, $-CF_3$, $-CH_2D$, $-CHD_2$, and $-CD_3$, or $R^{2a}$ and $R^{2b}$ together with the carbon atom to which they are attached form cyclopropyl or cyclopentyl; and in some specific embodiments, $R^{2a}$ and $R^{2b}$ are each independently selected from hydrogen, deuterium, F, and methyl, or $R^{2a}$ and $R^{2b}$ together with the carbon atom to which they are attached form cyclopropyl.

In some specific embodiments, $R^{21}$ and $R^{22}$ are each independently selected from hydrogen, deuterium, and $C_{1-3}$ alkyl, wherein the alkyl is optionally further substituted with deuterium; and in some specific embodiments, $R^{21}$ and $R^{22}$ are each independently selected from hydrogen, deuterium, methyl, ethyl, $-CH_2D$, - $CHD_2$, and $-CD_3$.

In some specific embodiments, $R^{23}$ and $R^{25}$ are each selected from hydrogen, deuterium, $C_{1-6}$ alkyl, and halo $C_{1-6}$ alkyl, wherein the alkyl is optionally further substituted with deuterium; in some specific embodiments, $R^{23}$ and $R^{25}$ are each selected from hydrogen, deuterium, $C_{1-3}$ alkyl, and halo $C_{1-3}$ alkyl, wherein the alkyl is optionally further substituted with deuterium; and in some specific embodiments, $R^{23}$ and $R^{25}$ are each selected from hydrogen, deuterium, methyl, ethyl, $-CH_2F$, $-CHF_2$, and $-CF_3$.

In some specific embodiments, each $R^{24}$ is independently selected from hydrogen, deuterium, hydroxyl, $C_{1-6}$ alkyl, and $-NHC_{1-6}$ alkyl, wherein the alkyl is optionally further substituted with deuterium; in some specific embodiments, each $R^{24}$ is independently selected from hydrogen, deuterium, hydroxyl, $C_{1-3}$ alkyl, and - $NHC_{1-3}$ alkyl, wherein the alkyl is optionally further substituted with deuterium; and in some specific embodiments, each $R^{24}$ is independently selected from hydrogen, deuterium, hydroxyl, methyl, ethyl, and $-NHCH_3$.

In some specific embodiments, each m is independently selected from 0, 1, 2, 3, or 4; in some specific embodiments, each m is independently selected from 0, 1, 2, or 3; in some specific embodiments, each m is independently selected from 0, 1, or 2; in some specific embodiments, each m is independently selected from 0 or 1; and in some specific embodiments, each m is independently selected from 1.

In some specific embodiments, each $R^A$ is independently selected from deuterium, F, cyano, hydroxyl, amino, methyl, ethyl, methoxy, ethoxy, $-CH_2F$, - $CHF_2$, $-CF_3$, $-OCH_2F$, $-OCHF_2$, $-OCF_3$, $-CH_2D$, $-CHD_2$, $-CD_3$, $-OCH_2D$, $-OCHD_2$, and $-OCD_3$.

In some specific embodiments, rings $F_1$, $F_2$, and $F_3$ are heteroaryl.

In some specific embodiments, ring C is selected from 5-membered cycloalkyl, 6-membered cycloalkyl, 5-membered heterocycloalkyl, 6-membered heterocycloalkyl, and 5-membered heteroaryl; and in some specific embodiments, ring C is selected from 5-membered cycloalkyl, 6-membered cycloalkyl, 6-membered heterocycloalkyl, and 5-membered heteroaryl.

In some specific embodiments, ring D is selected from 5-membered cycloalkyl, 6-membered cycloalkyl, 5-membered heteroaryl, and 6-membered heteroaryl; and in some specific embodiments, ring D is selected from 5-membered heteroaryl.

In some specific embodiments, ring E is selected from 5-membered heterocycloalkyl.

In some specific embodiments, ring G is selected from 5-membered cycloalkyl and 6-membered cycloalkyl; and in some specific embodiments, ring G is selected from phenyl, 5-membered heteroaryl, or 6-membered heteroaryl.

In some specific embodiments, ring B is selected from the following groups:

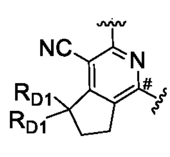 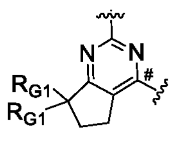 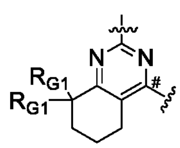 .

In the preceding compounds, # represents a connection site of ring B to ring A; and * represents a connection site of ring A to $R_2$.

In the preceding compounds, no particular mark indicates that any position can be used as a connection site.

The present invention provides the compound, or the stereoisomer, deuterated compound, solvate, prodrug, metabolite, pharmaceutically acceptable salt or eutectic thereof, wherein the compound has a structure selected from:

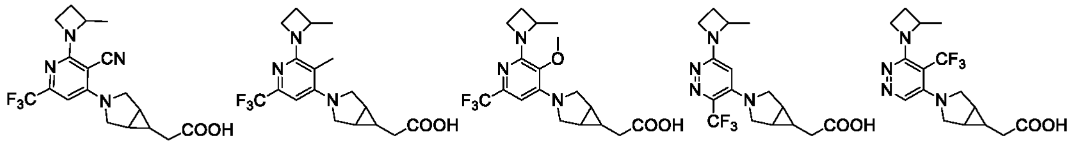

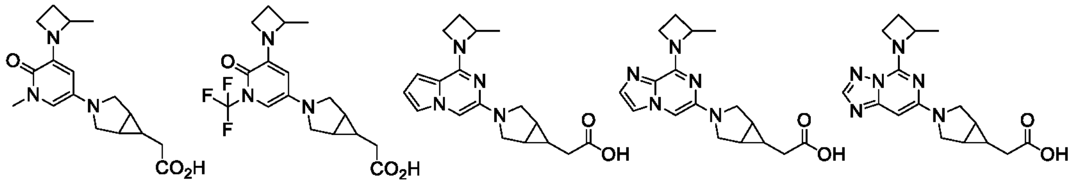

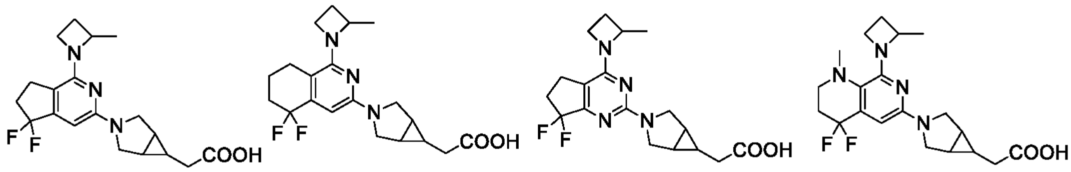

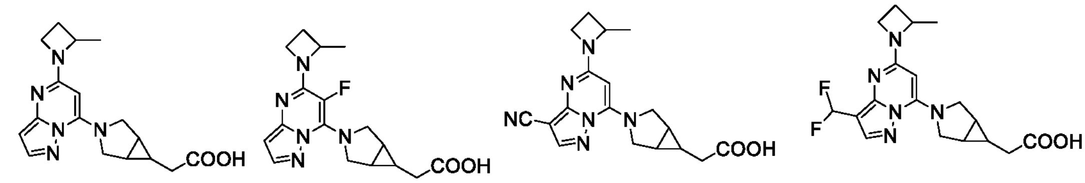

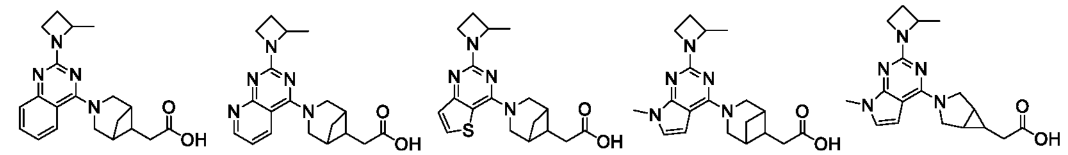

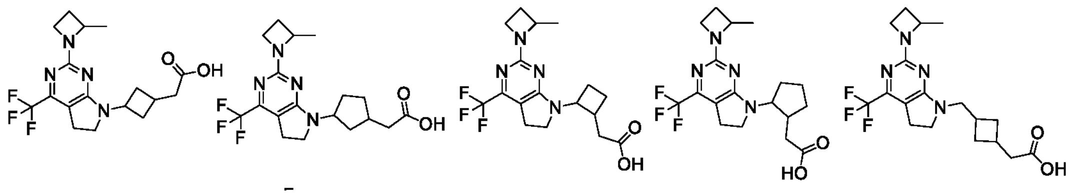

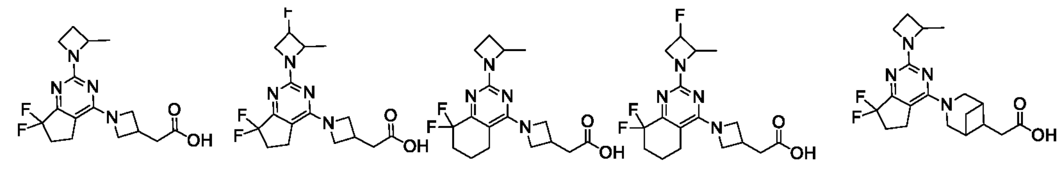

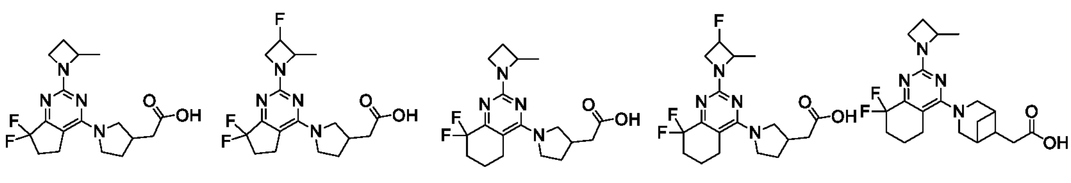

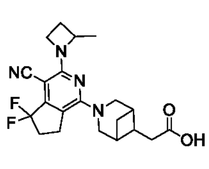 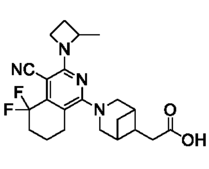 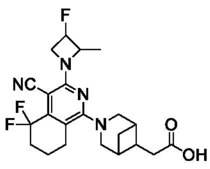 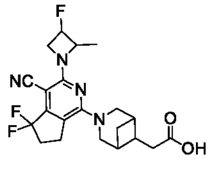 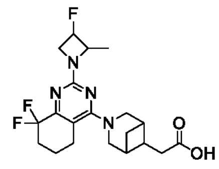

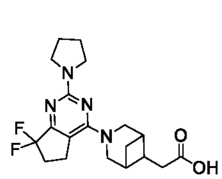 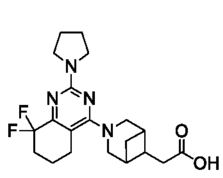 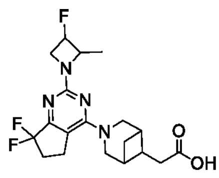 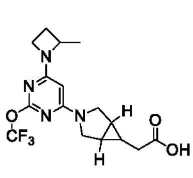 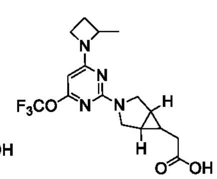

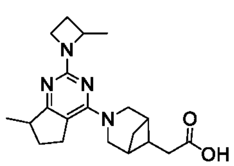 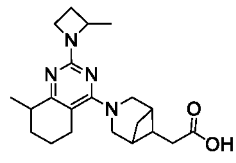 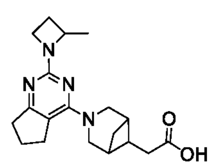 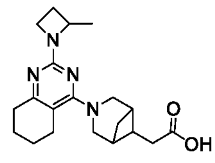

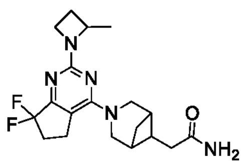 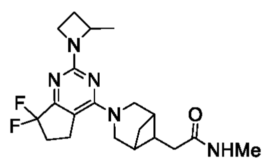 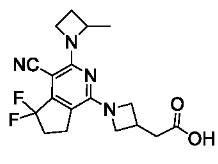 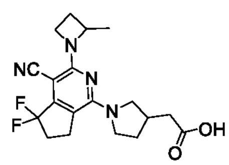

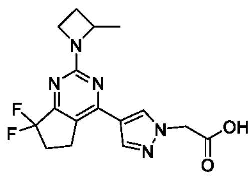 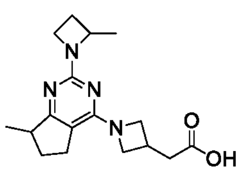 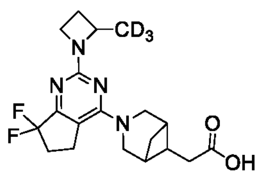 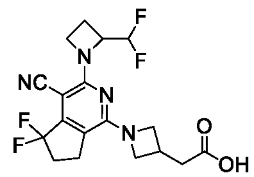

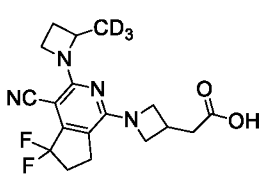 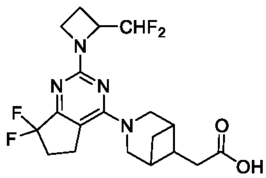 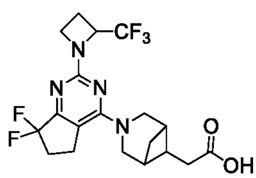 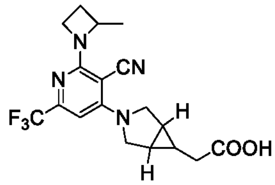

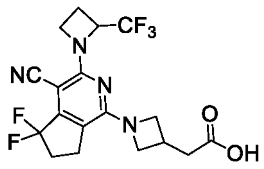 .

The present invention provides the compound, or the stereoisomer, deuterated compound, solvate, prodrug, metabolite, pharmaceutically acceptable salt or eutectic thereof, wherein the compound has a structure selected from:

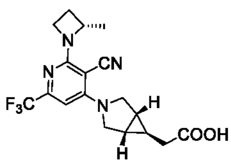 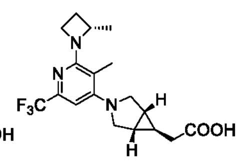 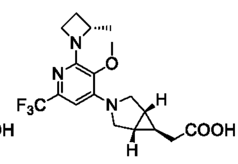 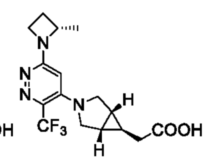 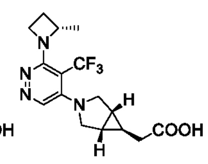

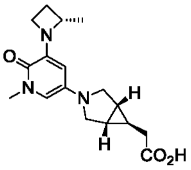
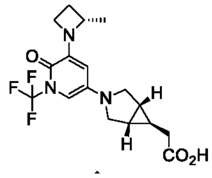
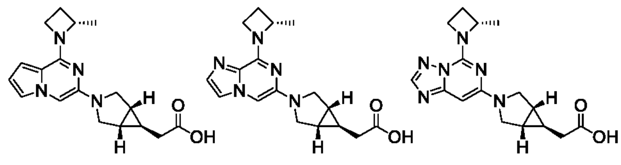

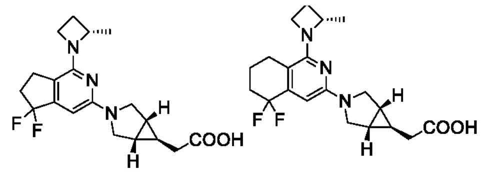
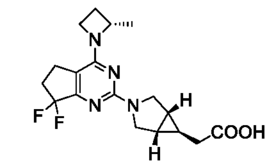
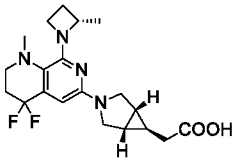

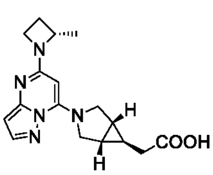
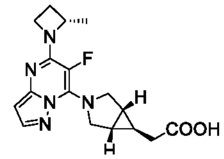
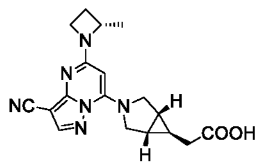
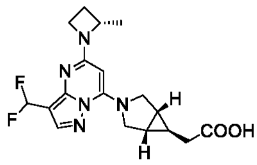

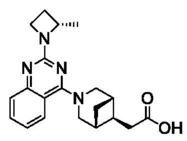
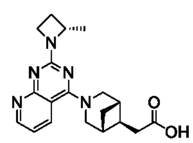
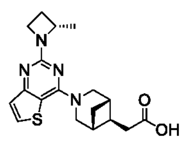
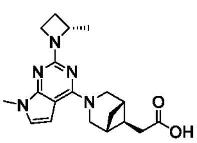
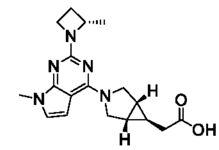

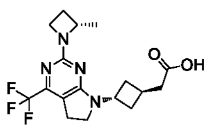
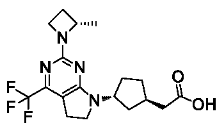
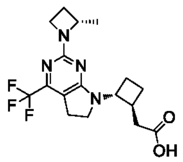
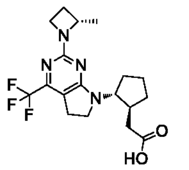
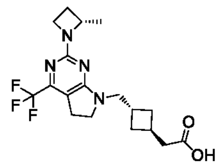

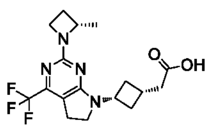
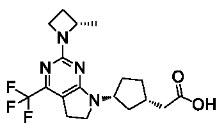
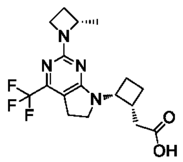
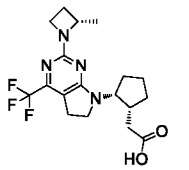
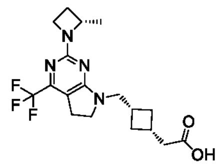

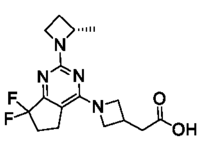
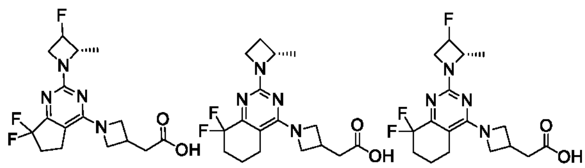
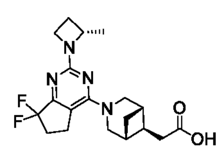

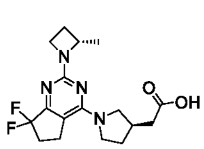
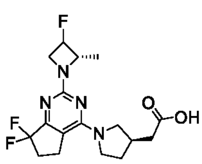
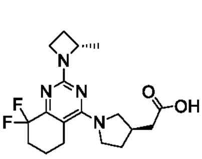
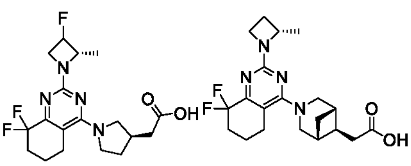

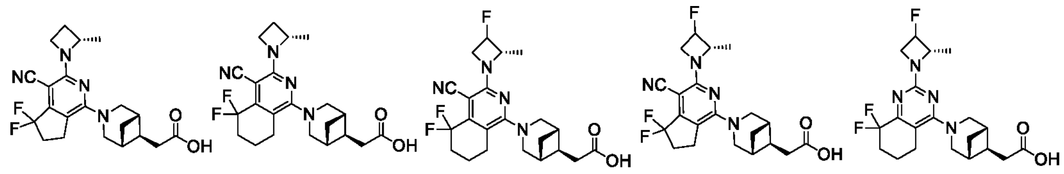

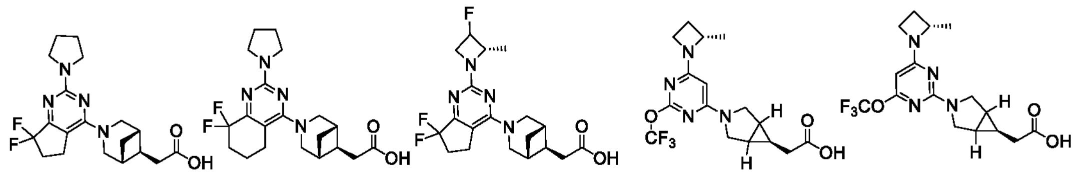

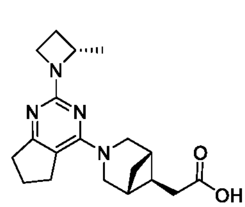
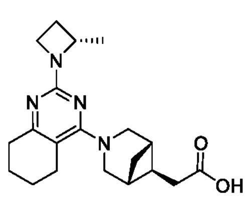
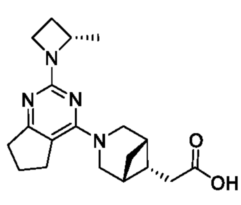
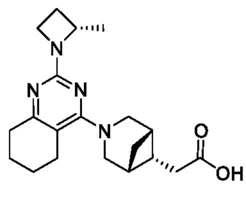

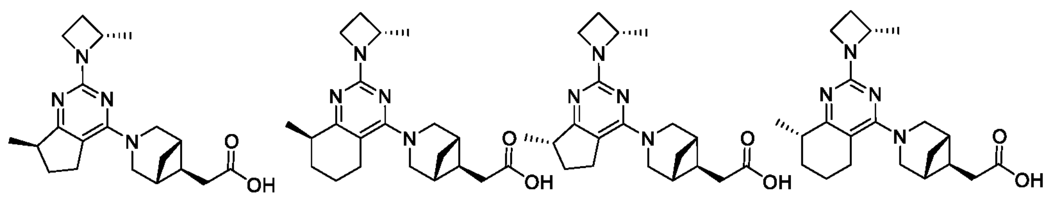

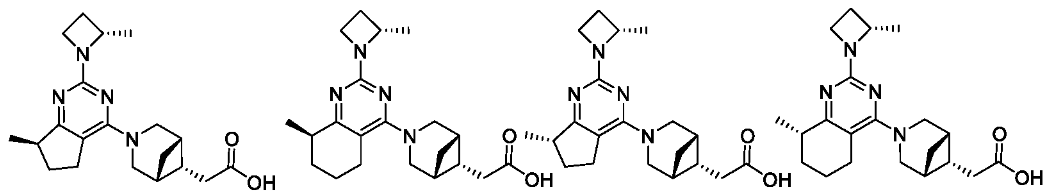

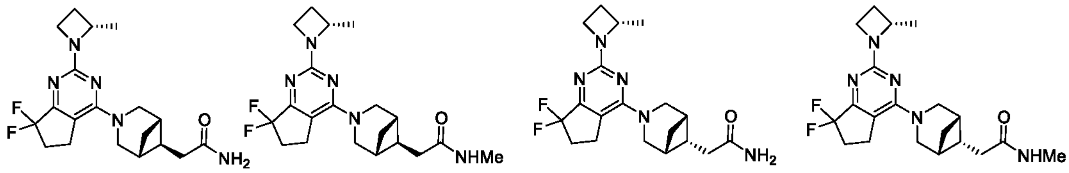

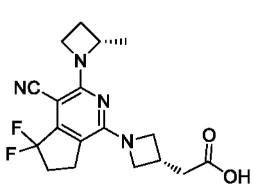
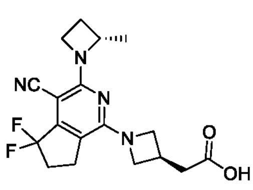
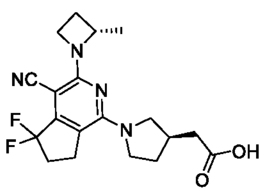
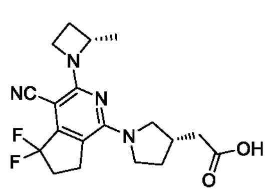

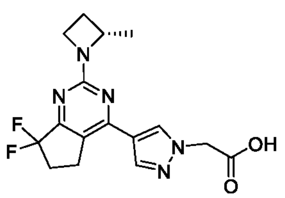

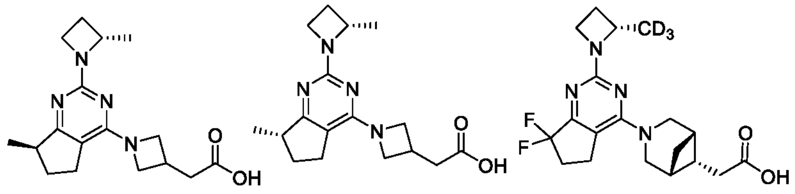

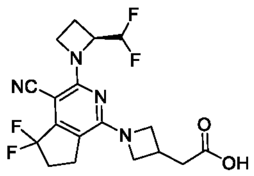

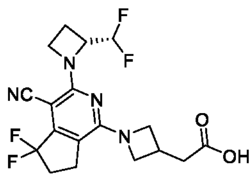

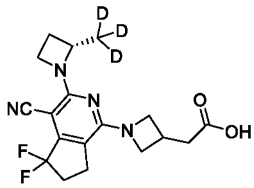

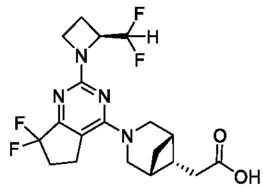

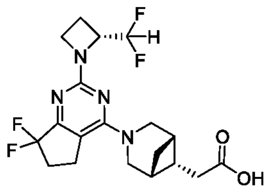

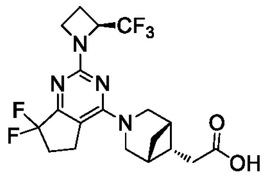

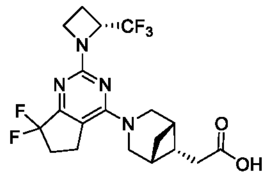

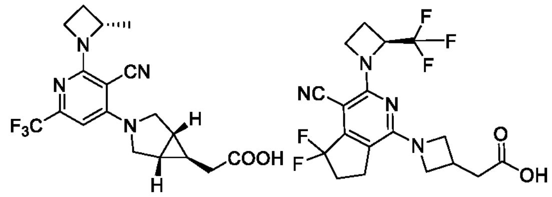

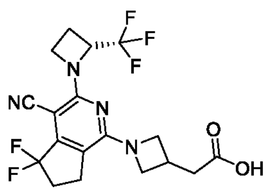.

The present invention also provides a pharmaceutical composition, characterized by comprising the compound, or the stereoisomer, deuterated compound, solvate, prodrug, metabolite, pharmaceutically acceptable salt or eutectic thereof according to the present invention, and a pharmaceutically acceptable carrier and/or excipient.

The present invention provides the use of the compound, or the stereoisomer, deuterated compound, solvate, prodrug, metabolite, pharmaceutically acceptable salt or eutectic thereof or the composition in the preparation of a drug for treating a KHK-mediated disease, wherein the KHK-mediated disease is non-alcoholic fatty liver disease.

**Synthetic Route**

Those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific, etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al.,

"Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalogue are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The expression "$C_{x-y}$ group" refers to a group comprising x to y carbon atoms, for example, "$C_{1-6}$ alkyl" refers to an alkyl group comprising 1-6 carbon atoms.

The term "halogen" refers to fluorine (F), chlorine (Cl), bromine (Br), iodine (I) or isotopes thereof.

The term "halo" or "substituted with halogen" means that the hydrogen atoms are substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen.

The term "halo $C_{1-6}$ alkyl" refers to an alkyl group comprising 1-6 carbon atoms in which one or more hydrogens are substituted with one or more halogen atoms (e.g., fluorine, chlorine, bromine, and iodine), wherein the upper limit of the number of halogen substituents is equal to the sum of the number of hydrogens that can be substituted in the alkyl group. Without particular limitation, the number of halogen substituents is any integer between 1 and the upper limit, preferably 1-5 halogen, 1-3 halogen, 1-2 halogen, or 1 halogen; and when the number of halogen substituents is greater than 1, the group to be substituted can be substituted with the same or different halogen. Examples include, but are not limited to $-CF_3$, $- CH_2Cl$, $-CH_2CF_3$, $-CCl_2$, $CF_3$, etc.

The term "deuterium" refers to the isotope deuterium of hydrogen (H).

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, 1-10 deuterium atoms, 1-6 deuterium atoms, 1-3 deuterium atoms, 1-2 deuterium atoms or 1 deuterium atom.

The term "alkyl" refers to a straight or branched saturated aliphatic hydrocarbon group. Unless otherwise specified, the alkyl refers to an alkyl group comprising 1 to 20 carbon atoms, preferably an alkyl group comprising 1 to

8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples of alkyl include methyl, ethyl, n-propyl, isopropyl, n-butyl, sec-butyl, neobutyl, tert-butyl, n-pentyl, isoamyl, neopentyl, n-hexyl, etc. The alkyl can be further substituted with any substituent.

The term "alkenyl" refers to a straight or branched hydrocarbon group comprising at least one carbon-carbon double bond (C=C), and comprises 2 to 18 (such as 2 to 8, further such as 2 to 6, and more further such as 2 to 4) carbon atoms unless otherwise specified. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl can be optionally further substituted with any group.

The term "alkynyl" refers to a straight or branched hydrocarbon group containing at least one carbon-carbon triple bond (C=C). The main chain comprises 2 to 18 (such as 2 to 8, further such as 2 to 6, and more further such as 2 to 4) carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc. The alkynyl can be optionally further substituted with any substituent.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples of alkoxy or alkyloxy include methoxy, ethoxy, n-propoxy, isopropoxy, n-butoxy, secbutoxy, tert-butoxy, n-pentoxy, n-hexyloxy, cyclopropoxy, cyclobutoxy, etc. The alkoxy can be optionally further substituted with any substituent.

The term "haloalkoxy" refers to -O-haloalkyl. Without particular limitation, haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, etc.

The term "cycloalkyl" refers to a substituted or unsubstituted, saturated, partially unsaturated or fully unsaturated non-aromatic hydrocarbon ring. Cycloalkyl can be a monocyclic, bicyclic or polycyclic ring, wherein the bicyclic or polycyclic ring can be a fused ring, a spiro ring or a bridged ring. Unless otherwise specified, cycloalkyl usually contains 3 to 20 carbon atoms. When cycloalkyl is monocyclic cycloalkyl, the cycloalkyl contains preferably 3-15 carbon atoms, preferably 3-10 carbon atoms, also preferably 3-8 carbon atoms, more preferably 3-6 carbon atoms, and further preferably 3-4 carbon atoms; and when cycloalkyl is bicyclic or polycyclic cycloalkyl, the cycloalkyl contains preferably 4-12 carbon atoms, preferably 4-11 carbon atoms, also preferably 5-11 carbon atoms, more preferably 6-11 carbon atoms, and further preferably 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

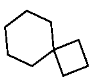, 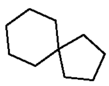, 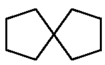, 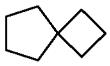, 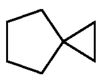, 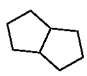, 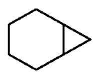,

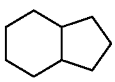, 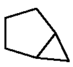, 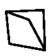, 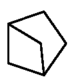, 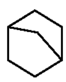,

etc.

The term "heterocycloalkyl" refers to a substituted or unsubstituted, saturated, partially unsaturated or fully unsaturated non-aromatic ring containing at least one heteroatom. Unless otherwise specified, heterocycloalkyl is a 3- to 20-membered ring. When heterocycloalkyl is monocyclic heterocycloalkyl, the heterocycloalkyl is preferably a 3- to 15-membered, preferably 3-10-membered, also preferably 3-8-membered, and further preferably 3-6-membered ring; and when heterocycloalkyl is bicyclic or polycyclic heterocycloalkyl, the heterocycloalkyl is preferably a 4-12-membered, preferably 4-11-membered, also preferably 5-11-membered, more preferably 6-11-membered, and further preferably 6-10-membered ring. Heterocycloalkyl can be a monocyclic, bicyclic or polycyclic ring, wherein the bicyclic or polycyclic ring can be a bridged ring, a fused ring and a spiro ring, in which the heteroatoms are selected from heteroatoms N, S, O, P and Si and oxidation states thereof. When heterocycloalkyl is a bicyclic or polycyclic ring, at least one ring contains

at least one heteroatom, and the heterocycloalkyl can be a bicyclic or polycyclic ring formed by a ring containing the heteroatom(s) and a ring containing no heteroatom. When heterocycloalkyl is connected to other groups, a connection point can be at a heteroatom or a carbon atom. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc.

The term "aryl" refers to a substituted or unsubstituted aromatic 6- to 15-membered carbocycle, and includes monocyclic aryl and fused aryl. Aryl is preferably a 6- to 10-membered aromatic ring, and further preferably a 6- to 8-membered aromatic ring. Aryl ring can be fused to a non-aryl ring (such as a heteroaryl, heterocycloalkyl, or cycloalkyl ring), wherein the aryl ring is the connection site. The "x-y-membered aryl" means that the aryl has a total number of ring atoms of x to y, and can be a phenyl fused non-aromatic ring in which the ring having aromatic character is the connection site. For example, "7-12-membered aryl" represents that the aryl, as the connection site, has a total number of ring atoms of 7-12, e.g., benzocyclobutyl and benzocyclopentyl. Non-limiting examples of aryl include phenyl, naphthyl, anthryl, phenanthryl,

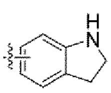, 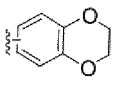, 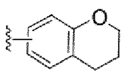, 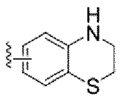, 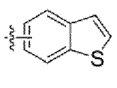, 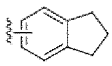, 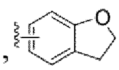, 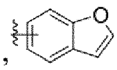,

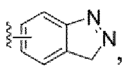, 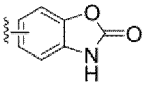, 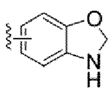, 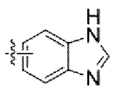, 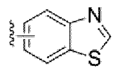 i¢ 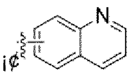 and 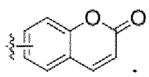.

The aryl can be optionally further substituted with any substituent.

The term "heteroaryl ring" or "heteroaryl" refers to a substituted or unsubstituted monocyclic ring (having aromatic character), or bicyclic or polycyclic ring (in which the ring of the connection site is an aromatic ring) containing at least one heteroatom or group selected from heteroatoms N, S, O, P and Si and oxidation states thereof, which can be a bridged ring, a fused ring, or a spiro ring. Bicyclic or polycyclic heteroaryl ring or heteroaryl can be formed by fusion of heteroaryl to a non-heteroaryl ring such as cycloalkyl, heterocycloalkyl and aryl, or of heteroaryl to heteroaryl, wherein the heteroaryl ring is the connection site. The "x-y-membered heteroaryl" means that the heteroaryl has a total number of ring atoms of x to y, which can be 5-6-membered heteroaryl, and can also be 5-6-membered heteroaryl fused to other rings (e.g., cycloalkyl, heterocycloalkyl, and aromatic rings) in which the heteroaromatic ring is the connection site. For example, "5-12-membered heteroaryl" represents that heteroaryl, as the connection site, has a total number of ring atoms of 5-12, e.g., pyridocyclobutyl and pyridocyclopentyl. Non-limiting examples of heteroaryl ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

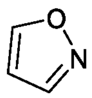,

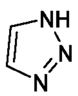, 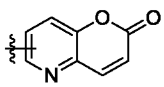,

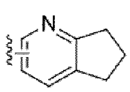, 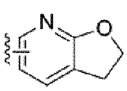, 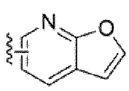, 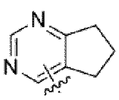, 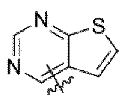, 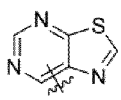, 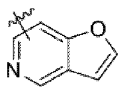,

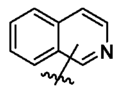, 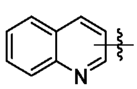,

etc. The heteroaryl can be optionally further substituted with any substituent.

The term "carboxyl" refers to -C(=O)-OH.

"Spiro ring" refers to a 5- to 20-membered polycyclic group sharing one carbon atom (referred to as a spiro atom) between substituted or unsubstituted rings, which may contain 0 to 5 double bonds, and may contain 0 to 5 heteroatoms or groups selected from N, O, S, P, Si and oxidation states thereof. The spiro ring is preferably 6- to 14-membered, further preferably 6- to 12-membered, and more preferably 6- to 10-membered. The spiro ring can be formed between cycloalkyl and heterocycloalkyl; preferably a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring; and non-limiting examples include

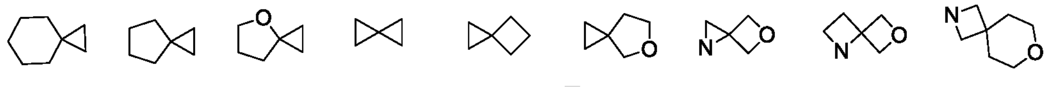

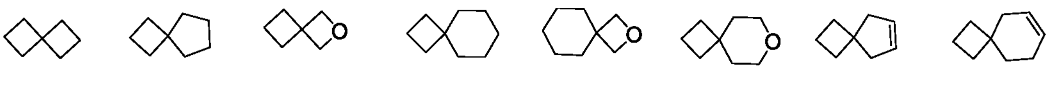

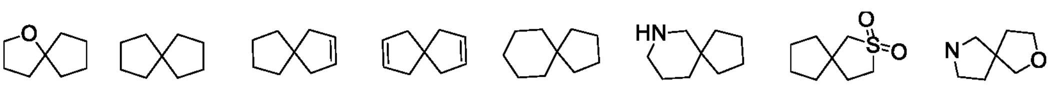

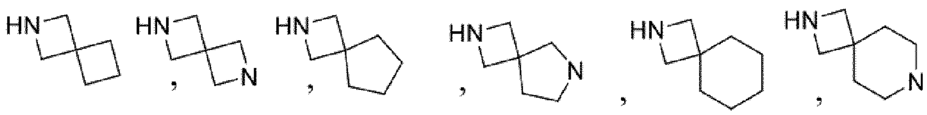
, , , , ,

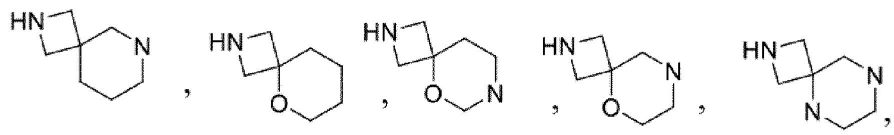
, , , , ,

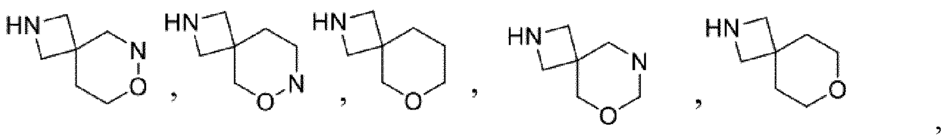
, , , , ,

wherein the spiro ring can be optionally further substituted with any substituent.

A "fused ring" refers to a polycyclic group in which the rings share two adjacent atoms, wherein one or more rings may contain 0 or more double bonds, which may be substituted or unsubstituted, and each ring in the fused ring system may contain 0 to 5 heteroatoms selected from N, S, O, P, Si and oxidation states thereof. The fused ring is preferably 5- to 20-membered, further preferably 5- to 14-membered, more preferably 5- to 12-membered, and still further preferably 5-to 10-membered. Preferably, the fused ring may be in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring; and non-limiting examples include purine, quinoline, isoquinoline, benzopyran, benzofuran, benzothiophene,

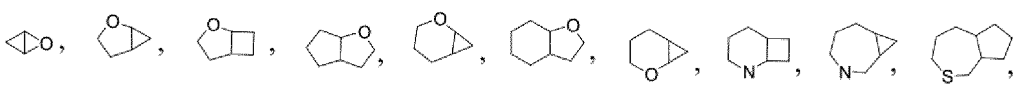
, , , , , , , , , ,

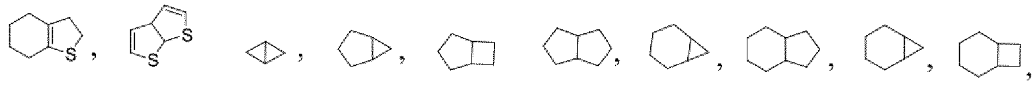

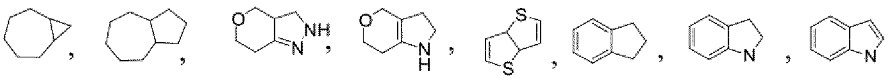

The fused ring can be optionally further substituted with any substituent.

A "bridged ring" refers to a ring system in which two rings share two non-adjacent atoms, which may contain 0 or more double bonds, and may be substituted or unsubstituted, wherein one or more rings may contain 0 to 5 heteroatoms selected from N, S, O, P, Si and oxidation states thereof. The ring atoms contain 5 to 20 atoms, preferably 5 to 14 atoms, further preferably 5 to 12 atoms, and still further preferably 5 to 10 atoms; and non-limiting examples include adamantane,

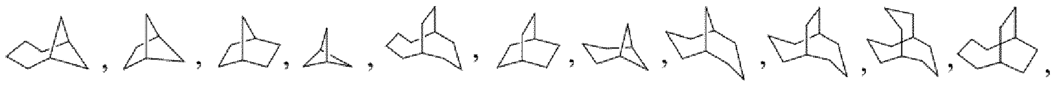

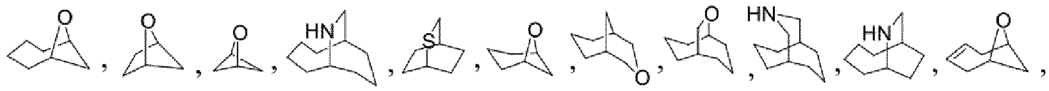

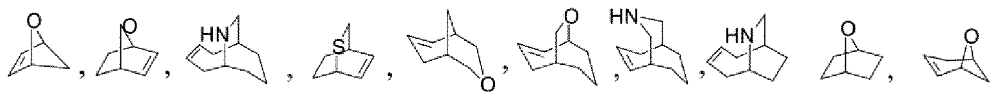

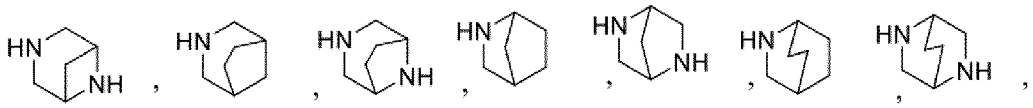

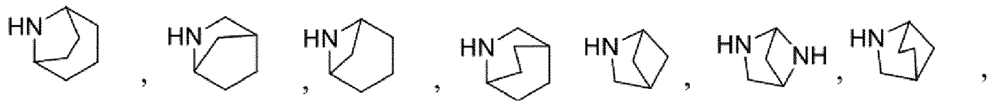

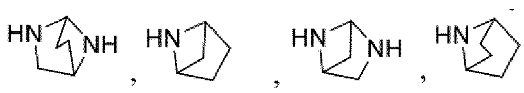

The term "optional" or "optionally" refers to that the event or circumstance subsequently described may but not necessarily occur, and the description includes the occasions where the events or circumstances occur or do not occur. For example, "alkyl optionally substituted with F" means that alkyl may but not necessarily be substituted with F, and the description includes the case where the alkyl is substituted with F and the case where the alkyl is not substituted with F.

Unless otherwise specified, substitution with a substituent described herein refers to substitution at a position allowed by chemical theory, and the number of substituents conforms to the rules of chemical bonding.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts, eutectics or deuterated compounds thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome,

etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "eutectic" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The eutectic is a multi-component crystal, which includes both a binary eutectic formed between two neutral solids and a multi-element eutectic formed between a neutral solid and a salt or solvate.

## Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the present invention, those skilled in the art can make unsubstantial modifications and adjustments to the embodiments, which still fall within the scope of protection of the present invention.

### Test Method

The structures of the compounds are determined by nuclear magnetic resonance (NMR) or (and) mass spectrometry (MS). The NMR shift (δ) is given in the unit of 10-6 (ppm). NMR is measured with (Bruker Avance III 400 and Bruker Avance 300) NMR instrument, and the solvent for determination is deuterated dimethyl sulfoxide (DMSO-$d_6$), deuterated chloroform ($CDCl_3$), and deuterated methanol ($CD_3OD$), and the internal standard is tetramethylsilane (TMS);

MS is measured with (Agilent 6120B(ESI) and Agilent 6120B(APCI));
HPLC is measured with Agilent 1260DAD high pressure liquid chromatography (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm - 0.20 mm, and the specification when separating and purifying a product by thin layer chromatography is 0.4 mm - 0.5 mm.
For the column chromatography, Yantai Huanghai silica gel of 200-300 mesh silica gel is generally used as a carrier.

Abbreviations:

NMP: N-methylpyrrolidone
DIPEA: N,N-diisopropylethylamine
TFA: trifluoroacetic acid
DBN: 1,5-diazabicyclo[4.3.0]-5-nonene

DAST: diethylaminosulphur trifluoride
DEA: diethylamine
**INT-2:**
2,4-dichloro-7,7-difluoro-6,7-dihydro-5H-cyclopenta[d]pyrimidine **(INT-2)**

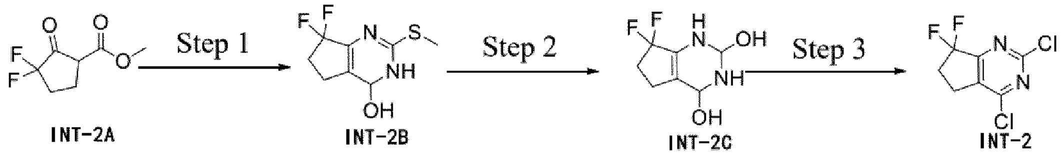


INT-2A INT-2B INT-2C INT-2

Step 1: 7,7-difluoro-2-(methylthio)-4,5,6,7-tetrahydro-3H-cyclopenta[d]pyrimidin-4-ol **(INT-2B)**

**INT-2A** (4.0 g, 22.5 mmol) was dissolved in water (50 mL), and methylisothiourea (3.9 g, 27 mmol) and sodium carbonate (4.7 g, 45.0 mmol) were added. The mixture was stirred at room temperature and reacted for 16 h. The mixture was adjusted to pH = 5-6 with 1 N hydrochloric acid, extracted with ethyl acetate (50 mL × 3), dried over anhydrous sodium sulphate and concentrated to obtain the crude product (**INT-2B**) (5 g, yield: 100%).
LCMS m/z = 221.2 [M+1].

Step 2: 7,7-difluoro-2,3,4,5,6,7-hexahydro-1H-cyclopenta[d]pyrimidine-2,4-diol (**INT-2C**)

**INT-2B** (5 g, 22.5 mmol) was dissolved in ethanol (40 mL), and 6 N hydrochloric acid (40 mL) was added. The mixture was reacted at 85°C for 16 h, and then concentrated to obtain the product **INT-2C** (4.5 g, yield: 100%).
LCMS m/z = 193.1 [M+1].

Step 3: 2,4-dichloro-7,7-difluoro-6,7-dihydro-5H-cyclopenta[d]pyrimidine (**INT-2**)

**INT-2C** (4.5 g, 22.5 mmol) was added to phosphorus oxychloride (100 mL), and then triethylamine (10 mL) was added. The mixture was reacted at 110°C for 16 h, subjected to rotary evaporation and then purified by column chromatography (petroleum ether:ethyl acetate = 5: 1) to obtain the product **INT-2** (1.7 g, yield: 100%).
LCMS m/z = 225.1 [M+1].

**INT-3:** 2-(methyl-d3)azetidine trifluoroacetate (INT-3)

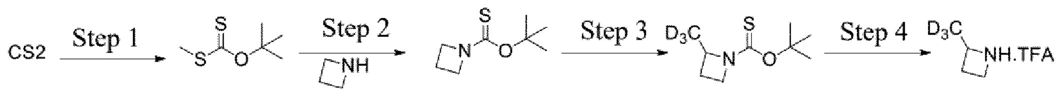


INT-3A INT-3B INT-3C INT-3D INT-3E INT-3

Step 1: O-(tert-butyl) S-methyl carbonodithioate (INT-3B)

Potassium tert-butoxide (73.7 g, 0.66 mol) was added to xylene (2 L). The mixture was warmed to 80°C, and then carbon disulphide (50 g, 0.66 mol) was added dropwise. The mixture was reacted at 80°C for 1 h, cooled to room temperature, and filtered. The solid was washed with diethyl ether and dried to obtain 100 g of a pale yellow solid, which was added to diethyl ether (1 L), and iodomethane (113 g, 0.796 mol) was added. The mixture was reacted at room temperature for 20 h and filtered, the solid was washed with diethyl ether, and the filtrate was concentrated under reduced pressure to obtain the title compound INT-3B (65 g, 60%).

Step 2: O-(tert-butyl) azetidine-1-carbothioate (INT-3D)

INT-3B (63 g, 0.385 mol) was added to n-hexane (600 mL), and INT-3C was added dropwise in an ice bath. The mixture was reacted at 0°C for 2 h. The reaction solution was directly concentrated under reduced pressure, and then the residue was separated and purified by silica gel column chromatography (EA: PE (v/v) = 5: 95) to obtain the title compound INT-3D (60 g, 90%).
LC-MS (ESI): m/z = 174.1 $[M+H]^+$.

Step 3: O-(tert-butyl) 2-(methyl-d3)azetidine-1-carbothioate (INT-3E)

INT-3D (15 g, 86.57 mmol) was added to tetrahydrofuran (400 mL), and the mixture was cooled to -78°C. N,N,N',N'-tetramethylethylenediamine (24.14 g, 207.77 mmol) was added, sec-butyllithium (166 mL, 216 mmol) was slowly added dropwise at -78°C, and the mixture was stirred at -78°C for 30 min. Deuterated iodomethane (37.65 g, 259.71 mmol) was slowly added dropwise (strong exothermicity), and the mixture was reacted at -78°C for 30 min. The reaction was quenched with an aqueous ammonium chloride solution and extracted with ethyl acetate. The organic phases were combined, washed with a saturated sodium chloride solution, dried over anhydrous sodium sulphate and filtered. The filtrate was concentrated under reduced pressure, and then the residue was separated and purified by silica gel column chromatography (EA: PE (v/v) = 4: 96) to obtain the title compound INT-3E (8 g, 48%).

LC-MS (ESI): m/z = 191.1 $[M+H]^+$.

Step 4: 2-(methyl-d3)azetidine trifluoroacetate (INT-3)

INT-3E (8 g, 42 mmol) was added to dichloromethane (50 mL), and then trifluoroacetic acid (30 mL) was added. The mixture was reacted at room temperature for 3 h, and the reaction solution was directly concentrated to dryness under reduced pressure to obtain the title compound INT-3 (10 g).

1H NMR (400 MHz, CDCl3) δ 4.47-4.39 (m, 1H), 3.98-3.87 (m, 1H), 3.85-3.71 (m, 1H), 2.48- 2.39 (m, 1H), 2.16-2.09(m, 1H).

**Example 1:**

(S)-2-(1-(8,8-difluoro-2-(2-methylazetidin-1-yl)-5,6,7,8-tetrahydroquinazolin-4-yl)azetidin-3-yl)acetic acid (**compound 1**)

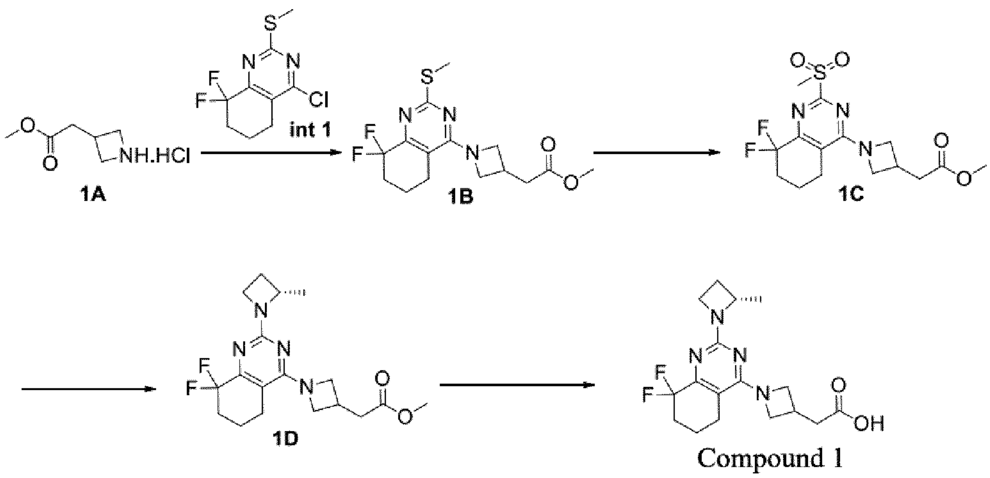


Step 1:

methyl 2-(1-(8,8-difluoro-2-(methylthio)-5,6,7,8-tetrahydroquinazolin-4-yl)azetidin-3-yl)acetate (**1B**)

Compound **1A** (0.48 g, 2.9 mmol) and **intermediate int-1** (synthesized according to WO 2020156445A1) (0.61 g, 2.44 mmol) were added successively to NMP (20 mL). The mixture was stirred uniformly, and then potassium carbonate (1.44 g, 10.4 mmol) was added. The system was warmed to 90°C and reacted for 4 h. The reaction was quenched by adding water (50 mL) and extracted with ethyl acetate (80 mL × 2). The organic phases were combined, washed with saturated brine (60 mL × 1), dried over anhydrous sodium sulphate and filtered. The filtrate was concentrated under reduced pressure, and the residue was separated by silica gel column chromatography (petroleum ether: ethyl acetate (v/v) = 2: 1) to obtain the target compound **1B** (0.58 g, yield: 48%).

LCMS m/z = 344.1 $[M +1]^+$.

Step 2:

methyl 2-(1-(8,8-difluoro-2-(methylsulfonyl)-5,6,7,8-tetrahydroquinazolin-4-yl)azetidin-3-yl)acetate (**1C**)

Compound **1B** (0.58 g, 1.69 mmol) was dissolved in dichloromethane (10 mL). The mixture was stirred uniformly, and then m-chloroperoxybenzoic acid (1.46 g, 6.76 mmol, 80% wt) was added in portions. The mixture was reacted at room temperature for 2 h. The reaction was quenched by adding saturated sodium bicarbonate (50 mL) and extracted with ethyl acetate (100 mL × 2). The organic phases were combined, washed with saturated brine (80 mL × 1), dried over anhydrous sodium sulphate and filtered. The filtrate was concentrated under reduced pressure, and the residue was separated by silica gel column chromatography (petroleum ether: ethyl acetate (v/v) = 1: 2) to obtain the target compound **1C** (0.54 g, yield: 85%).

LCMS m/z = 376.1 $[M + 1]^+$.

Step 3:

methyl (S)-2-(1-(8,8-difluoro-2-(2-methylazetidin-1-yl)-5,6,7,8-tetrahydroquinazolin-4-yl)azetidin-3-yl)acetate (**1D**)

Compound **1C** (0.54 g, 1.44 mmol) and (2S)-2-methylazetidine hydrochloride (0.23 g, 2.15 mmol) were successively added to NMP (20 mL), and the mixture was stirred uniformly. Then DIPEA (0.26 g, 3.0 mmol) was added, and the system was warmed to 100°C and reacted overnight. The reaction was quenched by adding water (40 mL) and extracted with ethyl acetate (80 mL × 2). The organic phases were combined, washed with saturated brine (60 mL × 1), dried over anhydrous sodium sulphate and filtered. The filtrate was concentrated under reduced pressure, and the residue was separated by silica gel column chromatography (petroleum ether: ethyl acetate (v/v) = 1: 2) to obtain the target compound **1D** (0.22 g, yield: 33%).

LCMS m/z = 367.1 $[M + 1]^+$.

Step 4:

(S)-2-(1-(8,8-difluoro-2-(2-methylazetidin-1-yl)-5,6,7,8-tetrahydroquinazolin-4-yl)azetidin-3-yl)acetic acid (**compound 1**)

Compound **1D** (0.22 g, 0.6 mmol) was dissolved in tetrahydrofuran (10 mL). Methanol (3 mL) and water (3 mL) were added, and the mixture was stirred uniformly. Then lithium hydroxide monohydrate (0.12 g, 3 mmol) was added, and the mixture was reacted at room temperature for 1 h. Water (20 mL) was added, and the pH was adjusted to 5 with hydrochloric acid (1 N aqueous solution). The mixture was extracted with ethyl acetate (50 mL × 2). The organic phases were combined, dried over anhydrous sodium sulphate and concentrated, and the residue was subjected to preparative high performance liquid chromatography on silica gel to obtain the trifluoroacetate of **compound 1** (52 mg, yield: 19%).

Preparation conditions: 1. Instrument: Waters 2767 (preparative liquid phase chromatographic instrument); chromatographic column: XBridge@Prep C18(19 mm × 250 mm); 2. The sample was dissolved in DMF and filtered with a 0.45 μm filter to prepare a sample solution; 3. Preparative liquid chromatography conditions: a. composition of mobile phases A and B; mobile phase A: acetonitrile; mobile phase B: water (containing 0.1% TFA); b. gradient elution: mobile phase A: 10%-50%; c. flow rate: 12 mL/min; d. elution time: 20 min, and tR = 12.97 min.

$^1$H NMR (400 MHz, CDCl3) δ 12.46 (s, 2H), 4.95-4.55 (m, 2H), 4.54-4.10 (m, 3H), 4.10-3.70 (m, 2H), 3.20-2.90 (m, 2H), 2.92-2.34 (m, 5H), 2.35-2.05 (m, 2H), 2.05-1.70 (m,3H), 1.57-1.38 (m, 2H).

$^{19}$F NMR (376 MHz, CDCl3) δ -73.57, -93.68.

LCMS m/z = 353.2 $[M+1]^+$.

**Example 2:**

2-((R)-1-(8,8-difluoro-2-((S)-2-methylazetidin-1-yl)-5,6,7,8-tetrahydroquinazolin-4-yl)pyrrolidin-3-yl)acetic acid (**isomer 1 of compound 2**)

2-((S)-1-(8,8-difluoro-2-((S)-2-methylazetidin-1-yl)-5,6,7,8-tetrahydroquinazolin-4-yl)pyrrolidin-3-yl)acetic acid (**isomer 2 of compound 2**)

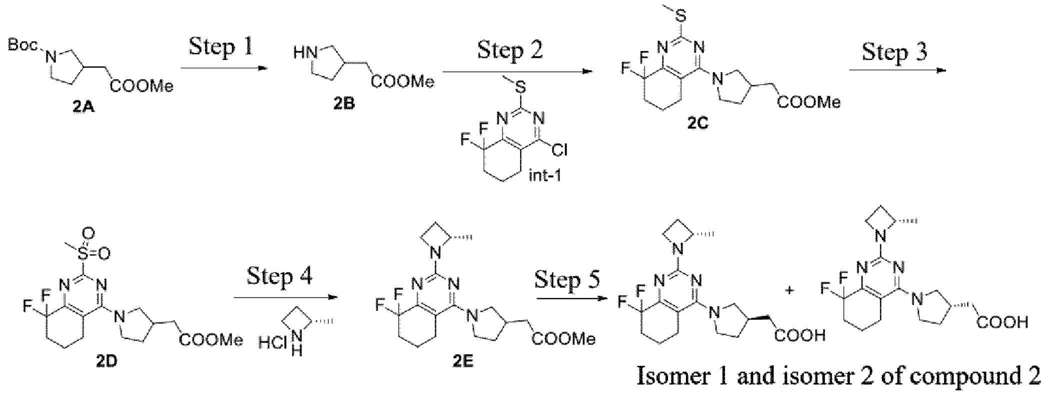

2A 2B 2C int-1 2D 2E

Isomer 1 and isomer 2 of compound 2

Step 1: methyl 2-(pyrrolidin-3-yl)acetate (**2B**)

**2A** (1 g, 4.11 mmol) was dissolved in a solution of hydrochloric acid in dioxane (4 M, 10 mL), and the mixture was reacted at room temperature for 2 h. The reaction solution was concentrated by rotary evaporation under reduced pressure to obtain the title compound **2B** as a crude (0.85 g), which was directly used in the next step without further purification.
LC-MS (ESI): m/z = 144.2 $[M+H]^+$.

Step 2: methyl 2-(1-(8,8-difluoro-2-(methylthio)-5,6,7,8-tetrahydroquinazolin-4-yl) pyrrolidine-3-yl)acetate (**2C**)

**2B** (0.34 g, 2.39 mmol) was dissolved in N-methylpyrrolidone (10 mL), and **int-1** (synthesized according to WO 2020156445A1) (0.5 g, 1.99 mmol) and potassium carbonate (0.55 g, 3.98 mmol) were added. Under nitrogen protection, the mixture was reacted overnight at 90°C and cooled to room temperature. 50 mL of water was added, and the mixture was extracted with ethyl acetate (20 mL × 3). The organic layers were combined, back-flushed with saturated brine (25 mL), dried over anhydrous sodium sulphate and concentrated, and the residue was separated and purified by silica gel column chromatography (petroleum ether: ethyl acetate (v/v) = 4: 1) to obtain the title compound **2C** (0.60 g, 84%).
LC-MS (ESI): m/z = 358.1 $[M+H]^+$.

Step 3: methyl 2-(1-(8,8-difluoro-2-(methylsulfonyl)-5,6,7,8-tetrahydroquinazolin-4-yl) pyrrolidin-3-yl)acetate (**2D**)

**2C** (0.6 g, 1.68 mmol) was dissolved in dichloromethane (15 mL), m-chloroperoxybenzoic acid (1.16 g, 6.71 mmol) was added, and then the mixture was reacted at room temperature overnight. The reaction was quenched by adding 30 mL of saturated sodium bicarbonate solution and extracted with ethyl acetate (20 mL × 3). The organic layers were combined, washed with saturated brine (25 mL), dried over anhydrous sodium sulphate and concentrated, and the residue was separated and purified by silica gel column chromatography (petroleum ether: ethyl acetate (v/v) = 3: 7) to obtain the title compound **2D** (0.31 g, 47%).
LC-MS (ESI): m/z = 390.1 $[M+H]^+$.

Step 4: methyl 2-(1-(8,8-difluoro-2-((S)-2-methylazetidin-1-yl)-5,6,7,8-tetrahydroquinazolin -4-yl)pyrrolidin-3-yl)acetate (**2E**)

**2D** (0.31 g, 0.82 mmol) was dissolved in 1,4-dioxane (5 mL), and (S)-2-methylazetidine hydrochloride (175 mg, 1.64 mmol) and potassium carbonate (0.45 g, 3.28 mmol) were added. Under nitrogen protection, the mixture was reacted at 100°C overnight and cooled to room temperature. 25 mL of water was added, and the mixture was extracted with ethyl acetate (15 mL × 3). The organic layers were combined, dried over anhydrous sodium sulphate and concentrated, and the residue was separated and purified by silica gel column chromatography (petroleum ether: ethyl acetate (v/v) = 2: 1) to obtain the title compound **2E** (0.21 g, 69%).
LC-MS (ESI): m/z = 381.2 $[M+H]^+$.

Step 5: 2-((R)-1-(8,8-difluoro-2-((S)-2-methylazetidin-1-yl)-5,6,7,8-tetrahydroquinazolin-4-yl)pyrrolidin-3-yl)acetic acid (**isomer 1** of compound **2**) 2-((S)-1-(8, 8-difluoro-2-((S)-2-methylazetidin-1-yl)-5,6,7, 8-tetrahydroquinazolin-4-yl)pyrrolidin-3-yl)acetic acid (**isomer 2** of compound **2**)

**2E** (0.21 g, 0.55 mmol) was dissolved in tetrahydrofuran (6 mL), and a solution of lithium hydroxide monohydrate

(116 mg, 2.75 mmol) in water (3 mL) was added dropwise in an ice bath. After the addition was completed, the mixture was reacted at room temperature for 2 h, adjusted to about pH 4 with 2 M hydrogen chloride solution and concentrated, and the residue was separated and purified by silica gel column chromatography (dichloromethane: methanol (v/v) = 10: 1) to obtain the product compound **2** (140 mg, 69%).

Compound **2** was subjected to SFC chiral preparative separation to obtain compound **2-isomer 1** (56 mg, ee% = 99.08%, yield: 27.6%) and compound **2-isomer 2** (52 mg, ee% = 99.62%, yield: 25.6%), with purification conditions as follows: (instrument name: MG II preparative SFC (SFC-14); chromatographic column: ChiralPak AD, 250 × 30 mm I.D., 10 μm; mobile phase: phase A: $CO_2$; phase B: ethanol (0.1% $NH_3 \cdot H_2O$); flow rate: 70 mL/min; column pressure: 100 bar; column temperature: 38°C; absorption wavelength: 220 nm; cycle time: about 3 min); retention time for compound **2-isomer 1:** 3.925 min; retention time for compound **2-isomer 2:** 4.257 min.

Compound **2-isomer 1:**

$^1H$ NMR (400 MHz, $CD_3OD$) δ 4.48-4.37 (m, 1H), 4.06-4.00 (td, *J* = 8.8, 4.8 Hz, 1H), 3.94-3.85 (m, 2H), 3.77-3.68 (m, 2H), 3.39 (dd, *J* = 11.2, 8.0 Hz, 1H), 2.81-2.74 (m, 2H), 2.62-2.51 (m, 1H), 2.43 (d, J = 8.0 Hz, 2H), 2.41-2.32 (m, 1H), 2.32-2.22 (m, 1H), 2.22-2.09 (m, 2H), 1.99-1.86 (m, 2H), 1.78 (br, 1H), 1.69-1.60 (m, 1H), 1.47 (d, J = 6.2 Hz, 3H).

LC-MS (ESI): m/z = 367.2 $[M+H]^+$.

Compound **2-isomer 2:**

$^1H$ NMR (400 MHz, $CD_3OD$) δ 4.49-4.42 (m, 1H), 4.07 (td, J = 8.8, 4.8 Hz, 1H), 3.96-3.87 (m, 2H), 3.83-3.77 (m, 1H), 3.72-3.64 (m, 1H), 3.37 (dd, J = 11.2, 8.0 Hz, 1H), 2.83-2.74 (m, 2H), 2.62-2.53 (m, 1H), 2.43 (d, J = 8.0 Hz, 2H), 2.42-2.35 (m, 1H), 2.32-2.24 (m, 1H), 2.22-2.11 (m, 2H), 1.99-1.86 (m, 2H), 1.78 (br, 1H), 1.69-1.60 (m, 1H), 1.48 (d, J = 6.2 Hz, 3H).

LC-MS (ESI): m/z = 367.2 $[M+H]^+$.

**Example 3:**

2-((1R,5S,6R)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (**isomer 1 of compound 3**)

2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (**isomer 2 of compound 3**)

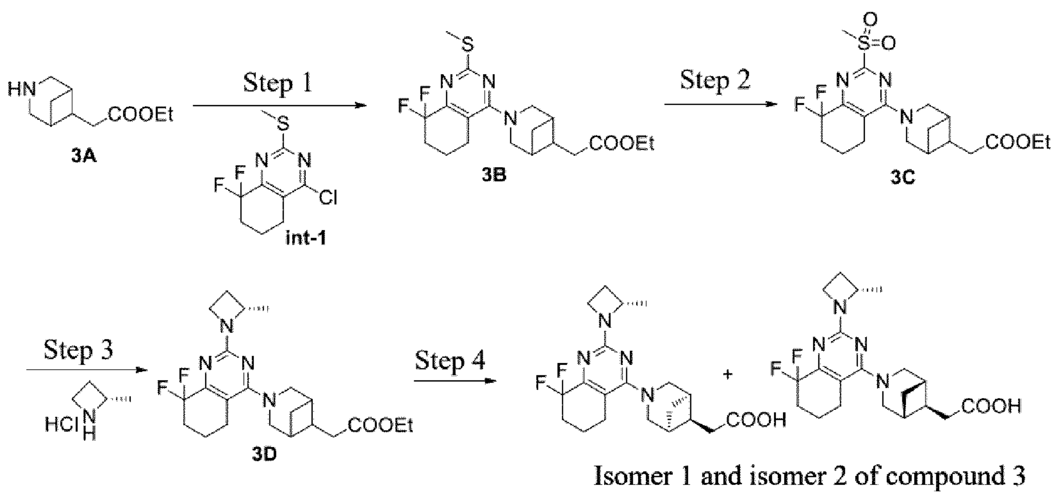


Step 1: ethyl 2-(3-(8,8-difluoro-2-(methylthio)-5,6,7,8-tetrahydroquinazolin-4-yl)-3-azabicyclo[3.1.1]heptan-6-yl)acetate (**3B**)

**3A** (0.55 g, 2.98 mmol) was dissolved in N-methylpyrrolidone (10 mL), and int-1 (synthesized according to WO 2020156445A1) (0.5 g, 1.99 mmol) and potassium carbonate (0.55 g, 3.98 mmol) were added. Under nitrogen protection,

the mixture was reacted overnight at 90°C and cooled to room temperature. 50 mL of water was added, and the mixture was extracted with ethyl acetate (25 mL × 3). The organic layers were combined, back-flushed with saturated brine (25 mL), dried over anhydrous sodium sulphate and concentrated, and the residue was separated and purified by silica gel column chromatography (petroleum ether: ethyl acetate (v/v) = 4: 1) to obtain the title compound **3B** (0.66 g, 83%).
LC-MS (ESI): m/z = 398.1 $[M+H]^+$.

Step 2: ethyl 2-(3-(8,8-difluoro-2-(methylsulfonyl)-5,6,7,8-tetrahydroquinazolin-4-yl)-3-azabicyclo[3.1.1]heptan-6-yl)acetate (**3C**)

**3B** (0.66 g, 1.66 mmol) was dissolved in dichloromethane (20 mL), m-chloroperoxybenzoic acid (1.15 g, 6.65 mmol) was added, and then the mixture was reacted at room temperature overnight. The reaction was quenched by adding 30 mL of saturated sodium bicarbonate solution and extracted with ethyl acetate (20 mL × 3). The organic layers were combined, washed with saturated brine (25 mL), dried over anhydrous sodium sulphate and concentrated, and the residue was separated and purified by silica gel column chromatography (petroleum ether: ethyl acetate (v/v) = 1: 1) to obtain the title compound **3C** (0.51 g, 71%).
LC-MS (ESI): m/z = 430.1 $[M+H]^+$.

Step 3: ethyl 2-(3-(8,8-difluoro-2-((S)-2-methylazetidin-1-yl)-5,6,7,8-tetrahydroquinazolin-4-yl)-3-azabicyclo[3.1.1]heptan-6-yl)acetate (compound **3D**)

**3C** (0.51 g, 1.19 mmol) was dissolved in 1,4-dioxane (5 mL), and (S)-2-methylazetidine hydrochloride (190 mg, 1.78 mmol) and potassium carbonate (0.66 g, 4.76 mmol) were added. Under nitrogen protection, the mixture was reacted at 100°C overnight and cooled to room temperature. 25 mL of water was added, and the mixture was extracted with ethyl acetate (15 mL × 3). The organic layers were combined, dried over anhydrous sodium sulphate and concentrated, and the residue was separated and purified by silica gel column chromatography (petroleum ether: ethyl acetate (v/v) = 4: 1) to obtain the title compound **3D** (0.34 g, 68%).
LC-MS (ESI): m/z = 421.3 $[M+H]^+$.

Step 4: 2-((1R,5S,6R)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (**isomer 1 of compound 3**)

2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (**isomer 2 of compound 3**)

**3D** (0.34 g, 0.81 mmol) was dissolved in tetrahydrofuran (10 mL), and a solution of lithium hydroxide monohydrate (170 mg, 4.05 mmol) in water (5 mL) was added dropwise in an ice bath. After the addition was completed, the mixture was reacted at room temperature for 4 h, adjusted to about pH 4 with 2 M hydrogen chloride solution and concentrated, and the residue was separated and purified by silica gel column chromatography (dichloromethane: methanol (v/v) = 10: 1) to obtain the product **compound 3** (150 mg, 47%).
Compound **3** was subjected to SFC chiral preparative separation to obtain compound **3-isomer 1** (51 mg, ee% = 100%, yield: 16.0%) and compound **3-isomer 2** (53 mg, ee% = 100%, yield: 16.7%), with purification conditions as follows: (instrument name: MG II preparative SFC (SFC-14); chromatographic column: ChiralPak AD, 250 × 30 mm I.D., 10 μm; mobile phase: phase A: $CO_2$; phase B: methanol (0.1% $NH_3 \cdot H_2O$); flow rate: 70 mL/min; column pressure: 100 bar; column temperature: 38°C; absorption wavelength: 220 nm; cycle time: about 2 min); retention time for compound **3-isomer 1:** 1.012 min; retention time for compound **3-isomer 2:** 1.283 min.

Compound **3-isomer 1:**

$^1H$ NMR (400 MHz, $CDCl_3$) δ 4.56 (m, 1H), 4.20 (m, 1H), 4.13-4.05 (m, 1H), 4.01-3.87 (m, 4H), 2.82 (br, 2H), 2.64 (q, J = 6.8 Hz, 1H), 2.57 (t, J = 6.0 Hz, 2H), 2.46 (d, J = 7.6 Hz, 2H), 2.43-2.37 (m, 1H), 2.32-2.20 (m, 2H), 2.13-2.05 (m, 1H), 1.96 (m, 1H), 1.85 (m, 2H), 1.50 (d, J = 6.0 Hz, 3H), 1.45 (m, 1H).
LC-MS (ESI): m/z = 393.3 $[M+H]^+$.

Compound **3-isomer 2:**

$^1H$ NMR (400 MHz, CDCl3) δ 4.45 (q, J = 6.4 Hz, 1H), 4.13-3.97 (m, 5H), 3.91 (d, J = 11.2 Hz, 1H), 2.75 (m, 2H), 2.71 (d, J = 8.0 Hz, 2H), 2.41-2.30 (m, 4H), 2.28-2.18 (m, 2H), 2.16-2.10 (m, 1H), 1.96-1.90 (m, 1H), 1.86-1.78 (m, 2H), 1.49 (d, J = 6.0 Hz, 3H), 1.45 (dd, J = 8.8, 5.2 Hz, 1H).

LC-MS (ESI): m/z = 393.3 $[M+H]^+$.

**Example 4:**

2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid (**isomer 1 of compound 4 or isomer 2 of compound 4**)

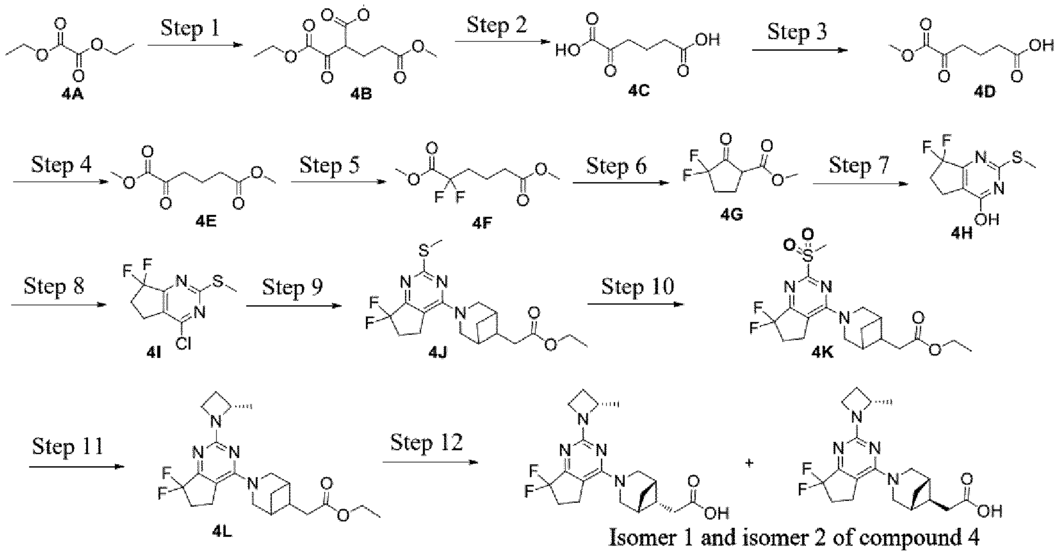


Step 1: 1-ethyl 2,4-dimethyl 1-oxobutane-1,2,4-tricarboxylate (**4B**)

Sodium ethoxide (233 mL, 684.28 mmol, 21% in ethanol) was added to tetrahydrofuran (600 mL). Diethyl oxalate (100 g, 684.28 mmol) was added slowly to the solution, and then dimethyl glutarate (109.60 g, 684.28 mmol) was added. The mixture was reacted at 25°C for 16 h. The reaction was quenched by adding 3 N hydrochloric acid (400 mL) and extracted three times with ethyl acetate (400 mL). The ethyl acetate phase was dried over anhydrous sodium sulphate, filtered and concentrated, and the residue was directly used in the next step.

Step 2: 2-oxohexanedioic acid (**4C**)

1-ethyl 2,4-dimethyl 1-oxobutane-1,2,4-tricarboxylate (the crude **4B** from the previous step) was added to 4 N hydrochloric acid (900 mL, 3600 mmol), and the mixture was reacted at 65°C for 6 h, subjected to rotary evaporation and recrystallized to obtain 72.02 g of compound, with two-step yield of 65.8%.
LCMS m/z = 161.2 [M+1].

Step 3: 6-methoxy-5,6-dioxohexanoic acid (**4D**)

2-oxohexanedioic acid **4C** (20 g, 125 mmol) was added to a solution of DBN (18.63 g, 150 mmol) in acetone (200 mL), and dimethyl sulphate (15.77 g, 125 mmol) was added dropwise at 0°C. The mixture was stirred at 25°C overnight and subjected to rotary evaporation. Water (100 mL) was added, and the mixture was adjusted to pH=2-3 and extracted with ethyl acetate (3 × 150 mL). The organic phases were combined, dried over anhydrous sodium sulphate, filtered and concentrated, and the residue **4D** was directly used in the next step.

Step 4: dimethyl 2-oxohexanedioate (**4E**)

6-methoxy-5,6-dioxohexanoic acid (**4D**) (the crude from the previous step) was dissolved in dichloromethane (200 mL), four drops of N,N-dimethylformamide were added, and oxalyl chloride (31.73 g, 250 mmol) was added at 0°C. The mixture was stirred at 5°C-10°C for 4 h and subjected to rotary evaporation, and methanol (20 mL) was added to the residue at 0°C. The mixture was subjected to rotary evaporation at 25°C for 30 min, and the residue was separated

and purified by silica gel column chromatography (petroleum ether: ethyl acetate (v/v) = 3: 1) to obtain the title compound **4E** (16.45 g, 70%).

$^1H$ NMR (400 MHz, $CDCl_3$) δ 3.90 (s, 3H), 3.76-3.71 (m, 3H), 2.95 (t, J=7.1, 2H), 2.45 (dd, J=9.5, 5.2, 2H), 1.97 (p, J=7.2, 2H).

Step 5: dimethyl 2,2-difluorohexanedioate (**4F**)

Dimethyl 2-oxohexanedioate (**4E**) (20 g, 106.38 mmol) was dissolved in dichloromethane (200 mL). DAST was slowly added dropwise, and the mixture was reacted at 25°C for 20 h. The reaction was quenched by adding water (100 mL) and extracted with dichloromethane (100 mL × 3). The combined organic phase was dried over anhydrous sodium sulphate, filtered and concentrated, and the residue was separated and purified by silica gel column chromatography (petroleum ether: ethyl acetate (v/v) = 5: 1) to obtain the title compound **4F** (8.3 g, 37%).

$^1H$ NMR (400 MHz, $CDCl_3$) δ 3.88 (s, 3H), 3.68 (s, 3H), 2.40 (t, J=7.3, 2H), 2.20-2.05 (m, 2H), 1.88-1.77 (m, 2H).

Step 6: methyl 3,3-difluoro-2-oxocyclopentane-1-carboxylate (**4G**)

Sodium hydride (60%) (2.37 g, 59.28 mmol) was dissolved in THF (190 mL), and a solution of dimethyl 2,2-difluorohexanedioate (**4F**) (8.3 g, 39.52 mmol) in THF (10 mL) was added. The mixture was reacted at 30°C for 4 h. The reaction was quenched by adding ice water, adjusted to pH=5-6 and extracted with ethyl acetate (100 mL × 5). The combined organic phase was dried over anhydrous sodium sulphate, filtered and concentrated, and the residue was separated and purified by silica gel column chromatography (petroleum ether: ethyl acetate (v/v) = 2: 1) to obtain the title compound **4G** (4.8 g, 75.84%).

$^1H$ NMR (400 MHz, $CDCl_3$) δ 9.46 (s, 0H), 3.84 (s, 3H), 2.56-2.49 (m, 1H), 2.42-2.33 (m, 1H).

Step 7: 7,7-difluoro-2-(methylthio)-6,7-dihydro-5H-cyclopenta[d]pyrimidin-4-ol (**4H**)

Methyl 3,3-difluoro-2-oxocyclopentane-1-carboxylate (**4G**) (2.4 g, 13.47 mmol) was dissolved in water (40 mL). Methylisothiourea sulphate (3.8 g, 20.2 mmol) and sodium carbonate (2.85 g, 26.9 mmol) were added, and the mixture was reacted at 25°C for 18 h, adjusted to pH=2 with 1 N hydrochloric acid and extracted with ethyl acetate (100 mL × 3). The combined organic phase was dried over anhydrous sodium sulphate, filtered and concentrated to obtain the title compound **4H** as a crude (3.0 g, 100%), which was directly used in the next step.

LCMS m/z = 219.1 [M+1].

Step 8: 4-chloro-7,7-difluoro-2-(methylthio)-6,7-dihydro-5H-cyclopenta[d]pyrimidine (**4I**)

7,7-difluoro-2-(methylthio)-6,7-dihydro-5H-cyclopenta[d]pyrimidin-4-ol (**4H**) (3.0 g, the crude from the previous step) was dissolved in 1,2-dichloroethane (10 mL). Phosphorus oxychloride (5 mL) was added, and the mixture was reacted at 110°C for 18 h, concentrated, adjusted to pH=7-8 with saturated sodium bicarbonate and extracted with ethyl acetate (50 mL × 3). The combined organic phase was dried over anhydrous sodium sulphate, filtered and concentrated, and the residue was separated and purified by silica gel column chromatography (petroleum ether: ethyl acetate (v/v) = 10: 1) to obtain the title compound **41** (2.02 g, 63.55%).

$^1H$ NMR (400 MHz, $CDCl_3$) δ 2.99 (m, J=8.2, 6.5, 3.1, 2H), 2.70-2.63 (m, 1H), 2.62 (s, 3H), 2.61-2.58 (m, 1H).

Step 9: ethyl-2-(3-(7,7-difluoro-2-(methylthio)-6,7-dihydro-5H-cyclopenta[d]pyrimidin-4-yl)-3-azabicyclo[3.1.1]heptan-6-yl)acetate (**4J**)

4-chloro-7,7-difluoro-2-(methylthio)-6,7-dihydro-5H-cyclopenta[d]pyrimidine (**4I**) (220 mg, 0.93 mmol) was dissolved in NMP (5 mL). Ethyl 2-((1R,5S)-3-azabicyclo[3.1.1]hept-6-yl)acetate (205 mg, 1.12 mmol) and potassium carbonate (37.33 mg, 2.05 mmol) were added, and the mixture was reacted at 90°C for 18 h. Water was added, and the mixture was extracted with ethyl acetate (20 mL × 3). The combined organic phase was dried over anhydrous sodium sulphate, filtered and concentrated to obtain the title compound **4J** (302 mg, 84.69%), which was directly used in the next step.

LCMS m/z = 384.2 [M+1].

Step 10: ethyl -2-(3-(7,7-difluoro-2-(m ethyl sulfonyl)-6,7-dihydro-5H-cyclopenta[d]pyrimidin-4-yl)-3-azabicyclo[3.1.1]heptan-6-yl)acetate (**4K**)

Compound (**4J**) (the crude from the previous step) was dissolved in dichloromethane (10 mL), and m-chloroperoxybenzoic acid (80%) (442 mg, 2.05 mmol) was added. The mixture was reacted at 25°C for 5 h and adjusted to about pH 8 by dropwise adding a saturated aqueous sodium bicarbonate solution. The organic layer was separated, and the remaining layer was extracted with dichloromethane (10 mL × 2). The combined organic phase was dried over anhydrous sodium sulphate, filtered and concentrated, and the residue was separated and purified by silica gel column chromatography (petroleum ether: ethyl acetate (v/v) = 3: 1) to obtain the title compound **4K** (251 mg, 65.03%).
LCMS m/z = 416.1 [M+1].

Step 11: ethyl-2-(3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetate (**4L**)

Compound (**4K**) (251 mg, 0.60 mmol) was dissolved in NMP (6 mL). DIPEA (0.5 mL) and (S)-2-methylazetidine hydrochloride (129 mg, 1.2 mmol) were added, and the mixture was reacted under microwave at 160°C for 2 h. Water was added, and the mixture was extracted with ethyl acetate (20 mL × 3). The combined organic phase was dried over anhydrous sodium sulphate, filtered and concentrated, and the residue was separated and purified by silica gel column chromatography (petroleum ether: ethyl acetate (v/v) = 1: 1) to obtain the title compound **4L** (202 mg, yield: 83.33%).
LCMS m/z = 407.2 [M+1].

Step 12: 2-((1R,SS,6R)-3-(7,7-difluoro-2-((S)-2-methylazetidin-1-yl)-6,7-dihydro-5H-cyclopenta[d]pyrimidin-4-yl)-3-azabicyclo[3 .1.1]heptan-6-yl)acetic acid (**isomer 1 of compound 4**)

2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid (**isomer 2 of compound 4**)

Compound (**4L**) (202 mg, 0.5 mmol) was dissolved in methanol/tetrahydrofuran/water (3/3/3 mL), and LiOH (47.9 mg, 2 mmol) was added. The mixture was reacted at 25°C for 3 h, adjusted to pH=5 with 1 N hydrochloric acid and extracted with ethyl acetate (10 mL × 3). The combined organic phase was dried over anhydrous sodium sulphate, filtered and concentrated, and the residue was separated and purified by silica gel column chromatography (dichloromethane: methanol (v/v) = 20: 1) to obtain the title **compound 4** (102 mg, 54%).
LCMS m/z = 379.2 [M+1].
Resolution of **compound 4:** instrument: Waters UPC2 analytical SFC (SFC-H); column: ChiralPak AD, 150 × 4.6 mm I.D., 3 μm; mobile phase: A: $CO_2$, B: ethanol (0.05% DEA); gradient: B 40%; flow rate: 2.5 mL/min; back pressure: 100 bar; column temperature: 35°C; wavelength: 220 nm; cycle: 13 min; sample preparation: dissolving compounds 5 and 6 in methanol/dichloromethane (15 mL); injection: 0.5 mL/injection. After the compound was separated, two optical isomers, **isomer 1 of compound 4** (retention time: 1.049 min, 25 mg, ee% = 99%) and **isomer 2 of compound 4** (retention time: 1.326 min, 30 mg, ee% = 99%) were obtained.

**Isomer 1 of compound 4**

$^1$H NMR (400 MHz, DMSO-$d_6$) δ 12.09 (s, 1H), 4.31 (dd, J=12.7, 6.3, 1H), 4.01-3.77 (m, 4H), 3.20 (d, J=3.7, 3H), 2.48 (d, J=5.2, 3H), 2.36 (m, J=15.1, 11.8, 5.3, 3H), 2.23 (d, J=7.1, 2H), 2.02 (m, J=14.2, 6.0, 1H), 1.89 (m, J=16.0, 8.0, 1H), 1.43 (d, J=6.1, 3H), 1.37 (d, J=9.2, 1H), 1.24 (s, 1H).
LCMS m/z = 379.2 $[M+1]^+$.

**Isomer 2 of compound 4**

$^1$H NMR (400 MHz, DMSO-$d_6$) δ 12.09 (s, 1H), 4.35 (dd, J=13.1, 6.5, 1H), 4.16-3.69 (m, 5H), 3.23-3.15 (m, 3H), 2.60 (d, J=7.9, 2H), 2.37 (dd, J=17.8, 8.6, 3H), 2.26 (d, J=5.9, 2H), 1.93 (m, J=26.3, 15.3, 8.3, 2H), 1.43 (d, J=6.2, 3H), 1.33 (dd, J=9.8, 5.3, 1H), 1.24 (s, 1H).
LCMS m/z = 379.2 [M+1].

**Example 5:**

2-(3-(2-((S)-2-methylazetidin-1-yl)quinazolin-4-yl)-3-azabicyclo[3.1.1]heptan-6-yl)acetic acid (**compound 5**)

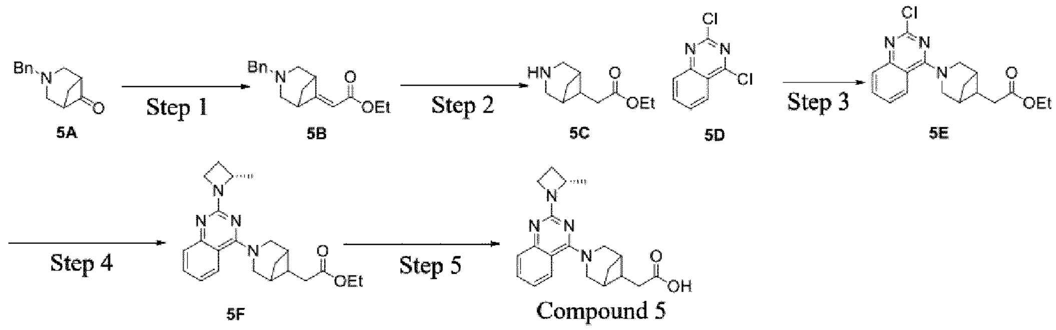


Step 1: ethyl 2-(3-benzyl-3-azabicyclo[3.1.1]heptan-6-ylidene)acetate (5B)

Sodium hydride (1.5 g, 37.3 mmol) and THF (40 mL) were added to a 250 mL single-necked flask. A solution of ethyl triethylphosphonoacetate (8.3 g, 37.3 mmol) in THF (40 mL) was added dropwise in an ice bath. The mixture was stirred for 1 h, and a solution of 3-benzyl-3-azabicyclo[3.1.1]heptane-6-one in THF (30 mL) was added dropwise. The mixture was stirred at room temperature overnight, washed with an aqueous solution (100 mL) and extracted with ethyl acetate (100 mL × 2). The organic phases were combined, washed with saturated sodium chloride (50 mL × 1), dried over anhydrous sodium sulphate and concentrated under reduced pressure, and then the residue was subjected to column chromatography (PE:EA = 4: 1) to obtain the title compound **5B** (4.8 g, yield: 71%).

LC-MS (ESI): m/z = 272.1 $[M+H]^+$.

Step 2: ethyl 2-(3-azabicyclo[3.1.1]heptan-6-yl)acetate (**5C**)

**5B** (4.8 g, 18.0 mmol) was added to a 100 mL single-necked flask and dissolved in methanol (40 mL). 10% palladium on carbon (1 g) was added. The mixture was subjected to hydrogen replacement three times, reacted at room temperature overnight and concentrated to obtain the title compound **5C** (3.0 g, yield: 93%).

LC-MS (ESI): m/z = 184.1 $[M+H]^+$.

Step 3: ethyl 2-(3-(2-chloroquinazolin-4-yl)-3-azabicyclo[3.1.1]heptan-6-yl)acetate (**5E**)

**5C** (0.26 g, 1.51 mmol) was added to a 100 mL single-necked flask and dissolved in THF (10 mL). Diisopropylethylamine (0.29 g, 2.27 mmol) was added, and **5D** (0.30 g, 1.51 mmol) was added in an ice bath. The reaction was allowed to warm to room temperature naturally and reacted for 2 h and concentrated, and the residue was separated by column chromatography (petroleum ether: ethyl acetate (v/v) = 2: 1-1: 3) to obtain the title compound **5E** (0.42 g, 80%).

LC-MS (ESI): m/z = 346.2 $[M+H]^+$.

Step 4: ethyl 2-(3-(2-((S)-2-methylazetidin-1-yl)quinazolin-4-yl)-3-azabicyclo[3.1.1]heptan -6-yl)acetate (**5F**)

**5E** (0.20 g, 0.58 mmol) was added to a 50 mL single-necked flask and dissolved in DMF (10 mL). Potassium carbonate (0.24 g, 1.74 mmol) and (S)-2-methylazetidin-1-yl (0.082 g, 1.16 mmol) were added. The mixture was reacted at 90°C for 16 h and concentrated, and the residue was subjected to column chromatography (petroleum ether: ethyl acetate (v/v) = 1: 1-1: 5) to obtain the title compound **5F** (0.15 g, 68%).

LC-MS (ESI): m/z = 381.3 $[M+H]^+$.

Step 5: 2-(3-(2-((S)-2-methylazetidin-1-yl)quinazolin-4-yl)-3-azabicyclo[3.1.1]heptan-6-yl)acetic acid (**compound 5**)

Substrate **5F** (0.20 g, 0.53 mmol) was added to a 50 mL single-necked flask and dissolved in methanol (5 mL), and a sodium hydroxide solution (2 M, 5 mL) was added. The mixture was stirred at room temperature overnight, adjusted to pH 7-8 with hydrochloric acid (2 M) and extracted with dichloromethane (10 mL × 5). The organic phases were combined and concentrated, and the residue was separated by preparative HPLC and lyophilized to obtain title compound **5** (40 mg, 21%).

Preparative HPLC separation methods: instrument: waters 2767 (preparative liquid phase chromatographic instrument); chromatographic column: SunFire@ Prep C18 (19 mm × 250 mm). The sample was dissolved in DMF and filtered with a 0.45 μm filter to prepare a sample solution. Preparative chromatography conditions: a. composition of mobile phases A and B: mobile phase A: acetonitrile; mobile phase B: water; b. gradient elution: mobile phase A: 25%-70%; c. flow rate: 12 mL/min; d. elution time: 15 min; retention time for compound **5:** 14 min.

LC-MS (ESI): m/z = 353.2 $[M+H]^+$.

$^1$H NMR (400 MHz, $CDCl_3$) $\delta$ 8.15-8.05 (m, 1H), 7.91-7.89 (m, 1H), 7.52-7.46 (m, 1H), 7.13-7.01 (m, 1H), 4.66-4.64 (m, 1H), 4.41-3.94 (m, 6H), 2.71-2.64 (m, 3H), 2.52-2.37 (m, 2H), 2.32 (d, 1H), 2.14-2.05 (m, 1H), 2.05-1.89 (m, 1H), 1.53 (d, 3H), 1.43 (d, 1H).

**Example 6:**

2-(3-(2-((S)-2-methylazetidin-1-yl)thieno[3,2-d]pyrimidin-4-yl)-3-azabicyclo[3.1.1]heptan-6-yl)acetic acid (**compound 6**)

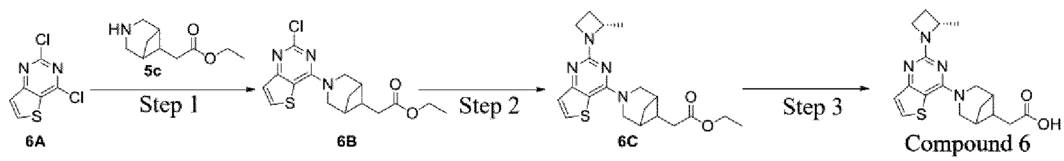


Step 1: ethyl 2-(3-(2-chlorothieno[3,2-d]pyrimidin-4-yl)-3-azabicyclo[3.1.1]heptan-6-yl) acetate (**6B**)

**6A** (0.30 g, 1.46 mmol) was added to a 100 mL single-necked flask and dissolved in THF (10 mL). Diisopropylethylamine (0.38 g, 2.92 mmol) was added, and **5C** (0.25 g, 1.46 mmol) was added in an ice bath. The reaction was allowed to warm to room temperature naturally and reacted for 2 h and concentrated, and the residue was separated by column chromatography (petroleum ether: ethyl acetate (v/v) = 2: 1-1: 3) to obtain the title compound **6B** (0.30 g, 58%).

LC-MS (ESI): m/z = 352.2 $[M+H]^+$.

Step 2: ethyl 2-(3-(2-((S)-2-methylazetidin-1-yl)thieno[3,2-d]pyrimidin-4-yl)-3-azabicyclo [3.1.1]heptan-6-yl)acetate (**6C**)

**6B** (0.30 g, 0.85 mmol) was added to a 50 mL single-necked flask and dissolved in DMF (10 mL). Potassium carbonate (0.47 g, 3.40 mmol) and (S)-2-methylazetidin-1-yl (0.18 g, 2.55 mmol) were added. The mixture was reacted at 90°C for 16 h and concentrated, and the residue was subjected to column chromatography (petroleum ether: ethyl acetate (v/v) = 1: 1-1: 5) to obtain the title compound **6C** (0.25 g, 76%).

LC-MS (ESI): m/z = 387.2 $[M+H]^+$.

Step 3: 2-(3-(2-((S)-2-methylazetidin-1-yl)thieno[3,2-d]pyrimidin-4-yl)-3-azabicyclo[3.1.1]heptan-6-yl)acetic acid (**compound 6**)

Substrate **6C** (0.25 g, 0.63 mmol) was added to a 50 mL single-necked flask and dissolved in methanol (5 mL), and a sodium hydroxide solution (2 M, 5 mL) was added. The mixture was stirred at room temperature overnight, adjusted to pH=7-8 with hydrochloric acid (2 M) and extracted with dichloromethane (10 mL $\times$ 5). The organic phases were combined and concentrated, and the residue was separated by preparative HPLC and lyophilized to obtain title **compound 6** (30 mg, 13%).

Preparative HPLC separation methods: instrument: waters 2767 (preparative liquid phase chromatographic instrument); chromatographic column: SunFire@ Prep C18 (19 mm $\times$ 250 mm). The sample was dissolved in DMF and filtered with a 0.45 $\mu$m filter to prepare a sample solution. Preparative chromatography conditions: a. composition of mobile phases A and B: mobile phase A: acetonitrile; mobile phase B: water (containing 1% TFA); b. gradient elution: mobile phase A: 15%-70%; c. flow rate: 12 mL/min; d. elution time: 15 min; retention time for **compound 6:** 13.5 min.

LC-MS (ESI): m/z = 359.2 $[M+H]^+$.

$^1$H NMR (400 MHz, $CDCl_3$) $\delta$7.72-7.70 (m, 1H), 7.56-7.53 (m, 1H), 4.66 (s, 1H), 4.33-4.20 (m, 6H), 2.80-2.60 (m, 3H), 2.59-2.41 (m, 2H), 2.35 (d, 1H), 2.24-2.09 (m, 1H), 2.03-1.98 (m, 1H), 1.55 (d, 3H), 1.48 (t, 1H).

**Example 7:**

(S)-2-(1-(4-cyano-5,5-difluoro-3-(2-methylazetidin-1-yl)-6,7-dihydro-5H-cyclopenta[c]pyridin-1-yl)azetidin-3-yl)acetic acid (**compound 7**)

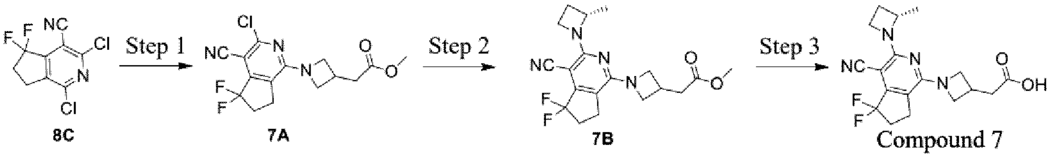


Step 1: methyl 2-(1-(3-chloro-4-cyano-5,5-difluoro-6,7-dihydro-5H-cyclopenta[c]pyridin-1-yl)azetidin-3-yl)acetate (**7A**)

**8C** (200 mg, 0.8 mmol) was dissolved in isopropanol (3 mL), and methyl 2-(azetidin-3-yl)acetate (155 mg, 1.2 mmol) and N,N-diisopropylethylamine (517 mg, 4 mmol) were added. The mixture was stirred at 40°C for 30 min and filtered, and the filter cake was collected and dried to obtain the target compound (**7A**) (270 mg, 99%).
LCMS m/z = 342.0 [M+1].

Step 2: methyl (S)-2-(1-(4-cyano-5,5-difluoro-3-(2-methylazetidin-1-yl)-6,7-dihydro-5H-cyclopenta[c]pyridin-1-yl)azetidin-3-yl)acetate (**7B**)

**7A** (270 mg, 0.79 mmol) was dissolved in dimethyl sulfoxide (3 mL). (S)-2-methylazetidine hydrochloride (102 mg, 0.75 mmol) and sodium bicarbonate (199 mg, 2.37 mmol) were added, and the mixture was warmed to 100°C and reacted for 2 h. After the reaction was completed, the mixture was cooled to room temperature, and the reaction was quenched by adding water and extracted with ethyl acetate. The organic layers were combined and washed with saturated brine, and the residue was separated and purified by silica gel column chromatography (ethyl acetate: petroleum ether (v/v) = 0-50%) to obtain the title compound **7B** (290 mg, 98%).
LCMS m/z = 377.2 [M+1].

Step 3: (S)-2-(1-(4-cyano-5,5-difluoro-3-(2-methylazetidin-1-yl)-6,7-dihydro-SH-cyclopenta[c]pyridin-1-yl)azetidin-3-yl)acetic acid (**compound 7**)

Compound **7B** (290 mg, 0.77 mmol) was dissolved in methanol/water (5/2 mL). LiOH (93 mg, 3.85 mmol) was added, and the mixture was reacted at 25°C for 2 h, adjusted to pH=3 with 1 N hydrochloric acid and extracted with ethyl acetate (10 mL × 3). The combined organic phase was dried over anhydrous sodium sulphate, filtered and concentrated, and the residue was separated and purified by silica gel column chromatography (methanol: dichloromethane (v/v) = 0-15%) to obtain the title **compound 7** (224 mg, 80%).
$^1$H NMR (400 MHz, $CDCl_3$) $\delta$ 4.58 (m, J = 12.6, 6.2 Hz, 1H), 4.45 (m, J = 8.9, 5.1 Hz, 1H), 4.40-4.36 (m, 1H), 4.14-4.09 (m, 1H), 3.89 (m, J = 14.9, 9.0, 5.6 Hz, 2H), 3.15-3.01 (m, 1H), 2.90-2.79 (m, 2H), 2.76 (d, J = 7.8 Hz, 2H), 2.60-2.46 (m, 2H), 2.41 (m, J = 10.9, 9.0, 5.0 Hz, 1H), 1.96 (m, J = 11.0, 9.1, 6.8 Hz, 1H), 1.47 (d, J = 6.2 Hz, 3H), 1.26 (t, J = 7.1 Hz, 1H).
LCMS m/z =363.1 [M+1].

**Example 8:**

2-((R)-1-(4-cyano-5,5-difluoro-3-((S)-2-methylazetidin-1-yl)-6,7-dihydro-5H-cyclopenta[c]pyridin-1-yl)pyrrolidin-3-yl)acetic acid 2-((S)-1-(4-cyano-5,5-difluoro-3-((S)-2-methylazetidin-1-yl)-6,7-dihydro-5H-cyclopenta[c]pyridin-1-yl)pyrrolidin-3-yl)acetic acid **(isomer 1 of compound 8 or isomer 2 of compound 8)**

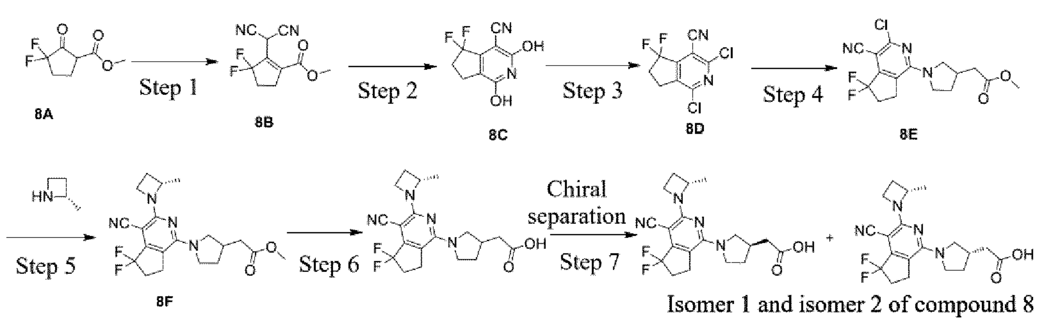

Step 1: methyl 2-(dicyanomethyl)-3,3-difluorocyclopent-1-ene-1-carboxylate (**8B**)

Compound **8A** (3.0 g, 16.8 mmol) (prepared with reference to WO 2020156445) was dissolved in methanol (30 mL). Malononitrile (1.7 g, 25.2 mmol) and imidazole (3.4 g, 50.5 mmol) were added, and after the addition was completed, the mixture was warmed to 45°C and reacted for 15 h. After the reaction was completed, water (50 mL) was added, the mixture was adjusted to pH=3-4 with 1 N hydrochloric acid and extracted with dichloromethane (50 mL × 4). The organic phases were combined, washed with saturated brine, dried over anhydrous sodium sulphate and concentrated to obtain compound **8B** as a crude (3.8 g, 100%), which was directly used in the next step without further purification.
LC-MS (ESI): m/z =227.0 $[M+H]^+$.

Step 2: 5,5-difluoro-1,3-dihydroxy-6,7-dihydro-5H-cyclopenta[c]pyridine-4-carbonitrile (**8C**)

The crude of compound **8B** (3.8 g, 16.8 mmol) was dissolved in a mixed solvent of purified water (38 mL) and DMF (10 mL), and the mixture was warmed to 100°C and reacted for 24 h. After the reaction was completed, the mixture was concentrated under reduced pressure to remove the solvent, and the residue was separated and purified by silica gel column chromatography (dichloromethane: methanol (v/v) = 10: 3) to obtain the title compound **8C** (2.1 g, 58.7%).
LC-MS (ESI): m/z =211.0 $[M-H]^-$.

Step 3: 1,3-dichloro-5,5-difluoro-6,7-dihydro-5H-cyclopenta[c]pyridine-4-carbonitrile **(8D)**

The compound **8C** (2.1 g, 9.9 mmol), phosphorus oxychloride (21 mL) and benzyltrimethylammonium chloride (3.7 g, 19.8 mmol) were successively added to a sealed tube, and the mixture was warmed to 150°C and reacted for 8 h. After the reaction was completed, the mixture was concentrated to remove phosphorus oxychloride, and the residue was slowly poured into water (200 mL). The reaction solution was adjusted to pH=7-8 with a saturated sodium bicarbonate solution and extracted twice with ethyl acetate (100 mL × 2). The organic layers were combined, washed with saturated brine and dried over anhydrous sodium sulphate. The residue was separated and purified by silica gel column chromatography (petroleum ether: ethyl acetate (v/v) = 10: 1) to obtain the title compound **8D** (1.8 g, 72.9%).
$^1H$ NMR (400 MHz, $CDCl_3$) δ: 3.14-3.09 (m, 2H), 2.83-2.72 (m, 2H).

Step 4: methyl 2-(1-(3-chloro-4-cyano-5,5-difluoro-6,7-dihydro-5H-cyclopenta[c]pyridin-1-yl)pyrrolidin-3-yl)acetate (**8E**)

Compound **8D** (250.0 mg, 1.0 mmol) was dissolved in isopropanol (5 mL). Diisopropylethylamine (387.0 mg, 3.0 mmol) and methyl 3-pyrrolidineacetate hydrochloride (270.0 mg, 1.5 mmol) were added, and after the addition was completed, the mixture was warmed to 40°C and reacted for 2 h. After the reaction was completed, the mixture was directly concentrated, and the residue was separated and purified by silica gel column chromatography (petroleum ether: ethyl acetate (v/v) = 5: 1) to obtain the title compound **8E** (260.0 mg, 73.3%).
LC-MS (ESI): m/z =356.1$[M+H]^+$.

Step 5: methyl 2-(1-(4-cyano-5,5-difluoro-3-((S)-2-methylazetidin-1-yl)-6,7-dihydro-SH-cyclopenta[c]pyridin-1-yl)pyrrolidin-3-yl)acetate **(8F)**

Compound **8E** (260.0 mg, 0.73 mmol) was dissolved in DMSO (5 mL). (S)-2-methylazetidine hydrochloride (157 mg, 1.46 mmol) and solid sodium bicarbonate (184 mg, 2.2 mmol) were added, and after the addition was completed, the mixture was warmed to 100°C and reacted for 3 h. After the reaction was completed, water (20 mL) was added. The mixture was extracted twice with ethyl acetate (50 mL × 2), and the organic layers were combined, washed with saturated brine, dried and concentrated. The residue was separated and purified by silica gel column chromatography (ethyl acetate: ethyl acetate (v/v) = 10: 1-5: 1) to obtain the title compound **8F** (230.0 mg, 80.8%).
LC-MS (ESI): m/z = 391.1$[M+H]^+$.

Step 6: 2-(1-(4-cyano-5,5-difluoro-3-((S)-2-methylazetidin-1-yl)-6,7-dihydro-SH-cyclopenta[c]pyridin-1-yl)pyrrolidin-3-yl)acetic acid (**compound 8**)

Compound **8F** (230 mg, 0.59 mmol) was dissolved in a mixed solvent of methanol (5 mL) and water (1 mL). Lithium hydroxide (253 mg, 5.9 mmol) was added, and after the addition was completed, the mixture was reacted at room temperature for 16 h. After the reaction was completed, the mixture was adjusted to pH=3-4 with 1 N hydrochloric acid and extracted twice with dichloromethane (50 mL × 2). The organic layers were combined, washed with saturated brine, dried and concentrated, and the residue was separated and purified by silica gel column chromatography (dichloromethane: methanol (v/v) = 10: 1) to obtain the title **compound 8** (180 mg, 80.9%).
LC-MS (ESI): m/z = 377.1[M+H]$^+$.

Step 7: 2-((R)-1-(4-cyano-5,5-difluoro-3-((S)-2-methylazetidin-1-yl)-6,7-dihydro-5H-cyclopenta[c]pyridin-1-yl)pyrrolidin-3-yl)acetic acid 2-((S)-1-(4-cyano-5,5-difluoro-3-((S)-2-methylazetidin-1-yl)-6,7-dihydro-5H-cyclopenta[c]pyridin-1-yl)pyrrolidin-3-yl)acetic acid (**isomer 1 of compound 8 or isomer 2 of compound 8)**

Compound **8** (180.0 mg, 0.48 mmol) was subjected to chiral resolution to obtain two optical isomers: **isomer 1 of compound 8** (retention time: 3.95 min, 65 mg, white solid, ee% = 100%) and **isomer 2 of compound 8** (retention time: 4.05 min, 70 mg, white solid, ee% = 98.88%).
Resolution conditions:

instrument: G II preparative SFC (SFC-14); column: ChiralCel OJ, 250 × 30 mm I.D., 10 μm.
mobile phase: A: $CO_2$, B: methanol containing 0.1% aqueous ammonia; gradient: B 25%; flow rate: 70 mL/min; back pressure: 100 bar;
column temperature: 38°C; wavelength: 220 nm; cycle: 10 min;
**isomer 1 of compound 8:**

$^1$H NMR (400 MHz, $CDCl_3$) δ 4.61-4.56 (m, 1H), 4.47-4.42 (m, 1H), 4.13-4.07 (m, 1H), 4.02-3.95(m, 1H), 3.87-3.82 (m, 1H), 3.66-3.59(m, 1H), 3.35-3.30(m,1H), 3.13-3.01(m,2H), 2.68-2.36(m,6H), 2.24-2.16(m,1H), 2.00-1.92(m,1H), 1.73-1.63 (m,1H), 1.47(d,3H).
LC-MS (ESI): m/z = 377.1[M+H]$^+$.

**isomer 2 of compound 8:**

$^1$H NMR (400 MHz, $CDCl_3$) δ 4.61-4.56 (m, 1H), 4.46-4.42 (m, 1H), 4.13-4.07 (m, 1H), 3.97-3.92(m, 1H), 3.87-3.64 (m, 2H), 3.39-3.34(m,1H), 3.08-3.05(m,2H), 2.68-2.36(m,6H), 2.24-2.16(m,1H), 2.00-1.92(m,1H), 1.72-1.63 (m,1H), 1.47(d,3H).
LC-MS (ESI): m/z = 377.1[M+H]$^+$.

**Example 9:**

2-((S)-1-(7,7-difluoro-2-((S)-2-methylazetidin-1-yl)-6,7-dihydro-5H-cyclopenta[d]pyrimidin-4-yl)pyrrolidin-3-yl)acetic acid 2-((R)-1-(7,7-difluoro-2-((S)-2-methylazetidin-1-yl)-6,7-dihydro-5H-cyclopenta[d]pyrimidin-4-yl)pyrrolidin-3-yl)acetic acid **(isomer 1 of compound 9 or isomer 2 of compound 9)**

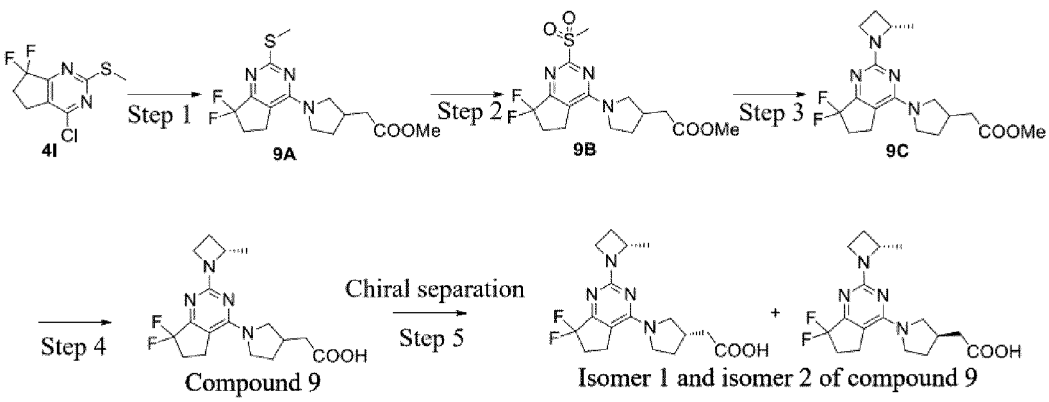


Step 1: methyl-2-(1-(7,7-difluoro-2-(methylthio)-6,7-dihydro-5H-cyclopenta[d]pyrimidin-4-yl)pyrrolidin-3-yl)acetate **(9A)**

Compound **41** (1.0 g, 4.2 mmol) was dissolved in N-methylpyrrolidinone (20 mL) at room temperature. Methyl 2-(pyrrolidin-3-yl)acetate hydrochloride (0.8 g, 4.6 mmol) and diisopropylethylamine (1.2 g, 9.3 mmol) were added, and the mixture was heated to 100°C and reacted for 4 h. The reaction solution was poured into water (120 mL), and the mixture was extracted with ethyl acetate (50 mL × 1). The organic phase was concentrated to dryness to obtain compound

**9A** (1.2 g, yield: 82%).
LC-MS (ESI): m/z =344.1 $[M+H]^+$.

Step 2: methyl-2-(1-(7,7-difluoro-2-(methylsulfonyl)-6,7-dihydro-5H-cyclopenta[d]pyrimidin-4-yl)pyrrolidin-3-yl)acetate **(compound 9B)**

Compound **9A** (1.2 g, 3.5 mmol) was dissolved in dichloromethane (50 mL) at room temperature, and m-chloroperoxybenzoic acid (1.3 g, 7.7 mmol) was added. The mixture was reacted at room temperature for 2 h. The reaction solution was washed with a saturated sodium bicarbonate solution (30 mL × 2). The organic phase was concentrated to dryness, and the residue was purified by column chromatography (petroleum ether: ethyl acetate (v:v) = 5: 1-1: 1) to obtain compound **9C** (1 g, yield: 76%).

Step 3: methyl-2-(1-(7,7-difluoro-2-((S)-2-methylazetidin-1-yl)-6,7-dihydro-5H-cyclopenta[d]pyrimidin-4-yl)pyrrolidin-3-yl)acetate **(compound 9C)**

Compound **9B** (1.0 g, 2.7 mmol) was dissolved in 1,4-dioxane (40 mL) at room temperature. (S)-2-methylazetidine hydrochloride (0.7 g, 6.7 mmol) and diisopropylethylamine (1.2 g, 9.3 mmol) were added, and the mixture was heated to 100°C and reacted in a sealed tube for 10 h. The reaction solution was poured into water (100 mL), and the mixture was extracted with ethyl acetate (50 mL × 1). The organic phase was concentrated to dryness, and the residue was purified by column chromatography (petroleum ether: ethyl acetate (v:v) = 5: 1-1: 1) to obtain compound **9C** (0.8 g, yield: 82%).
LC-MS (ESI): m/z =367.2$[M+H]^+$.

Step 4: 2-(1-(7,7-difluoro-2-((S)-2-methylazetidin-1-yl)-6,7-dihydro-5H-cyclopenta[d]pyrimidin-4-yl)pyrrolidin-3-yl)acetic acid **(compound 9)**

Compound **9C** (0.8 g, 2.2 mmol) was dissolved in ethanol (5 mL) and water (50 mL) at room temperature. Lithium hydroxide (230 mg, 5.5 mmol) was added, and the mixture was reacted at room temperature for 2 h. Water (100 mL) was added, and the mixture was adjusted to about pH 6-7 with acetic acid and extracted with ethyl acetate (50 mL × 1). The organic phase was concentrated to dryness to obtain compound **9** (600 mg, yield: 78%).
LC-MS (ESI): m/z =353.4$[M+H]^+$.
$^1$H NMR (400 MHz,$CDCl_3$) δ4.47-4.40 (m,1H), 4.11-4.05 (m,1H), 4.01-3.91 (m,2H), 3.87-3.81 (m,1H),3.67-3.61 (m,1H),3.05-3.03 (m,2H), 2.77-2.60 (m,2H), 2.48-2.18 (m,5H), 2.21-2.15 (m,1H), 1.97-1.89 (m,1H), 1.71-1.62 (m,1H), 1.50-1.48 (m,3H).

Step 5: 2-((S)-1-(7,7-difluoro-2-((S)-2-methylazetidin-1-yl)-6,7-dihydro-5H-cyclopenta[d]pyrimidin-4-yl)pyrrolidin-3-yl)acetic acid 2-((R)-1-(7,7-difluoro-2-((S)-2-methylazetidin-1-yl)-6,7-dihydro-5H-cyclopenta[d]pyrimidin-4-yl)pyrrolidin-3-yl)acetic acid **(isomer 1 of compound 9 or isomer 2 of compound 9)**

Compound **9** (500 mg) was subjected to chiral separation, with chiral preparation conditions (preparation instrument: MG II preparative SFC (SFC-14); preparative column: ChiralPak AD, 150 × 4.6 mm I.D., 3 μm; mobile phase: A: $CO_2$, B: methanol (0.1% $NH_3 \cdot H_2O$); gradient elution: B 20%; elution time: 9 min; flow rate: 60 mL/min; column temperature: 38°C) to obtain **isomer 1 of compound 9** (240 mg, retention time: 3.51 min, yield: 96%) and **isomer 2 of compound 9** (164 mg, retention time: 3.67 min, yield: 66%).

**Isomer 1 of compound 9**

LC-MS (ESI): m/z =353.4$[M+H]^+$.
$^1$H NMR (400 MHz, CDCl3) δ4.47-4.40 (m,1H), 4.11-4.06 (m,1H), 4.01-3.94 (m,2H), 3.82-3.78 (m,1H),3.70-3.60 (m,1H),3.05-3.02 (m,2H), 2.67-2.60 (m,1H), 2.48-2.33 (m,6H), 2.19-2.17 (m,1H), 1.97-1.89 (m,1H), 1.71-1.61 (m,1H), 1.50-1.48 (m,3H).

**Isomer 2 of compound 9**

LC-MS (ESI): m/z =353.4$[M+H]^+$.
$^1$H NMR (400 MHz,CDCl3) δ4.47-4.42 (m,1H), 4.11-4.05 (m,1H), 4.01-3.96 (m,2H), 3.87-3.81 (m,1H),3.63-3.60 (m,1H),3.07-3.03 (m,2H), 2.71-2.60 (m,2H), 2.48-2.37 (m,5H), 2.21-2.17 (m,1H), 1.97-1.89 (m,1H), 1.71-1.64 (m,1H), 1.50-1.48 (m,3H).

**Example 10:**

2-((1R,5S,6S)-3-(4-cyano-5,5-difluoro-3-((S)-2-methylazetidin-1-yl)-6,7-dihydro-5H-cyclopenta[c]pyridin-1-yl)-3-azabicyclo[3.1.1]heptan-6-yl)acetic acid 2-((1R,5S,6R)-3-(4-cyano-5,5-difluoro-3-((S)-2-methylazetidin-1-yl)-6,7-dihydro-5H-cyclopenta[c]pyridin-1-yl)-3-azabicyclo[3.1.1]heptan-6-yl)acetic acid **(isomer 1 of compound 10 or isomer 2 of compound 10)**

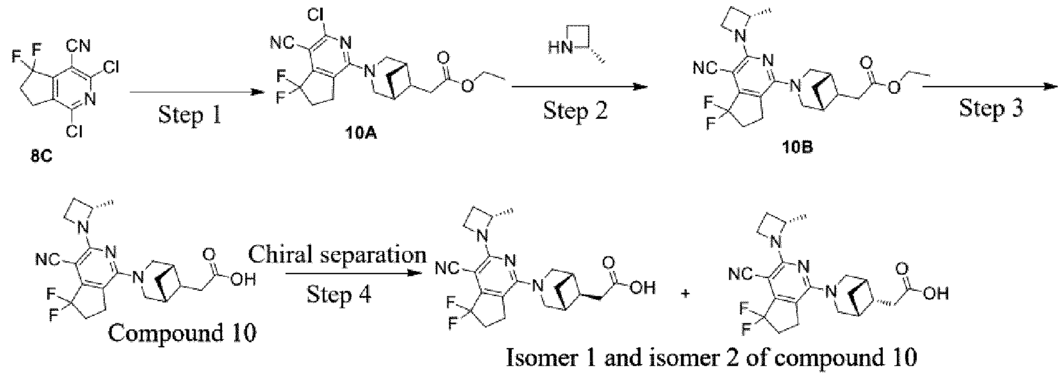


8C 10A 10B Compound 10 Isomer 1 and isomer 2 of compound 10

Step 1: ethyl 2-((1R,5S)-3-(3-chloro-4-cyano-5,5-difluoro-6,7-dihydro-5H-cyclopenta[c]pyridin-1-yl)-3-azabicyclo[3.1.1]heptan-6-yl)acetate (**10A**)

Compound **8C** (250.0 mg, 1.0 mmol) was dissolved in isopropanol (5 mL). Diisopropylethylamine (387.0 mg, 3.0 mmol) and ethyl 2-((1R,5S)-3-azabicyclo[3.1.1]heptyl-6-yl)acetate hydrochloride (330.0 mg, 1.5 mmol) were added, and after the addition was completed, the mixture was warmed to 40°C and reacted for 2 h. After the reaction was completed, the mixture was directly concentrated, and the residue was separated and purified by silica gel column chromatography (petroleum ether: ethyl acetate (v/v) = 5: 1) to obtain the title compound **10A** (240.0 mg, 60.6%).
LC-MS (ESI): m/z =396.1$[M+H]^+$.

Step 2: ethyl 2-((1R,SS)-3-(4-cyano-5,5-difluoro-3-((S)-2-methylazetidin-1-yl)-6,7-dihydro-5H-cyclopenta[c]pyridin-1-yl)-3-azabicyclo[3.1.1]heptan-6-yl)acetate (**10B**)

Compound **10A** (240.0 mg, 0.61 mmol) was dissolved in DMSO (5 mL). (S)-2-methylazetidine hydrochloride (132 mg, 1.22 mmol) and solid sodium bicarbonate (154 mg, 1.83 mmol) were added, and after the addition was completed, the mixture was warmed to 100°C and reacted for 3 h. After the reaction was completed, water (20 mL) was added. The mixture was extracted twice with ethyl acetate (50 mL × 2), and the organic layers were combined, washed with saturated brine, dried and concentrated. The residue was separated and purified by silica gel column chromatography (ethyl acetate: ethyl acetate (v/v) = 10: 1-5: 1) to obtain the title compound **10B** (200.0 mg, 76.0%).
LC-MS (ESI): m/z =431.2$[M+H]^+$.

Step 3: 2-((1R,SS)-3-(4-cyano-5,5-difluoro-3-((S)-2-methylazetidin-1-yl)-6,7-dihydro-5H-cyclopenta[c]pyridin-1-yl)-3-azabicyclo[3.1.1]heptan-6-yl)acetic acid **(compound 10)**

Compound **10B** (200 mg, 0.46 mmol) was dissolved in a mixed solvent of methanol (5 mL) and water (1 mL). Lithium hydroxide (198 mg, 4.6 mmol) was added, and after the addition was completed, the mixture was reacted at room temperature for 16 h. After the reaction was completed, the mixture was adjusted to pH=3-4 with 1 N hydrochloric acid and extracted twice with dichloromethane (50 mL × 2). The organic layers were combined, washed with saturated brine, dried and concentrated, and the residue was separated and purified by silica gel column chromatography (dichloromethane: methanol (v/v) = 10: 1) to obtain the title **compound 10** (160 mg, 86.2%).
LC-MS (ESI): m/z =403.1$[M+H]^+$.

Step 4: 2-((1R,SS,6S)-3-(4-cyano-5,5-difluoro-3-((S)-2-methylazetidin-1-yl)-6,7-dihydro-5H-cyclopenta[c]pyridin-1-yl)-3-azabicyclo[3.1.1]heptan-6-yl)acetic acid

2-((1R,5S,6R)-3-(4-cyano-5,5-difluoro-3-((S)-2-methylazetidin-1-yl)-6,7-dihydro-5H-cyclopenta[c]pyridin-1-yl)-3 -azabicyclo[3.1.1]heptan-6-yl)acetic acid **(isomer 1 of compound 10 or isomer 2 of compound 10)**

**Compound 10** (160.0 mg, 0.40 mmol) was subjected to chiral resolution to obtain two optical isomers, **isomer 1** of compound 10 (retention time: 1.22 s, 50 mg, ee% = 100%) and **isomer 2 of compound 10** (retention time: 1.48 s, 80 mg, ee% = 99.52%).

Resolution conditions:

instrument: G II preparative SFC (SFC-14); column: ChiralPak AD, 250 × 30 mm I.D., 10 μm; mobile phase: A: $CO_2$, B: isopropanol; gradient: B 30%; flow rate: 70 mL/min; back pressure: 100 bar; column temperature: 38°C; wavelength: 220 nm; cycle: 5 min.

**Isomer 1 of compound 10:**

$^1$H NMR (400 MHz, $CDCl_3$) δ 4.64-4.57(m, 1H), 4.50-4.44 (m, 1H), 4.51-4.09 (m, 1H), 4.04-3.82(m, 4H), 3.23-3.21 (m, 2H), 2.65-2.43(m, 6H), 2.41(d,2H), 2.12-2.07(m,1H), 2.01-1.93(m,1H), 1.48-1.45(m,4H).
LC-MS (ESI): m/z =403.1[M+H]$^+$.

**Isomer 2 of compound 10:**

$^1$H NMR (400 MHz, $CDCl_3$) δ 4.63-4.57(m, 1H), 4.50-4.44 (m, 1H), 4.14-3.93(m, 5H), 3.22-3.18 (m, 2H), 2.73(d,2H), 2.55-2.35(m, 6H), 2.15-2.10(m,1H), 2.01-1.93(m,1H), 1.48-1.43(m,4H).
LC-MS (ESI): m/z =403.1[M+H]$^+$.

**Example 11:**

2-(1-(7,7-difluoro-2-((2R,3R)-3-fluoro-2-methylazetidin-1-yl)-6,7-dihydro-5H-cyclopenta[d]pyrimidin-4-yl)azetidin-3-yl)acetic acid **(compound 11)**

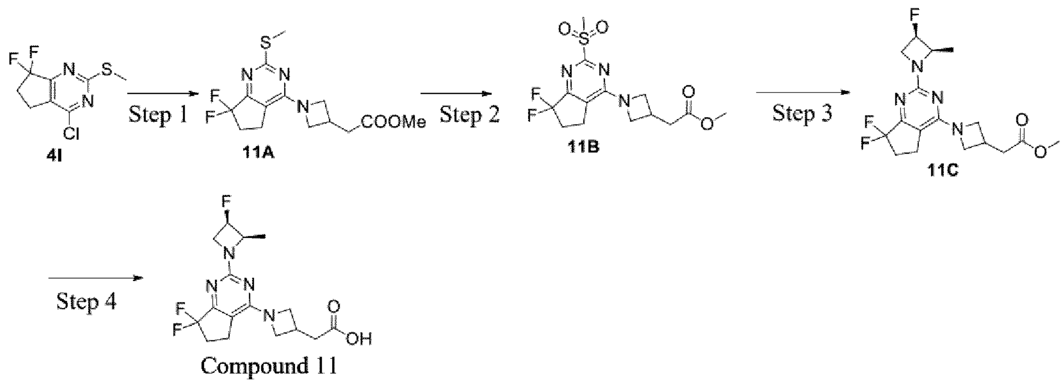


Step 1: methyl 2-(1-(7,7-difluoro-2-(methylthio)-6,7-dihydro-5H-cyclopenta[d]pyrimidin-4-yl)azetidin-3-yl)acetate **(11A)**

Compound **4l** (0.5 g, 2.1 mmol) was dissolved in N-methylpyrrolidinone (10 mL) at room temperature. Methyl 2-(azetidin-3-yl)acetate trifluoroacetate (0.61 g, 2.5 mmol) and potassium carbonate (0.64 g, 4.6 mmol) were added, and the mixture was heated to 100°C and reacted for 4 h. The reaction solution was poured into water (100 mL), and the mixture was extracted with ethyl acetate (50 mL × 1). The organic phase was concentrated to dryness to obtain compound **11A** (0.6 g, yield: 86%).

LC-MS (ESI): m/z =330.4 [M+H]$^+$.

Step 2: methyl 2-(1-(7,7-difluoro-2-(methylsulfonyl)-6,7-dihydro-5H-cyclopenta[d]pyrimidin-4-yl)azetidin-3-yl)acetate **(11B)**

Compound **11A** (0.6 g, 1.8 mmol) was dissolved in dichloromethane (30 mL) at room temperature, and m-chloroperoxybenzoic acid (0.7 g, 4.1 mmol) was added. The mixture was reacted at room temperature for 2 h. The reaction solution was washed with a saturated sodium bicarbonate solution (20 mL × 2). The organic phase was concentrated to dryness, and the residue was purified by column chromatography (petroleum ether: ethyl acetate (v:v) = 5: 1-1: 1) to obtain compound **11B** (1 g, yield: 76%).

Step 3: methyl 2-(1-(7,7-difluoro-2-((2R,3R)-3-fluoro-2-methylazetidin-1-yl)-6,7-dihydro-5H-cyclopenta[d]pyrimidin-4-yl)azetidin-3-yl)acetate (**11C**)

**11B** (0.11 g, 0.3 mmol), (2R,3R)-3-fluoro-2-methylazetidine (0.08 g, 0.9 mmol) (obtained with reference to the synthetic method in Journal of Medicinal Chemistry, 1994, Vol.37, No.24 4195-4210*)* and DIPEA (0.12 g, 0.9 mmol) were added to isopropanol (10 mL). The mixture was reacted in a sealed tube at 100°C overnight and concentrated directly to dryness, and the residue was purified by column chromatography (petroleum ether: ethyl acetate = 3: 1) to obtain methyl 2-(1-(7,7-difluoro-2-((2R,3R)-3-fluoro-2-methylazetidin-1-yl)-6,7-dihydro-5H-cyclopenta[d]pyrimidin-4-yl)azetidin-3-yl)acetate (**11C**) (110 mg, yield: 85.9%).
LCMS m/z =371.2 [M+1].

Step 2: 2-(1-(7,7-difluoro-2-((2R,3R)-3-fluoro-2-methylazetidin-1-yl)-6,7-dihydro-5H-cyclopenta[d]pyrimidin-4-yl)azetidin-3-yl)acetic acid (**compound 11**)

**11C** (110 mg, 0.3 mmol) and lithium hydroxide (0.14 g, 6 mmol) were dissolved in methanol (4 mL) and water (1 mL), and the mixture was stirred at room temperature for 2 h, concentrated to remove methanol, adjusted to pH 5-6 with 2 N hydrochloric acid and extracted with ethyl acetate (20 mL). The ethyl acetate phase was concentrated to dryness, and separated and purified by a liquid phase preparative column (liquid phase preparative conditions: C18 reverse-phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 7.26 min) to obtain 2-(1-(7,7-difluoro-2-((2R,3R)-3-fluoro-2-methylazetidin-1-yl)-6,7-dihydro-SH-cyclopenta[d]pyrimidin-4-yl)azetidin-3-yl)acetic acid (**compound 11**) (60 mg, yield: 57.1%).
$^1$HNMR (400 MHz, $CDCl_3$) δ 5.01- 4.84 (m, 1H), 4.63 - 4.50 (m, 4H), 4.24 - 4.05 (m, 3H), 3.20 - 3.12 (m, 1H), 2.90 - 2.87 (m, 2H), 2.73 - 2.71 (d, 2H), 2.61 - 2.50 (m, 2H), 1.54 - 1.52 (d, 3H).
LCMS m/z =357.2 $[M+1]^+$.

**Example 12:**

(S)-2-(4-(7,7-difluoro-2-(2-methylazetidin-1-yl)-6,7-dihydro-5H-cyclopenta[d]pyrimidin-4-yl)-1H-pyrazol-1-yl)acetic acid **(compound 12)**

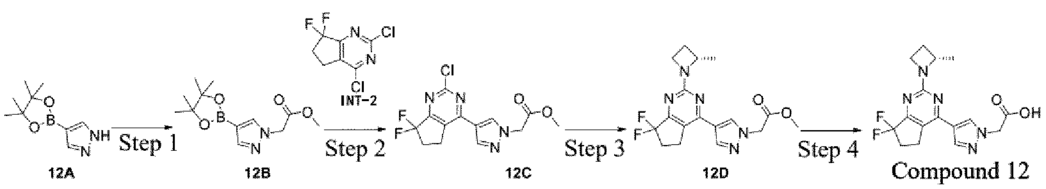


Step 1: methyl 2-(4-(4,4,5,5-tetramethyl-1,3,2-dioxaborolan-2-yl)-1H-pyrazol-1-yl)acetate **(12B)**

Compound **12A** (3 g, 15.5 mmol) was dissolved in N,N-dimethylformamide (30 mL) at room temperature. Methyl bromoacetate (2.8 g, 15.5 mmol) and caesium carbonate (11.1 g, 34.1 mmol) were added, and the mixture was reacted at room temperature for 16 h. The reaction solution was poured into water (150 mL), and the mixture was extracted with ethyl acetate (100 mL × 1). The organic phase was concentrated to dryness, and the residue was purified by column chromatography (petroleum ether: ethyl acetate (v:v) = 10: 1-1: 1) to obtain compound **12B** (3 g, yield: 73%).
LC-MS (ESI): m/z =267.1 $[M+H]^+$.

Step 2: methyl 2-(4-(2-chloro-7,7-difluoro-6,7-dihydro-5H-cyclopenta[d]pyrimidin-4-yl)-1H-pyrazol-1-yl)acetate **(compound 12C)**

Compound **12B** (0.24 g, 0.89 mmol) was dissolved in 1,4-dioxane (20 mL) and water (2 mL) at room temperature. **INT-2** (0.2 g, 0.89 mmol), potassium carbonate (0.26 g, 1.9 mmol) and pd(dppf)$Cl_2$ (65 mg, 0.089 mmol) were added. The mixture was subjected to nitrogen replacement and protection, heated to 100°C and reacted for 4 h. The reaction solution was concentrated to dryness, and the residue was purified by column chromatography (petroleum ether: ethyl acetate (v:v) = 5: 1-1: 1) to obtain compound **12C** (0.2 g, yield: 68%).

Step 3: (S)-methyl 2-(4-(7,7-difluoro-2-(2-methylazetidin-1-yl)-6,7-dihydro-SH-cyclopenta[d]pyrimidin-4-yl)-1H-pyrazol-1-yl)acetate **(compound 12D)**

Compound **12C** (0.2 g, 0.61 mmol) was dissolved in 1,4-dioxane (10 mL) at room temperature. (S)-2-methylazetidine hydrochloride (0.14 g, 1.3 mmol) and diisopropylethylamine (0.32 g, 2.4 mmol) were added, and the mixture was heated to 100°C and reacted in a sealed tube for 8 h. The reaction solution was poured into water (100 mL), and the mixture was extracted with ethyl acetate (50 mL × 1). The organic phase was concentrated to dryness to obtain compound **12D** (0.15 g, yield: 68%).

LC-MS (ESI): m/z =364.4$[M+H]^+$.

Step 4: (S)-2-(4-(7,7-difluoro-2-(2-methylazetidin-1-yl)-6,7-dihydro-5H-cyclopenta[d]pyrimidin-4-yl)-1H-pyrazol-1-yl)acetic acid **(compound 12)**

Compound **12D** (0.15 g, 0.41 mmol) was dissolved in ethanol (3 mL) and water (20 mL) at room temperature. Lithium hydroxide (52 mg, 1.2 mmol) was added, and the mixture was reacted at room temperature for 2 h. Water (20 mL) was added, and the mixture was adjusted to about pH 5-6 with 1 N hydrochloric acid and extracted with ethyl acetate (50 mL × 1). The organic phase was concentrated to dryness to obtain compound **12** (80 mg, yield: 55%).

LC-MS (ESI): m/z=350.3$[M+H]^+$.

$^1H$ NMR (400 MHz,$CDCl_3$) δ8.15 (s,1H), 8.06 (s,1H), 5.03 (s,2H), 4.61-4.55 (m,1H), 4.21-4.04 (m,2H), 2.98-2.97 (m,2H), 2.65-2.43 (m,3H), 2.01-1.98 (m,1H), 1.57-1.55 (m,3H).

**Example 13**

2-((S)-1-(7,7-difluoro-2-((2S,3S)-3-fluoro-2-methylazetidin-1-yl)-6,7-dihydro-5H-cyclopenta[d]pyrimidin-4-yl)pyrrolidin-3-yl)acetic acid 2-((R)-1-(7,7-difluoro-2-((2S,3S)-3-fluoro-2-methylazetidin-1-yl)-6,7-dihydro-5H-cyclopenta[d]pyrimidin-4-yl)pyrrolidin-3-yl)acetic acid 2-((S)-1-(7,7-difluoro-2-((2R,3R)-3-fluoro-2-methylazetidin-1-yl)-6,7-dihydro-5H-cyclopenta[d]pyrimidin-4-yl)pyrrolidin-3-yl)acetic acid 2-((R)-1-(7,7-difluoro-2-((2R,3R)-3-fluoro-2-methylazetidin-1-yl)-6,7-dihydro-5H-cyclopenta[d]pyrimidin-4-yl)pyrrolidin-3-yl)acetic acid **(isomer 1, isomer 2, isomer 3 and isomer 4 of compound 13)**

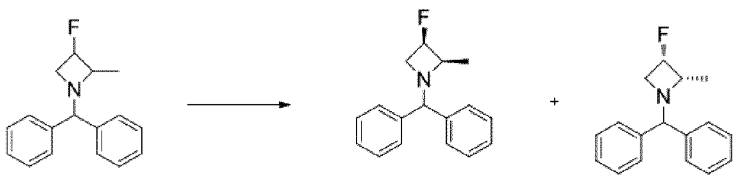

13A

13A-P1 and 13A-P2

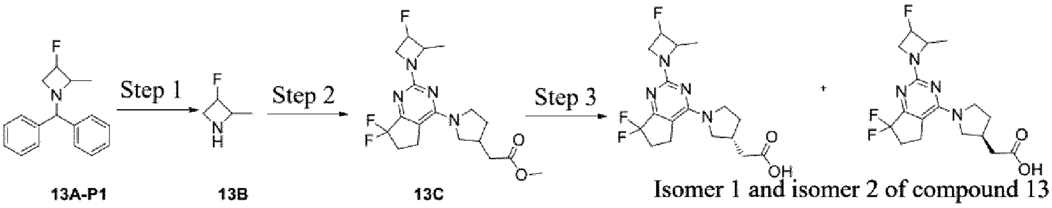


13A-P1 13B 13C Isomer 1 and isomer 2 of compound 13

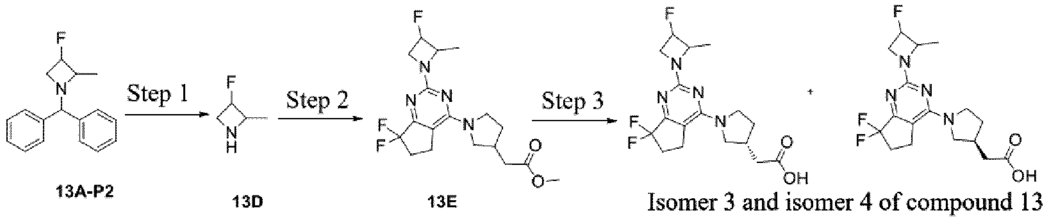


13A-P2 13D 13E Isomer 3 and isomer 4 of compound 13

Step 1: (2S,3S)-3-fluoro-2-methylazetidine (**13B**)

(**13A-P1**) (0.6 g, 2.35 mmol) (obtained with reference to the synthetic method in Journal of Medicinal Chemistry, 1994, Vol.37, No.24 4195-4210, with the resolved P1) (0.8 g, 3.13 mmol) (retention time: 2.705 min) (chiral resolution conditions: instrument: Waters UPC2 analytical SFC(SFC-H); chromatographic column: ChiralCel OJ, 250 × 30 mm I.D., 10 μm; mobile phase: A: $CO_2$, B: methanol (0.1% $NH_3{\cdot}H_2O$); gradient: B 20%; flow rate: 60 mL/min; wavelength: 220 nm; back pressure: 100 bar; column temperature: 35°C) and isopropyl alcohol (10 mL) were added to an autoclave, and 20% palladium hydroxide (0.3 g, 2.35 mmol) was added. The mixture was subjected to hydrogen replacement to achieve 30 atm, stirred at room temperature for 4 h, filtered and washed with a small amount of isopropyl alcohol (5 mL). The mother liquor obtained was used directly in the next step.
LCMS m/z =90.1 $[M+1]^+$.

Step 2: methyl 2-(1-(7,7-difluoro-2-((2S,3S)-3-fluoro-2-methylazetidin-1-yl)-6,7-dihydro-5H-cyclopenta[d]pyrimidin-4-yl)pyrrolidin-3-yl)acetate (13C)

A solution of (**13B**) (209 mg, 2.35 mmol) in isopropanol, **9B** (0.8 g, 2.13 mmol) and DIPEA (0.85 g, 6.39 mmol) were added to a sealed tube. The mixture was reacted at 100°C overnight and concentrated directly to dryness, and the residue was purified by column chromatography (petroleum ether: ethyl acetate = 3: 1) to obtain methyl 2-(1-(7,7-difluoro-2-((2S,3S)-3-fluoro-2-methylazetidin-1-yl)-6,7-dihydro-5H-cyclopenta[d]pyrimidin-4-yl)pyrrolidin-3-yl)acetate (**13C**) (630 mg, yield: 77%).
LCMS m/z =385.2 $[M+1]^+$.

Step 3: 2-((S)-1-(7,7-difluoro-2-((2S,3S)-3-fluoro-2-methylazetidin-1-yl)-6,7-dihydro-5H-cyclopenta[d]pyrimidin-4-yl)pyrrolidin-3-yl)acetic acid 2-((R)-1-(7,7-difluoro-2-((25,3S)-3-fluoro-2-methylazetidin-1-yl)-6,7-dihydro-5H-cyclopenta[d]pyrimidin-4-yl)pyrrolidin-3-yl)acetic acid (**isomer 1 of compound 13 and isomer 2 of compound 13)**

(**13C**) (630 mg, 1.63 mmol) and lithium hydroxide (0.3 g, 7.14 mmol) were dissolved in methanol (8 mL) and water (2 mL), and the mixture was stirred at room temperature for 2 h, concentrated to remove methanol, adjusted to pH 5-6 with 2 N hydrochloric acid and extracted with ethyl acetate (20 mL). The ethyl acetate phase was concentrated to dryness (chiral resolution conditions: instrument: Waters 150 mgm, chromatographic column: DAICEL CHIRALPAK AY(250 mm × 30 mm, 10 μm); mobile phase: A: $CO_2$, B: IPA (0.1% $NH_3{\cdot}H_2O$); gradient: B 30%; flow rate: 130 mL/min; wavelength: 220 nm; back pressure: 100 bar; column temperature: 35°C) to obtain **isomer 1 of compound 13** (retention time: 1.443 min) (197 mg, yield: 32.4%) and **isomer 2 of compound 13** (retention time: 1.816 min) (0.202 mg, yield: 33.3%).
With reference to the synthetic methods of **isomer 1 and isomer 2 of the compound 13, 13A-P2** (0.8 g, 3.13 mmol) (obtained with reference to the synthetic method in Journal of Medicinal Chemistry, 1994, Vol. 37, No.24 4195-4210, with the resolved P2) (0.8 g, 3.13 mmol, retention time: 2.979 min) was used as a raw material to synthesize **isomer 3 of compound 13** (retention time: 1.432 min, 0.176 g, yield: 39%) and **isomer 4 of compound 13** (retention time: 1.915 min, 0.176 g, yield: 37.7%).
13A-P1 and 13A-P2 have structures as follows:

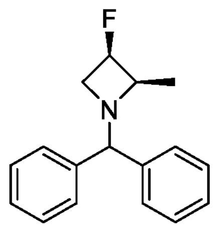

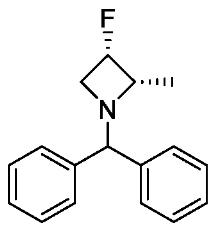.

Isomer 1 of compound 13, isomer 2 of compound 13, isomer 3 of compound 13 and isomer 4 of compound 13 have structures as follows:

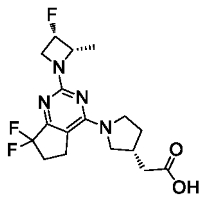

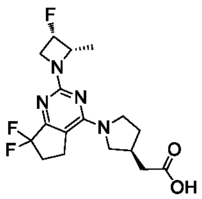

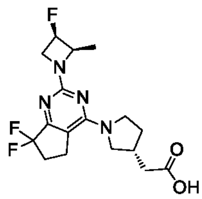

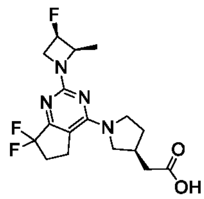.

**Isomer 1 of compound 13** $^1$HNMR (400 MHz, DMSO-$d_6$) δ 12.18 (s, 1H), 5.12 - 4.93 (m, 1H), 4.28 - 4.17 (m, 2H), 3.84 - 3.59 (m, 4H), 3.26 - 3.07 (m, 3H), 2.49 - 2.33 (m, 5H), 2.08 - 2.05 (m, 1H), 1.62 - 1.59 (m, 1H), 1.47 - 1.45 (d, 3H).

LCMS m/z =371.2 $[M+1]^+$.

**Isomer 2 of compound 13** $^1$HNMR (400 MHz, DMSO-$d_6$) δ 12.13 (s, 1H), 5.11 - 4.96 (m, 1H), 4.27 - 4.16 (m, 2H), 3.83 - 3.58 (m, 4H), 3.29 - 3.07 (m, 3H), 2.49 - 2.33 (m, 5H), 2.10 - 2.07 (m, 1H), 1.62 - 1.59 (m, 1H), 1.47 - 1.45 (d, 3H).

LCMS m/z =371.2 $[M+1]^+$.

**Isomer 3 of compound 13** $^1$HNMR (400 MHz, DMSO-$d_6$) δ 12.33 (s, 1H), 5.12 - 4.94 (m, 1H), 4.28 - 4.16 (m, 2H), 3.84 - 3.59 (m, 4H), 3.25 - 3.05 (m, 3H), 2.49 - 2.32 (m, 5H), 2.08 - 2.05 (m, 1H), 1.62 - 1.59 (m, 1H), 1.47 - 1.45 (d, 3H).

LCMS m/z =371.2 $[M+1]^+$.

**Isomer 4 of compound 13** $^1$HNMR (400 MHz, DMSO-$d_6$) δ 12.18 (s, 1H), 5.11 - 4.96 (m, 1H), 4.28 - 4.16 (m, 2H), 3.84 - 3.58 (m, 4H), 3.26 - 3.07 (m, 3H), 2.49 - 2.33 (m, 5H), 2.10 - 2.07 (m, 1H), 1.62 - 1.59 (m, 1H), 1.47 - 1.45 (d, 3H).

LCMS m/z =371.2 $[M+1]^+$.

**Example 14:**

2-((1R,5S,6S)-3-(2-((S)-2-methylazetidin-1-yl)-6,7-dihydro-5H-cyclopenta[d]pyrimidin-4-yl)-3-azabicyclo[3.1.1]heptan-6-yl)acetic acid 2-((1R,5S,6R)-3-(2-((S)-2-methylazetidin-1-yl)-6,7-dihydro-5H-cyclopenta[d]pyrimidin-4-yl)-3-azabicyclo[3.1.1]heptan-6-yl)acetic acid **(isomer 1 of compound 14 and isomer 2 of compound 14)**

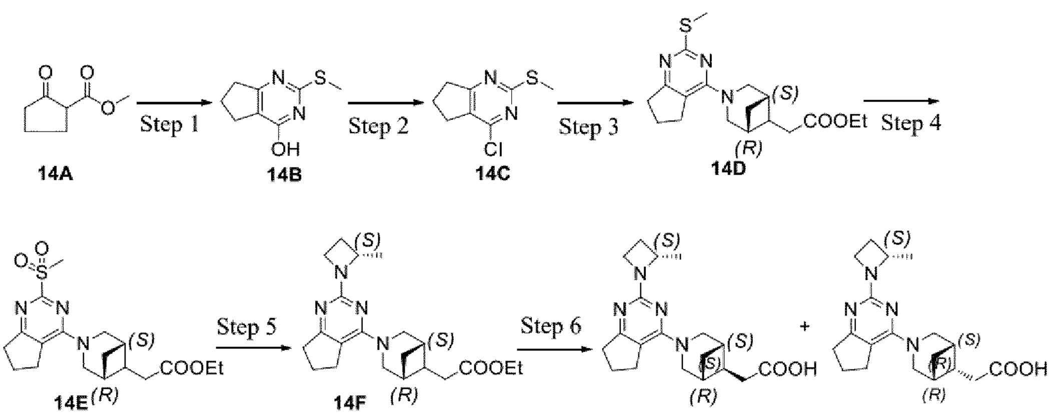


Isomer 1 and isomer 2 of compound 14

Step 1: 2-(methylthio)-6,7-dihydro-5H-cyclopenta[d]pyrimidin-4-ol **(compound 14B)**

Compound **14A** (10 g, 70.35 mmol) was dissolved in water (100 mL) at room temperature. 2-methyl-2-thiourea sulphate (15.90 g, 84.42 mmol) and sodium carbonate (18.64 g, 175.88 mmol) were added, and the mixture was reacted for 16 h. The reaction solution was adjusted to pH 6-7 with 6 N hydrochloric acid and filtered, and the solid was dried to obtain compound **14B** (6.5 g, yield: 51%).
LC-MS (ESI): m/z =183.1 $[M+H]^+$.

Step 2: 4-chloro-2-(methylthio)-6,7-dihydro-5H-cyclopenta[d]pyrimidine **(compound 14C)**

Compound **14B** (2 g, 10.97 mmol) was dissolved in dichloroethane (40 mL) at room temperature. Phosphorus oxychloride (16.80 g, 109.7 mmol) was added, and the mixture was reacted at 90°C for 2 h. The reaction solution was poured into ice water (200 mL), and the mixture was adjusted to about pH 7-8 with potassium carbonate and extracted with ethyl acetate (100 mL × 1). The organic phase was concentrated to dryness to obtain compound **14C** (2.0 g, yield: 91%).

Step 3: ethyl 2-((1R,5S)-3-(2-(methylthio)-6,7-dihydro-5H-cyclopenta[d]pyrimidin-4-yl)-3-azabicyclo[3.1.1]heptan-6-yl)acetate **(compound 14D)**

Compound **14C** (0.5 g, 2.49 mmol) was dissolved in N-methylpyrrolidone (10 mL) at room temperature. Ethyl 2-((1R,5S)-3-azabicyclo[3.1.1]heptan-6-yl)acetate hydrochloride (0.82 g, 3.74 mmol) and potassium carbonate (1.03 g, 7.47 mmol) were added, and the mixture was heated to 100°C and reacted for 4 h. The reaction solution was poured into water (100 mL), and the mixture was extracted with ethyl acetate (50 mL × 1). The organic phase was concentrated to dryness, and the residue was purified by column chromatography (petroleum ether: ethyl acetate (v:v) = 10: 1-2: 1) to obtain compound **14D** (0.4 g, yield: 46%).

Step 4: ethyl 2-((1R,5S)-3-(2-(methylsulfonyl)-6,7-dihydro-5H-cyclopenta[d]pyrimidin-4-yl)-3-azabicyclo[3.1.1]heptan-6-yl)acetate **(compound 14E)**

Compound **14D** (0.4 g, 1.15 mmol) was dissolved in dichloromethane (30 mL) at room temperature, and m-chloroperoxybenzoic acid (0.44 g, 2.53 mmol) was added. The mixture was reacted at room temperature for 2 h. The reaction solution was washed with a saturated sodium bicarbonate solution (20 mL × 2). The organic phase was concentrated to dryness, and the residue was purified by column chromatography (petroleum ether: ethyl acetate (v:v) = 5: 1-1: 1) to obtain compound **14E** (0.3 g, yield: 69%).

Step 5: ethyl 2-((1R,SS)-3-(2-((S)-2-methylazetidin-1-yl)-6,7-dihydro-5H-cyclopenta[d]pyrimidin-4-yl)-3-azabicyclo[3.1.1]heptan-6-yl)acetate **(compound 14F)**

Compound **14E** (0.3 g, 0.79 mmol) was dissolved in 1,4-dioxane (20 mL) at room temperature. (S)-2-methylazetidine hydrochloride (0.21 g, 1.98 mmol) and diisopropylethylamine (0.41 g, 3.16 mmol) were added, and the mixture was heated to 100°C and reacted in a sealed tube for 16 h. The reaction solution was poured into water (100 mL), and the mixture was extracted with ethyl acetate (50 mL × 1). The organic phase was concentrated to dryness, and the residue was purified by column chromatography (petroleum ether: ethyl acetate (v:v) = 5: 1-1: 1) to obtain compound **14F** (0.2 g, yield: 68%).
LC-MS (ESI): m/z =371.5$[M+H]^+$.

Step 6: 2-((1R,5S,6S)-3-(2-((S)-2-methylazetidin-1-yl)-6,7-dihydro-5H-cyclopenta[d]pyrimidin-4-yl)-3-azabicyclo[3.1.1]heptan-6-yl)acetic acid 2-((1R,5S,6R)-3-(2-((S)-2-methylazetidin-1-yl)-6,7-dihydro-5H-cyclopenta[d]pyrimidin-4-yl)-3-azabicyclo[3.1.1]heptan-6-yl)acetic acid **(isomer 1 of compound 14 and isomer 2 of compound 14)**

Compound **14F** (0.2 g, 0.54 mmol) was dissolved in ethanol (2 mL) and water (10 mL) at room temperature. Lithium hydroxide (68 mg, 1.62 mmol) was added, and the mixture was reacted at room temperature for 2 h. Water (20 mL) was added, and the mixture was adjusted to about pH 6-7 with acetic acid and extracted with ethyl acetate (20 mL × 1).The organic phase was concentrated to dryness, and the residue was subjected to chiral preparative separation (separation conditions: preparative instrument: Waters 150 Mgm; preparative column: DAICEL CHIRALPAK AD (250 mm × 30 mm, 10 μm); mobile phase: A: $CO_2$, B: methanol (0.1% $NH_3 \cdot H_2O$); gradient elution: B 20%; elution time: 4 min; flow rate: 140 mL/min; column temperature: 35°C) to obtain **isomer 1 of compound 14** (56.2 mg, retention time: 1.40 min, yield: 62%) and **isomer 2 of compound 14** (39.8 mg, retention time: 1.60 min, yield: 44%).

**Isomer 1 of compound 14:** LC-MS (ESI): m/z =343.4[M+H]$^+$.

$^1$H NMR (400 MHz, $CD_3OD$) δ4.57-4.52 (m,1H), 4.11-3.95 (m,6H), 3.24-3.21 (m,2H), 2.79-2.75 (m,2H), 2.67-2.61 (m,1H), 2.54-2.50 (m,3H), 2.23-2.21 (m,2H), 2.12-1.99 (m,4H),1.52-1.50 (m,3H), 1.43-1.41 (m,1H).

**Isomer 2 of compound 14:** LC-MS (ESI): m/z =343.4[M+H]$^+$.

$^1$H NMR (400 MHz, $CD_3OD$) δ4.51-4.46 (m,1H), 4.07-3.90 (m,6H), 3.24-3.17 (m,2H), 2.76-2.72 (m,2H), 2.56-2.54 (m,2H), 2.49-2.41 (m,2H), 2.31-2.29 (m,2H), 2.10-1.97 (m,4H),1.50-1.49 (m,3H), 1.39-1.35 (m,1H).

**Example 15**

2-(1-((R)-7-methyl-2-((S)-2-methylazetidin-1-yl)-6,7-dihydro-5H-cyclopenta[d]pyrimidin-4-yl)azetidin-3-yl)acetic acid
2-(1-((S)-7-methyl-2-((S)-2-methylazetidin-1-yl)-6,7-dihydro-5H-cyclopenta[d]pyrimidin-4-yl)azetidin-3-yl)acetic acid
(**isomer 1 of compound 15 and isomer 2 of compound 15**)

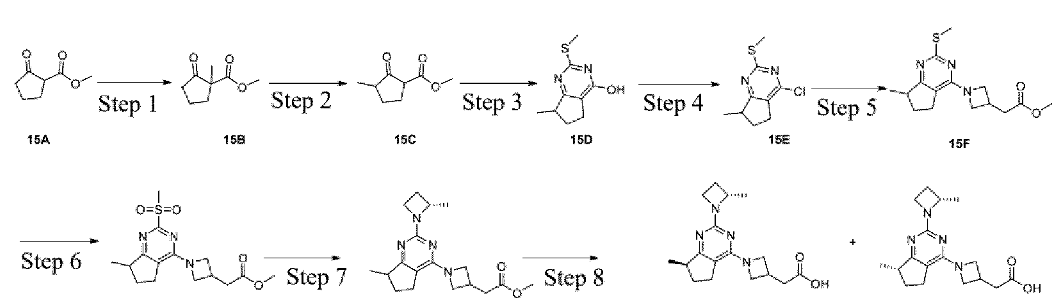


Isomer 1 and isomer 2 of compound 15

Step 1: methyl 1-methyl-2-oxocyclopentane-1-carboxylate (**15B**)

(**15A**) (14.0 g, 98 mmol), methyl iodide (34.9 g, 246 mmol) and potassium carbonate (34.2 g, 246 mmol) were added to acetone (120 mL), and the reaction was refluxed for 12 h. The mixture was concentrated under reduced pressure to remove most of the solvent. Water (200 mL) was added, and the mixture was extracted with ethyl acetate (200 mL). The ethyl acetate phase was concentrated to dryness to obtain **15B** (14.0 g, 91%).

Step 2: methyl 3-methyl-2-oxocyclopentane-1-carboxylate (**15C**)

(**15B**) (14.0 g, 89 mmol) was added to a solution of sodium methoxide in methanol (230 mL, 0.78 mol/L), and the mixture was reacted at 70°C for 3 h. The mixture was concentrated to dryness under reduced pressure, and then toluene (230 mL) was added. The mixture was reacted at 100°C for 5 h and concentrated under reduced pressure to remove toluene. Water (200 mL) was added, and the mixture was extracted with ethyl acetate (200 mL). The ethyl acetate phase was concentrated to dryness, and the residue was separated by column chromatography (PE:EA = 5: 1) to obtain methyl 3-methyl-2-oxocyclopentane-1-carboxylate (**15C**) (10.5 g, 75%).

LC-MS (ESI): m/z =157.2 [M+H] $^+$.

$^1$H NMR (400 MHz, $CDCl_3$): 3.75 - 3.74 (m, 3H), 3.28 - 3.12 (m, 1H), 2.30 - 2.09 (m, 3H), 1.70 - 1.43 (m, 1H), 1.22 - 1.20 (m, 1H), 1.15 - 1.13(m, 3H).

Step 3: 7-methyl-2-(methylthio)-6,7-dihydro-5H-cyclopenta[d]pyrimidin-4-ol (**15D**)

(**15C**) (10.5 g, 67 mmol), thioxamidsaeure-methylester (19.0 g, 100 mmol) and sodium carbonate (17.8 g, 167 mmol) were added to water (105 mL), and the mixture was stirred at room temperature overnight and adjusted to pH 5-6 with 2 N hydrochloric acid, and a large amount of solid was precipitated and filtered to obtain 7-methyl-2-(methylthio)-6,7-dihydro-5H-cyclopenta[d]pyrimidin-4-ol (**15D**) (9.0 g, 68%).

LC-MS (ESI): m/z =197.1 [M+H] $^+$.

Step 4: 4-chloro-7-methyl-2-(methylthio)-6,7-dihydro-5H-cyclopenta[d]pyrimidine (**15E**)

(**15D**) (3.0 g, 15.3 mmol) was added to a mixed solution of phosphorus oxychloride (3 mL) and 1,2-dichloroethane (6 mL), and the mixture was reacted at 90°C for 2 h and concentrated to dryness. Water (50 mL) was added, and the mixture was extracted with ethyl acetate (50 mL). The ethyl acetate phase was concentrated to dryness to obtain 4-chloro-7-methyl-2-(methylthio)-6,7-dihydro-5H-cyclopenta[d]pyrimidine (**15E**) (3.0 g, 91%).

Step 5: methyl 2-(1-(7-methyl-2-(methylthio)-6,7-dihydro-5H-cyclopenta[d]pyrimidin-4-yl)azetidin-3-yl)acetate (**15F**)

(**15E**) (0.5 g, 2.3 mmol), methyl 2-(azetidin-3-yl)acetate trifluoroacetate (0.75 g, 5.83 mmol) and potassium carbonate (0.96 g, 7.0 mmol) were added to N-methylpyrrolidinone (5 mL), and the mixture was reacted at 100°C for 4 h. Water (50 mL) was added, and the mixture was extracted with ethyl acetate (50 mL). The ethyl acetate phase was concentrated directly to dryness, and the residue was purified by column chromatography (petroleum ether: ethyl acetate = 2: 1) to obtain methyl 2-(1-(7-methyl-2-(methylthio)-6,7-dihydro-5H-cyclopenta[d]pyrimidin-4-yl)azetidin-3-yl)acetate (**15F**) (0.6 g, yield: 84%).
LC-MS (ESI): m/z =308.1 $[M+H]^+$.

Step 6: methyl 2-(1-(7-methyl-2-(methylsulfonyl)-6,7-dihydro-5H-cyclopenta[d]pyrimidin-4-yl)azetidin-3-yl)acetate (**15G**)

Compound **15F** (700 mg, 2.28 mmol) was dissolved in 50 mL of dichloromethane, and m-chloroperoxybenzoic acid (784 mg, 4.56 mmol) was added with stirring. The mixture was stirred at room temperature overnight. After LCMS showed that the reaction was completed, a saturated aqueous sodium bicarbonate solution was added, and the mixture was extracted with dichloromethane. The organic phase was dried and concentrated, and the residue was subjected to column chromatography (PE:EA = 1: 1) to obtain compound **15G** (655 mg, 85%).
LC-MS (ESI): m/z =340.1 $[M+H]^+$.
$^1H$ NMR (400 MHz, $CDCl_3$): 4.41-4.47(m, 1H), 4.29-4.33(m, 2H), 4.08-4.10(m, 1H), 3.97-3.99(m, 1H), 3.84-3.89(m, 2H), 3.01-3.06(m, 2H), 2.73-2.75(m, 1H), 2.57-2.67(m, 2H), 2.36-2.37(m, 1H), 2.20-2.22(m, 1H), 1.91(t, 1H), 1.55-1.57(m, 1H), 1.46-1.48(m, 3H), 1.20-1.29(m, 5H).

Step 7: methyl 2-(1-(7-methyl-2-((S)-2-methylazetidin-1-yl)-6,7-dihydro-5H-cyclopenta[d]pyrimidin-4-yl)azetidin-3-yl)acetate (**15H**)

Compound **15G** (655 mg, 1.93 mmol) was dissolved in 10 mL of 1,4-dioxane. Triethylamine (1.25 mL, 9.65 mmol) and (S)-2-methylazetidine hydrochloride (623 mg, 5.79 mmol) were added, and in a sealed tube, the mixture was heated to 110°C and reacted overnight. After LCMS showed that the reaction of raw materials was completed, the reaction solution was concentrated and extracted with EA and saturated brine. The organic phase was dried and concentrated, and the residue was subjected to column chromatography (DCM:MeOH = 15: 1) to obtain compound **15H** (330 mg, 52%).
LC-MS (ESI): m/z =331.2 $[M+H]^+$.

Step 8: 2-(1-((R)-7-methyl-2-((S)-2-methylazetidin-1-yl)-6,7-dihydro-5H-cyclopenta[d]pyrimidin-4-yl)azetidin-3-yl)acetic acid 2-(1-((S)-7-methyl-2-((S)-2-methylazetidin-1-yl)-6,7-dihydro-5H-cyclopenta[d]pyrimidin-4-yl)azetidin-3-yl)acetic acid (**isomer 1 of compound 15 and isomer 2 of compound 15)**

Compound **15H** (330 mg, 1 mmol) was dissolved in 10 mL of methanol, and lithium hydroxide (210 mg, 5 mmol) dissolved in 2 mL of water was added. The mixture was stirred at room temperature for 2 h. After LCMS showed that the reaction was completed, the reaction solution was concentrated, adjusted to pH 5-6 with 2 N aqueous hydrochloric acid and extracted with EA. The organic phase was dried and concentrated, and the residue was separated by column chromatography (DCM:MeOH = 10: 1) to obtain a mixture (285 mg, 90%), which was subjected to chiral preparative separation (separation conditions: preparative instrument: waters 150 Mgm; preparative column: DAICEL CHIRALPAK OJ (250 mm × 30 mm, 10 μm); mobile phase: A: $CO_2$, B: ethanol (0.1% $NH_3 \cdot H_2O$); gradient elution: B 10%; elution time: 2.1 min; flow rate: 140 mL/min; column temperature: 35°C) and subjected to chiral resolution to obtain **isomer 1 of compound 15** (110 mg, retention time: 1.356 min) and **isomer 2 of compound 15** (98 mg, retention time: 1.978 min).
**Isomer 1 of compound 15:** LC-MS (ESI): m/z =317.2 $[M+H]^+$.
$^1H$ NMR (400 MHz, $CDCl_3$): 4.41-4.47(m, 1H), 4.29-4.33(m, 2H), 4.08-4.10(m, 1H), 3.97-3.99(m, 1H), 3.84-3.89(m, 2H), 3.01-3.06(m, 2H), 2.73-2.75(m, 1H), 2.57-2.67(m, 2H), 2.36-2.37(m, 1H), 2.20-2.22(m, 1H), 1.91(t, 1H), 1.55-1.57(m, 1H), 1.46-1.48(m, 3H), 1.20-1.29(m, 5H).
**Isomer 2 of compound 15:** LC-MS (ESI): m/z =317.2 $[M+H]^+$.
$^1H$ NMR (400 MHz, $CDCl_3$): 4.56-4.58(m, 1H), 4.33-4.42(m, 4H), 3.98(s, 2H), 3.36-3.39(m, 1H), 3.08(t, 1H), 2.64-2.83(m, 4H), 2.43-2.52(m, 1H), 2.21-2.26(m, 1H), 1.93-1.97(m, 1H), 1.66-1.72(m, 1H), 1.49(d, 3H), 1.32(d, 3H).

**Example 16:**

2-((1R,5S,6R)-3-(7,7-difluoro-2-((S)-2-(methyl-d3)azetidin-1-yl)-6,7-dihydro-5H-cyclopenta[d]pyrimidin-4-yl)-3-azabicyclo[3.1.1]heptan-6-yl)acetic acid (**compound 16**)

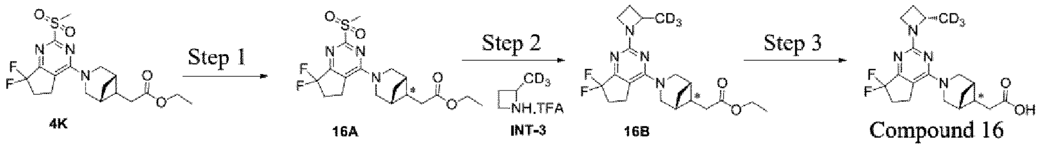


4K 16A 16B Compound 16

Step 1: ethyl 2-((1R,5S)-3-(7,7-difluoro-2-(methylsulfonyl)-6,7-dihydro-5H-cyclopenta[d]pyrimidin-4-yl)-3-azabicyclo[3.1.1]heptan-6-yl)acetate (**16A**) g). **4K** (10.5 g) was subjected to chiral separation to obtain compound **16A** (5.4

The chiral preparative separation method: 1. Instrument: Waters SFC 350; chromatographic column: DAICEL CHIRALPAK OD (250 mm × 50 mm, 10 μm); 2. Preparative chromatography conditions: A for $CO_2$ and B for IPA (0.1% $NH_3 \cdot H_2O$); flow rate: 200 mL/min; retention time: 1.865 min; elution time: 6.9 min.

Step 2: ethyl 2-((1R,SS,6S)-3-(7,7-difluoro-2-(2-(methyl-d3)azetidin-1-yl)-6,7-dihydro-5H-cyclopenta[d]pyrimidin-4-yl)-3-azabicyclo[3.1.1]heptan-6-yl)acetate (**16B**)

**16A** (1.2 g, 2.89 mmol), **INT-3** (1.63 g, 8.67 mmol), N,N-diisopropylethylamine (2.61 g, 20.23 mmol) and 1,4-dioxane (15 mL) were added to a sealed tube, and the mixture was reacted at 100°C for 16 h and cooled to room temperature. Water (30 mL) was added, and the mixture was extracted with ethyl acetate. The organic phase was washed with a saturated sodium chloride solution, dried over anhydrous sodium sulphate and filtered. The filtrate was concentrated under reduced pressure, and then the residue was separated and purified by silica gel column chromatography (EA: PE (v/v) = 1: 3) to obtain the title compound **16B** (1.1 g, 93%).

LC-MS (ESI): m/z = 410.2 $[M+H]^+$.

Step 3: 2-((1R,SS,6R)-3-(7,7-difluoro-2-((S)-2-(methyl-d3)azetidin-1-yl)-6,7-dihydro-5H-cyclopenta[d]pyrimidin-4-yl)-3-azabicyclo[3.1.1]heptan-6-yl)acetic acid (**compound 16**)

**16B** (1.1 g, 2.69 mmol) and lithium hydroxide (0.56 g, 13.45 mmol) were added to methanol (15 mL) and water (5 mL), and the mixture was reacted at room temperature for 2 h. The system was concentrated under reduced pressure to remove methanol. Water (20 mL) was added, and the pH was adjusted to 5 with acetic acid, and a white solid was precipitated, filtered, washed with water and dried under reduced pressure to obtain 0.95 g of a white solid, which was subjected to chiral separation to obtain the target **compound 16** (0.4 g, 39%) (retention time: 1.326 min).

The chiral preparative separation method: 1. Instrument: MG II preparative SFC (SFC-14); chromatographic column: ChiralPak AD, 250 × 30 mm I.D., 10 μm; 2. Preparative chromatography conditions: A for $CO_2$ and B for ethanol (0.1% $NH_3 \cdot H_2O$) B 30%; flow rate: 70 mL/min; retention time: 1.972 min; elution time: 3 min.

$^1H$ NMR (400 MHz, $CDCl_3$) δ 4.45 (t, 1H), 4.18-4.08 (m, 1H), 4.06-3.94 (m, 5H), 3.20-3.09 (m, 2H), 2.73 (d, 2H), 2.50-2.31 (m, 6H), 2.16-2.11 (m, 1H), 1.98-1.87 (m, 1H), 1.44 (dd, 1H).

LC-MS (ESI): m/z = 382.2 $[M+H]^+$.

**Example 17:**

2-(1-(4-cyano-5,5-difluoro-3-(2-methylazetidin-1-yl)-6,7-dihydro-5H-cyclopenta[c]pyridin-1-yl)azetidin-3-yl)acetic acid (**isomer 1 and isomer 2 of compound 17**)

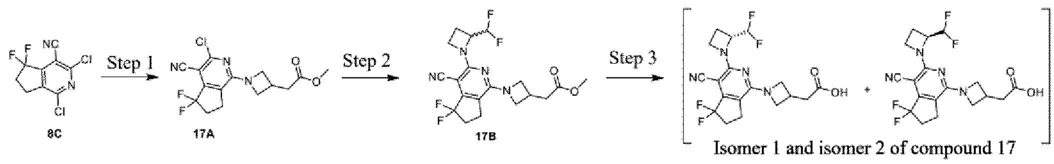


8C 17A 17B

Step 1: methyl 2-(1-(3-chloro-4-cyano-5,5-difluoro-6,7-dihydro-5H-cyclopenta[c]pyridin-1-yl)azetidin-3-yl)acetate (**17A**)

(**8C**) (200 mg, 0.8 mmol) was dissolved in isopropanol (3 mL), and **18G** (155 mg, 1.2 mmol) and N,N-diisopropylethylamine (517 mg, 4 mmol) were added. The mixture was stirred at 40°C for 30 min and filtered to obtain the target compound (**17A**) (270 mg, 99%).
LCMS m/z =342.0 [M+1].

Step 2: (S)-methyl 2-(1-(4-cyano-3-(2-(difluoromethyl)azetidin-1-yl)-5,5-difluoro-6,7-dihydro-5H-cyclopenta[c]pyridin-1-yl)azetidin-3-yl)acetate (**17B**)

(**17A**) (300 mg, 0.88 mmol) was dissolved in dimethyl sulfoxide (3 mL). 2-(difluoromethyl)azetidine trifluoroacetate (389.17 mg, 1.76 mmol) and sodium bicarbonate (221.79 mg, 2.64 mmol) were added, and the mixture was warmed to 100°C and reacted for 16 h. After the reaction was completed, the mixture was cooled to room temperature, and the reaction was quenched by adding water and extracted with ethyl acetate. The organic layers were combined and washed with saturated brine, and the residue was separated and purified by silica gel column chromatography (ethyl acetate: petroleum ether (v/v) = 0-50%) to obtain the title compound **17B** (300 mg, 82.6%).
LCMS m/z =413.2 $[M+1]^+$.

Step 3: methyl 2-(1-(4-cyano-3-(2-(difluoromethyl)azetidin-1-yl)-5,5-difluoro-6,7-dihydro -5H-cyclopenta[c]pyridin-1-yl)azetidin-3-yl)acetate (**compound 17**)

(**Compound 17B**) (300 mg, 0.73 mmol) was dissolved in methanol/water (5/2 mL). LiOH (87.42 mg, 3.65 mmol) was added, and the mixture was reacted at 25°C for 2 h, adjusted to pH=3 with 1 N hydrochloric acid and extracted with ethyl acetate (10 mL × 3). The combined organic phase was dried over anhydrous sodium sulphate, filtered and concentrated, and the residue was separated and purified by silica gel column chromatography (methanol: dichloromethane (v/v) = 0-15%) to obtain the title **compound 17** (250 mg, 86%).
LCMS m/z =399.1 $[M+1]^+$.
Resolution of **isomer 1** and **isomer 2 of compound 17:**
**Compound 17** was subjected to resolution to obtain two optical isomers, **isomer 1 of compound 17** (retention time: 1.430 min, 86 mg, ee% = 100%) and **isomer 2 of compound 17** (retention time: 1.710 min, 102 mg, ee% = 99%).
Resolution conditions:

instrument: Waters UPC2 analytical SFC (SFC-H); column: ChiralCel OJ, 250 × 30 mm I.D., 10 μm;
mobile phase: A: $CO_2$, B: ethanol (0.1% $NH_3 \cdot H_2O$); gradient: B 25%; flow rate: 70 mL/min; back pressure: 100 bar; column temperature: 38°C; wavelength: 220 nm; cycle: 4.6 min; sample preparation: dissolving compounds 17A and 17B in methanol/dichloromethane (25 mL); injection: 1 mL/injection.

**Isomer 1 of compound 17:**

$^1$H NMR (400 MHz, $CDCl_3$) δ 6.34-6.02 (m, 1H), 4.76-4.59 (m, 1H), 4.49 (td, J = 8.7, 5.0 Hz, 1H), 4.39 (dt, J = 17.6, 8.6 Hz, 2H), 4.25 (dd, J = 15.8, 8.6 Hz, 1H), 3.94 (dd, J = 9.0, 5.6 Hz, 1H), 3.88 (dd, J = 8.9, 5.6 Hz, 1H), 3.10 (dt, J = 13.3, 5.5 Hz, 1H), 2.84 (dq, J = 7.5, 3.7 Hz, 2H), 2.77 (s, 1H), 2.76 (s, 1H), 2.65-2.45 (m, 3H), 2.43-2.24 (m, 1H).
LCMS m/z =399.1 $[M+1]^+$.

**Isomer 2 of compound 17:**

$^1$H NMR (400 MHz, $CDCl_3$) δ 6.37-6.00 (m, 1H), 4.75-4.61 (m, 1H), 4.50 (td, J = 8.7, 5.0 Hz, 1H), 4.39 (dt, J = 17.5, 8.6 Hz, 2H), 4.25 (dd, J = 15.8, 8.7 Hz, 1H), 3.94 (dd, J = 9.0, 5.6 Hz, 1H), 3.89 (dd, J = 9.0, 5.6 Hz, 1H), 3.16-3.01 (m, 1H), 2.84 (dq, J = 7.5, 3.7 Hz, 2H), 2.78 (s, 1H), 2.76 (s, 1H), 2.67-2.45 (m, 3H), 2.41-2.27 (m, 1H).
LCMS m/z =399.1 $[M+1]^+$.

**Example 18:**

(S)-2-(1-(4-cyano-5,5-difluoro-3-(2-(methyl-d3)azetidin-1-yl)-6,7-dihydro-SH-cyclopenta[c]pyridin-1-yl)azetidin-3-yl)acetic acid (**compound 18**)

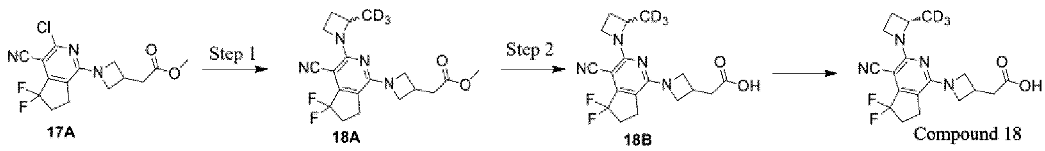


Step 1: methyl 2-(1-(4-cyano-5,5-difluoro-3-(2-(methyl-d3)azetidin-1-yl)-6,7-dihydro-SH-cyclopenta[c]pyridin-1-yl)azetidin-3-yl)acetate (**18A**)

(**17A**) (570 mg, 1.67 mmol) was dissolved in dimethyl sulfoxide (6 mL). 2-(methyl-d3)azetidine hydrochloride (628 mg, 3.34 mmol) and sodium bicarbonate (421 mg, 5.01 mmol) were added, and the mixture was warmed to 100°C and reacted for 8 h. After the reaction was completed, the mixture was cooled to room temperature, and the reaction was quenched by adding water and extracted with ethyl acetate. The organic layers were combined and washed with saturated brine, and the residue was separated and purified by silica gel column chromatography (ethyl acetate: petroleum ether (v/v) = 0-50%) to obtain the title compound **18A** (580 mg, 91.5%).
LCMS m/z =380.2 [M+1].

Step 2: (S)-2-(1-(4-cyano-5,5-difluoro-3-(2-(methyl-d3)azetidin-1-yl)-6,7-dihydro-5H-cyclopenta[c]pyridin-1-yl)azetidin-3-yl)acetic acid (**compound 18B**)

**Compound 18A** (780 mg, 2.06 mmol) was dissolved in methanol/water (5/2 mL). LiOH (247 mg, 10.3 mmol) was added, and the mixture was reacted at 25°C for 3 h, adjusted to pH=3 with 1 N hydrochloric acid and extracted with ethyl acetate (10 mL × 3). The combined organic phase was dried over anhydrous sodium sulphate, filtered and concentrated, and the residue was separated and purified by silica gel column chromatography (methanol: dichloromethane (v/v) = 0-15%) to obtain the title compound **18B** (600 mg, 80%).
LCMS m/z =366.2 [M+1].

Resolution of **compound 18B:**

**Compound 18B** was subjected to resolution to obtain **compound 18** (retention time: 4.170 min, 284 mg, ee% = 100%), with resolution conditions: instrument: MG II preparative SFC (SFC-14); column: ChiralPak AS, 250 × 30 mm I.D., 10 μm; mobile phase: A: $CO_2$, B: ethanol; gradient: B 25%; flow rate: 70 mL/min; back pressure: 100 bar; column temperature: 38°C; wavelength: 220 nm; cycle: 7 min; sample preparation: dissolving **compound 18B** in methanol/dichloromethane (40 mL); injection: 2.5 mL/injection.

**Compound 18:**

$^1$H NMR (400 MHz, $CDCl_3$) δ = 4.52 (t, J=7.2, 1H), 4.43-4.37 (m, 1H), 4.31 (m, J=17.4, 8.8, 2H), 4.05 (dd, J=15.9, 8.9, 1H), 3.86 (m, J=9.0, 5.6, 1H), 3.81 (m, J=9.0, 5.6, 1H), 3.05-2.95 (m, 1H), 2.73 (m, J=7.1, 3.6, 2H), 2.70 (s, 1H), 2.68 (s, 1H), 2.46 (m, J=14.2, 6.8, 2H), 2.39-2.27 (m, 1H), 1.89 (m, J=10.8, 9.2, 1H).
LCMS m/z =366.2 [M+1].

**Example 19:**

2-((1R,5S,6S)-3-(2-((R)-2-(difluoromethyl)azetidin-1-yl)-7,7-difluoro-6,7-dihydro-5H-cyclopenta[d]pyrimidin-4-yl)-3-azabicyclo[3.1.1]heptan-6-yl)acetic acid

2-((1R,5S,6R)-3-(2-((S)-2-(difluoromethyl)azetidin-1-yl)-7,7-difluoro-6,7-dihydro-5H-cyclopenta[d]pyrimidin-4-yl)-3-azabicyclo[3.1.1]heptan-6-yl)acetic acid (**isomers 1 and 2 of compound 19**)

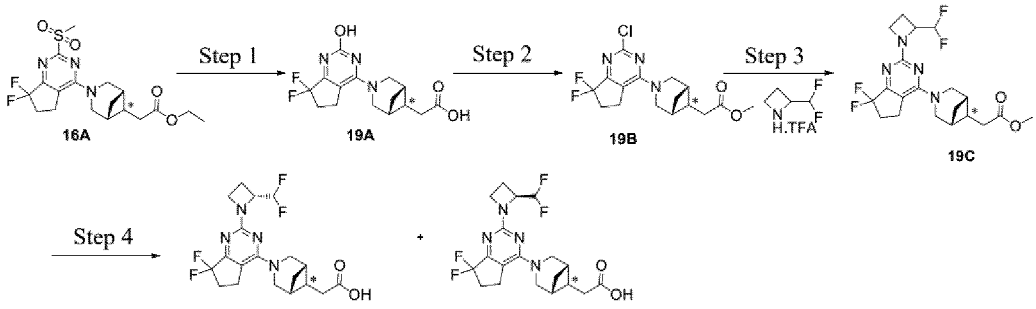

16A 19A 19B 19C

Isomer 1 and isomer 2 of compound 19

Step 1: 2-((1R,5S,6S)-3-(7,7-difluoro-2-hydroxy-6,7-dihydro-5H-cyclopenta[d]pyrimidin-4-yl)-3-azabicyclo[3.1.1]heptan-6-yl)acetic acid (**19A**)

**16A** (2.5 g, 6 mmol) was added to concentrated hydrochloric acid (30 mL). The mixture was warmed to 100°C and reacted for 2 h. The reaction mixture was cooled to room temperature and directly concentrated under reduced pressure. Toluene was then added, and the mixture was concentrated under reduced pressure again to remove water, so as to obtain the title compound **19A** as a crude (2.1 g).
LC-MS (ESI): m/z = 326.1 $[M+H]^+$.

Step 2: methyl 2-((1R,5S,6S)-3-(2-chloro-7,7-difluoro-6,7-dihydro-5H-cyclopenta[d]pyrimidin-4-yl)-3-azabicyclo[3.1.1]heptan-6-yl)acetate (**19B**)

Crude **19A** (2.1 g) and benzyltrimethylammonium chloride (2.4 g, 12.92 mmol) were added to phosphorus oxychloride (20 mL) and dichloroethane (10 mL), and the mixture was warmed to 95°C (external temperature) and reacted for 16 h. The reaction solution was cooled to room temperature and concentrated under reduced pressure to remove most of the solvent. The residue was added in portions to methanol (15 mL), and the mixture was stirred for 30 min and concentrated under reduced pressure to remove most of the methanol. Water (30 mL) was added, and the mixture was adjusted to pH=7-8 with sodium carbonate and extracted with dichloromethane. The organic phase was concentrated under reduced pressure, and then separated and purified by silica gel column chromatography (EA: PE (v/v) = 1: 3) to obtain the title compound **19B** (1.7 g).
LC-MS (ESI): m/z = 358.1 $[M+H]^+$.

Step 3: methyl 2-((1R,SS,6S)-3-(2-(2-(difluoromethyl)azetidin-1-yl)-7,7-difluoro-6,7-dihydro-5H-cyclopenta[d]pyrimidin-4-yl)-3-azabicyclo[3.1.1]heptan-6-yl)acetate (**19C**)

**19B** (0.4 g, 1.12 mol), 2-(difluoromethyl)azetidine trifluoroacetate (0.74 g, 3.36 mmol) and caesium carbonate (1.46 g, 4.48 mmol) were added to N-methylpyrrolidinone (8 mL), and the mixture was reacted under microwave at 150°C for 4 h and cooled to room temperature. Water (40 mL) was added, and the mixture was extracted with ethyl acetate. The organic phase was washed with a saturated sodium chloride solution, dried over anhydrous sodium sulphate and filtered. The filtrate was concentrated under reduced pressure, and then the residue was separated and purified by silica gel column chromatography (EA: PE (v/v) = 1: 3) to obtain the title compound **19C** (0.34 g, 71%).
LC-MS (ESI): m/z = 429.2 $[M+H]^+$.

Step 4: 2-((1R,SS,6S)-3-(2-((R)-2-(difluoromethyl)azetidin-1-yl)-7,7-difluoro-6,7-dihydro-5H-cyclopenta[d]pyrimidin-4-yl)-3-azabicyclo[3.1.1]heptan-6-yl)acetic acid 2-((1R,5S,6R)-3-(2-((S)-2-(difluoromethyl)azetidin-1-yl)-7,7-difluoro-6,7-dihydro-5H-cyclopenta[d]pyrimidin-4-yl)-3-azabicyclo[3.1.1]heptan-6-yl)acetic acid **(isomers 1 and 2 of compound 19)**

**19C** (0.3 g, 0.7 mmol) and lithium hydroxide monohydrate (0.15 g, 3.5 mmol) were added to methanol (10 mL) and water (2 mL), and the mixture was reacted at room temperature for 2 h and concentrated under reduced pressure to remove the organic solvent. Water (15 mL) was added, and the mixture was adjusted to pH= 5-6 with acetic acid (a large amount of white solid precipitated) and filtered. The solid was washed with water and dried to obtain **compound 19** (0.24 g, 83%).
LC-MS (ESI): m/z = 415.2 $[M+H]^+$.
Resolution of **compound 19: Compound 19** (240 mg) was subjected to resolution to obtain **isomer 1 of compound 19** (retention time: 1.074 min, 55 mg) and **isomer 2 of compound 19** (retention time: 1.181 min, 60 mg).
Resolution conditions: instrument: Waters SFC 150 Mgm; chromatographic column: DAICEL CHIRALCEL OJ

(250 mm × 30 mm, 10 μm); mobile phase: A: $CO_2$, B: MeOH; gradient: B 10%; flow rate: 130 mL/min; back pressure: 100 bar; column temperature: 35°C; wavelength: 220 nm; cycle: about 3.5 min; sample preparation: dissolving the compound at a concentration of 5.4 mg/mL in methanol; injection: 4 mL/injection.

**Isomer 1 of compound 19**

$^1$H NMR (400 MHz, DMSO-$d_6$) δ 6.31 (dd, 1H), 4.60-4.51 (m, 1H), 4.21-4.03 (m, 2H), 3.94-3.82 (m, 3H), 3.82-3.68 (m, 1H), 3.25-3.19 (m, 2H), 2.57 (d, 2H), 2.46-2.29 (m, 5H), 2.27-2.24(m, 2H), 1.98-1.93 (m, 1H), 1.32 (dd, 1H).
LCMS m/z =415.2 [M+1]$^+$.

**Isomer 2 of compound 19**

$^1$H NMR (400 MHz, DMSO-$d_6$) δ 6.31 (dd, 1H), 4.62-4.46 (m, 1H), 4.14-3.71 (m, 6H), 3.23-3.20 (m, 2H), 2.56 (d, 2H), 2.45-2.28 (m, 5H), 2.27-2.24(m, 2H), 1.98-1.93(m, 1H), 1.32 (dd, 1H).
LCMS m/z =415.2 [M+1]$^+$.

**Example 20:**

2-((1R,5S,6S)-3-(7,7-difluoro-2-((R)-2-(trifluoromethyl)azetidin-1-yl)-6,7-dihydro-5H-cyclopenta[d]pyrimidin-4-yl)-3-azabicyclo[3.1.1]heptan-6-yl)acetic acid

2-((1R,5S,6S)-3-(7,7-difluoro-2-((S)-2-(trifluoromethyl)azetidin-1-yl)-6,7-dihydro-SH-cyclopenta[d]pyrimidin-4-yl)-3-azabicyclo[3.1.1]heptan-6-yl)acetic acid (**isomers 1 and 2 of compound 20**)

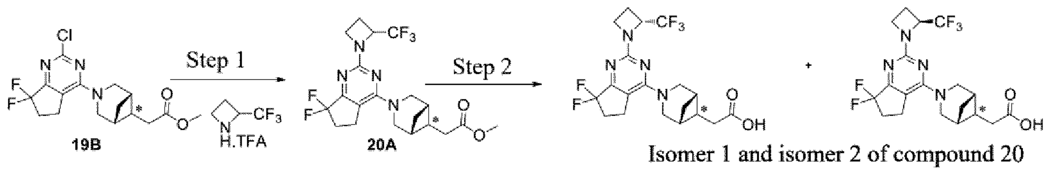


19B 20A

Isomer 1 and isomer 2 of compound 20

Step 1: methyl 2-((1R,5S,6S)-3-(7,7-difluoro-2-(2-(trifluoromethyl)azetidin-1-yl)-6,7-dihydro-5H-cyclopenta[d]pyrimidin-4-yl)-3-azabicyclo[3.1.1]heptan-6-yl)acetate (**20A**)

**19B** (0.6 g, 1.68 mol), 2-(trifluoromethyl)azetidine trifluoroacetate (0.63 g, 5.04 mmol) and sodium carbonate (0.71 g, 6.72 mmol) were added to dimethyl sulfoxide (8 mL), and the mixture was reacted under microwave at 150°C for 5 h and cooled to room temperature. Water (40 mL) was added, and the mixture was extracted with ethyl acetate. The organic phase was washed with a saturated sodium chloride solution, dried over anhydrous sodium sulphate and filtered. The filtrate was concentrated under reduced pressure, and then the residue was separated and purified by silica gel column chromatography (EA: PE (v/v) = 1: 3) to obtain the title compound **20A** (0.5 g, 66.7%).
LC-MS (ESI): m/z = 447.2 [M+H]$^+$.

Step 2: 2-((1R,5S,6S)-3-(7,7-difluoro-2-((R)-2-(trifluoromethyl)azetidin-1-yl)-6,7-dihydro-5H-cyclopenta[d]pyrimidin-4-yl)-3-azabicyclo[3.1.1]heptan-6-yl)acetic acid 2-((1R,5S,6S)-3-(7,7-difluoro-2-((S)-2-(trifluoromethyl)azetidin-1-yl)-6,7-dihydro-SH-cyclopenta[d]pyrimidin-4-yl)-3-azabicyclo[3.1.1]heptan-6-yl)acetic acid (**isomers 1 and 2 of compound 20**)

**20A** (0.42 g, 0.93 mmol) and lithium hydroxide monohydrate (0.2 g, 4.65 mmol) were added to methanol (10 mL) and water (2 mL), and the mixture was reacted at room temperature for 2 h and concentrated under reduced pressure to remove the organic solvent. Water (15 mL) was added, and the mixture was adjusted to pH= 5-6 with acetic acid (a large amount of white solid precipitated) and filtered. The solid was washed with water and dried to obtain **compound 20** (0.35 g, 87%).
LC-MS (ESI): m/z = 432.2 [M+H]$^+$.
Resolution of **compound 20:** Compound 20 (350 mg) was subjected to resolution to obtain **isomer 1 of compound 20** (retention time: 1.10 min, 180 mg) and **isomer 2 of compound 20** (retention time: 1.266 min, 112 mg).
Resolution conditions: instrument: Waters SFC 150 Mgm; chromatographic column: DAICEL CHIRALCEL OJ (250 mm × 30 mm, 10 μm); mobile phase: A: $CO_2$, B: EtOH (0.1% $NH_3 \cdot H_2O$); gradient: B 20%; flow rate: 120 mL/min;

back pressure: 100 bar; column temperature: 35°C; wavelength: 220 nm; cycle: about 4.8 min; sample preparation: dissolving the compound at a concentration of 12.5 mg/mL in ethanol; injection: 3 mL/injection.

**Isomer 1 of compound 20**

$^1$H NMR (400 MHz, $CDCl_3$) δ 4.78-4.73(m, 1H), 4.26-4.19 (m, 1H), 4.16-3.78 (m, 5H), 3.32-3.17 (m, 2H), 2.73 (d, 2H), 2.56-2.38 (m, 5H), 2.37-2.35 (m, 2H), 2.15-2.10(m, 1H), 1.44 (dd, 1H).
LC-MS (ESI): m/z = 432.2 $[M+H]^+$.

**Isomer 2 of compound 20**

$^1$H NMR (400 MHz, $CDCl_3$) δ 4.79-4.74(m, 1H), 4.30-4.20 (m, 1H), 4.19-3.76 (m, 5H), 3.21-3.18 (m, 2H), 2.73 (d, 2H), 2.55-2.38 (m, 5H), 2.38-2.35 (m, 2H), 2.15-2.10 (m, 1H), 1.44 (dd, 1H).
LC-MS (ESI): m/z = 432.2 $[M+H]^+$.

**Example 21:**

2-((1R,5S,6R)-3-(3-cyano-2-((S)-2-methylazetidin-1-yl)-6-(trifluoromethyl)pyridin-4-yl)-3-azabicyclo[3.1.0]hexan-6-yl)acetic acid (**compound 21**)

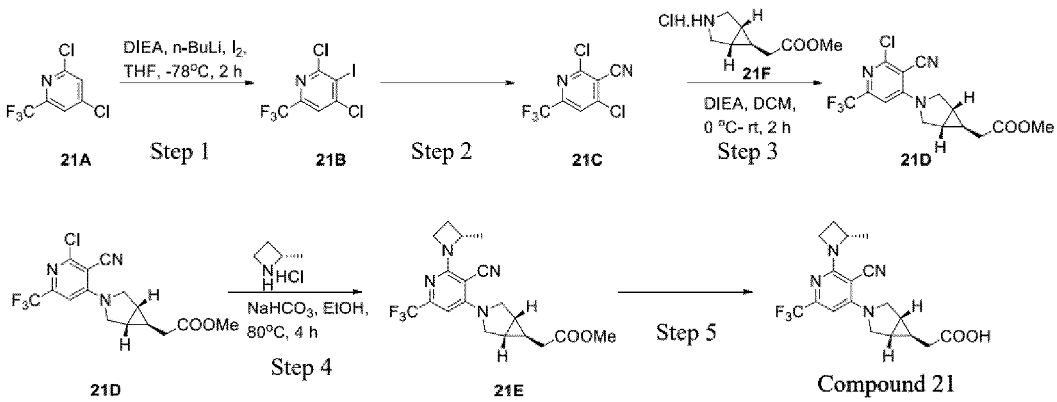


21A 21B 21C 21D 21D 21E Compound 21

Step 1: 2,4-dichloro-3-iodo-6-(trifluoromethyl)pyridine (**21B**)

In a 500 mL three-necked flask, compound **21A** (8.64 g, 40 mmol) and DIEA (5.68 g, 44 mmol) were added and dissolved in 150 mL of dry THF. n-Butyllithium (27.6 mL, 44 mmol, 1.6 M) was added slowly at -78°C under nitrogen protection, and the reaction mixture was then reacted at -78°C for another 45 min. Iodine (11.2 g, 44 mmol) was dissolved in 15 mL of dry THF and slowly added, and the reaction mixture was then reacted at -78°C for 1 h and returned to room temperature. EA was added, and the reaction was quenched with a saturated solution of ammonium chloride and $Na_2S_2O_3$. The organic layer was washed with a saturated sodium bicarbonate solution, dried over anhydrous sodium sulphate and concentrated under reduced pressure, and the residue was subjected to column chromatography (petroleum ether/ethyl acetate = 20/1) to obtain compound **21B** (12.1 g, yield: 88%).

Step 2: 2,4-dichloro-6-(trifluoromethyl)nicotinonitrile (**21C**)

Compound **21B** (8.02 g, 23.5 mmol) was dissolved in 80 mL of DMA. Cuprous cyanide (3.15 g, 35.2 mmol) was added, and the mixture was reacted at 130°C for 2 h. After the reaction was completed as monitored by TLC, 1 M aqueous ammonia and EA were added, and a precipitate was precipitated and filtered. The organic phase was then washed with water and saturated sodium chloride, dried over anhydrous sodium sulphate, subjected to rotary evaporation, and purified by silica gel column chromatography (petroleum ether/ethyl acetate = 10/1) to obtain compound **21C** (4.5 g, 80%).

Step 3: methyl 2-((1R,5S,6S)-3-(2-chloro-3-cyano-6-(trifluoromethyl)pyridin-4-yl)-3-azabicyclo[3.1.0]hexan-6-yl)acetate (**21D**)

Compound **21C** (1.2 g, 5.0 mmol) was dissolved in dichloromethane (30 mL), and **21F** (1.06 g, 5.5 mmol) and DIEA (1.94 g, 15.0 mmol) were added at 0°C. Then the mixture was warmed to room temperature and reacted for 2 h. After the reaction was completed as monitored by TLC, the solvent was removed by rotary evaporation, and the crude was separated by silica gel column chromatography (PE/EA = 5/1) to obtain compound **21D** (1.642 g, 91%).
LC-MS (ESI): m/z =360.2 $[M+H]^+$.

Step 4: methyl 2-((1R,5S,6R)-3-(3-cyano-2-((S)-2-methylazetidin-1-yl)-6-(trifluoromethyl)pyridin-4-yl)-3-azabicyclo[3.1.0]hexan-6-yl)acetate (**21E**)

Compound **21D** (718 mg, 2.0 mmol) was dissolved in 10 mL of ethanol. Compound (S)-2-methylazetidine hydrochloride (430 mg, 4.0 mmol) and sodium bicarbonate (672 mg, 8.0 mmol) were added, and the mixture was reacted at 80°C for 4 h, cooled to room temperature and subjected to rotary evaporation to remove ethanol. Water was added, and the mixture was extracted with EA. The organic phase was washed with saturated brine, dried over anhydrous sodium sulphate, filtered and concentrated, and the residue was separated by silica gel column chromatography (PE/EA =2/1) to obtain compound **21E** (712 mg, 90%).
LC-MS (ESI): m/z =395.2 $[M+H]^+$.

Step 5: 2-((1R,5S,6R)-3-(3-cyano-2-((S)-2-methylazetidin-1-yl)-6-(trifluoromethyl)pyridin-4-yl)-3-azabicyclo[3.1.0]hexan-6-yl)acetic acid (**compound 21**)

Compound **21E** (712 mg, 1.81 mmol) was dissolved in 11 mL of ethanol. 1 M sodium hydroxide (5.5 mL, 5.5 mmol) was added, and the mixture was stirred at room temperature and reacted for 1 h. After the reaction was completed as monitored by TLC, the mixture was concentrated, diluted with water and adjusted to about pH 5 with 1 N aqueous hydrochloric acid to precipitate a white solid, which was filtered and washed with water, and the filter cake was dried to obtain the target **compound 21** (625 mg, 91%).
LC-MS (ESI): m/z =381.2 $[M+H]^+$.
$^1H$ NMR (400 MHz, DMSO-$d_6$) δ 12.25 (br, 1H), 6.39 (s, 1H), 4.59-4.57 (m, 1H), 4.35-4.32 (m, 1H), 4.01-3.76 (m, 4H), 3.62-3.59 (m, 1H), 2.43-2.39 (m, 1H), 2.23 (d, *J* = 4 Hz, 2H), 1.93-1.89 (m, 1H), 1.64 (s, 2H), 1.37 (d, *J* = 8 Hz, 3H), 0.76-0.73 (m, 1H).

**Example 22:**

(S)-2-(1-(4-cyano-5,5-difluoro-3-(2-(trifluoromethyl)azetidin-1-yl)-6,7-dihydro-5H-cyclopenta[c]pyridin-1-yl)azetidin-3-yl)acetic acid (R)-2-(1-(4-cyano-5, 5-difluoro-3-(2-(trifluoromethyl)azetidin-1-yl)-6,7-dihydro-SH-cyclopenta[c]pyridin-1-yl)azetidin-3-yl)acetic acid (**isomers 1 and 2 of compound 22)**

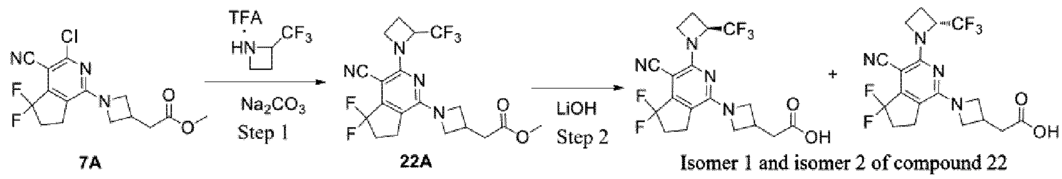

7A 22A Isomer 1 and isomer 2 of compound 22

methyl 2-(1-(4-cyano-5,5-difluoro-3-(2-(trifluoromethyl)azetidin-1-yl)-6,7-dihydro-SH-cyclopenta[c]pyridin-1-yl)azetidin-3-yl)acetate (**22A**)

Compound **7A** (400 mg, 1.17 mmol), 2-(trifluoromethyl)azetidine trifluoroacetate (746 mg, 3.52 mmol) and sodium carbonate (373 mg, 3.52 mmol) were added to a microwave tube, and dimethyl sulfoxide (5 mL) was added. The mixture was reacted in a microwave reactor (140°C, 3 h). After the reaction was completed, water (5 mL) and ethyl acetate (5 mL) were added. The organic phase was separated, and the aqueous phase was extracted with ethyl acetate (5 mL × 2). The organic phases were combined, dried over anhydrous sodium sulphate and concentrated, and the residue was purified by silica gel column chromatography (eluent ratio: PE: EA = 0%-15%) to obtain compound **22A** (300 mg, yield: 59.5%) as a light yellow solid.

LCMS m/z =342.1 [M+1].

Step 2: (S)-2-(1-(4-cyano-5,5-difluoro-3-(2-(trifluoromethyl)azetidin-1-yl)-6,7-dihydro-SH-cyclopenta[c]pyridin-1-yl)azetidin-3-yl)acetic acid (R)-2-(1-(4-cyano-5, 5-difluoro-3-(2-(trifluoromethyl)azetidin-1-yl)-6,7-dihydro-SH-cyclopenta[c]pyridin-1-yl)azetidin-3-yl)acetic acid (**isomers 1 and 2 of compound 22**)

Compound **22A** (300 mg, 0.70 mmol) was dissolved in tetrahydrofuran (7 mL). Water (3.5 mL) and lithium hydroxide (84 mg, 3.50 mmol) were added, and the mixture was stirred at room temperature and reacted for 2 h. After the reaction was completed, ethyl acetate (10 mL) was added. The organic phase was separated, and the remaining phase was extracted with ethyl acetate (5 mL × 2). The organic phases were combined, dried with anhydrous sodium sulphate and concentrated to obtain a crude, which was separated and purified by a liquid phase preparative column (liquid phase preparative conditions: chromatographic column: Chiralcel OJ-3 150 × 4.6 mm I.D., 3 μm; mobile phase: 30% of ethanol (0.05% DEA) in $CO_2$; elution time: 3 min; flow rate: 2.5 mL/min; column temperature: 35°C) to obtain **isomer 1** (78 mg, 26.8%, retention time: about 1.19 min, undetermined configuration) and **isomer 2** (67 mg, 23.0%, retention time: about 1.62 min, undetermined configuration) **of compound 22.**

**Isomer 1 of compound 22**

$^1H$ NMR (400 MHz, $CDCl_3$) δ 4.95 (dd, 1H), 4.60 (dd, 1H), 4.41 (dd, 2H), 4.19-4.09 (m, 1H), 3.93 (dd, 2H), 3.14-3.04 (m, 1H), 2.86 (d, 2H), 2.76 (d, 2H), 2.65-2.45 (m, 4H).
MS M/Z (ESI): m/z =417.2(M+1).

**Isomer 2 of compound 22**

$^1H$ NMR (400 MHz, $CDCl_3$) δ 5.01-4.88 (m, 1H), 4.60 (dd, 1H), 4.45-4.35 (m, 2H), 4.20-4.08 (m, 1H), 3.93 (td, 2H), 3.10 (dt, 1H), 2.91-2.81 (m, 2H), 2.76 (d, 2H), 2.65-2.44 (m, 4H).
MS M/Z (ESI): m/z =417.2 (M+1).

**Biological test:**

**Experiment of inhibitory activity against KHK in vitro**

With the involvement of ATP, fructokinase phosphorylates fructose to produce fructose-1-phosphate and ADP. In this experiment, the effect of the compound on the activity of fructokinase was determined by measuring the amount of ADP produced in the process. Test compound stocks were prepared in DMSO. Before use, the stocks were 3-fold serially diluted with assay buffers (25 mM HEPES, 300 mM KCl, 10 mM MgClz, 10 mM CaClz, pH 7.0) to 5-fold the final concentration tested, and the DMSO content was adjusted to 1%. In a 384-well plate, the test compound was added to achieve a final concentration of 7.6 nM to 100 μM (assay buffers containing 1% DMSO were added to background and control wells). 5 ng/μL purified hKHK-C (assay buffers were added to background wells), 50 mM fructose and 0.2 mM ATP were added to reach a total volume of 10 μL and reacted at room temperature for 30 min. After the reaction was completed, 10 μL of ADP-glo (promega) was added and mixed uniformly, and the plate was incubated at room temperature for 40 min. Then the ADP-glo was added again, mixed uniformly and reacted for 40 min. The spontaneous luminescence intensity was measured using an Envision microplate reader. The inhibition rate of the compound against fructose activity was calculated using the following formula,

$$Inhibition\ rate = 100\% \times \frac{(RLU_{ZPE} - RLU_{blank}) - (RLU_{cpd} - RLU_{blank})}{RLU_{ZPE} - RLU_{blank}}$$

wherein

$RLU_{ZPE}$ = luminescence intensity of control well
$RLU_{blank}$ = luminescence intensity of background well
$RLU_{cpd}$ = luminescence intensity of test compound well

A curve of inhibition rate (y-axis) versus compound concentration (x-axis) was fitted by logarithmic (inhibitor) and normalized response (variable slope) in GraphPad Prism, and $EC_{50}$ was calculated. The results were as shown in Table 1.

Table 1 Inhibitory activity against KHK

| Compound No. | hKHKc (nM) | hKHKa (nM) | mKHKc (nM) | rKHKc (nM) |
|---|---|---|---|---|
| Isomer 2 of compound 3 | 37.41 | 51.84 | 134.2 | 145.9 |
| Isomer 2 of compound 4 | 19.38 | - | - | - |
| Compound 7 | 36.5 | 35.16 | - | - |
| Isomer 1 of compound 8 | 58.58 | 59.67 | - | - |
| Compound 16 | 37.7 | - | - | - |
| Compound 18 | 52.67 | - | - | - |
| Isomer 2 of compound 19 | 60.57 | - | - | - |
| Isomer 2 of compound 20 | 49.77 | - | - | - |
| Control compound | 123.3 | | | |
| -: not detected. | | | | |

The control compound was the compound of Example **4** in patent WO 2017115205A1

Conclusion: The compound of the present invention has a higher inhibitory effect on KHK.

2. **Pharmacokinetic experiment in rats**

**2.1 Experimental animals:** Male SD rats, about 220 g, 6-8 weeks old, 6 rats/compound, purchased from Hunan SJA Laboratory Animal Co., Ltd.

**2.2 Experimental design:** On the day of the experiment, 6 SD rats were randomly grouped according to their body weight. The animals were fasted with water available for 12 to 14 h one day before the administration, and were fed 4 h after the administration.

Table 2 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Isomer 2 of compound 20 | 1 | 0.2 | 5 | Plasma | Intravenously |
| G2 | 3 | Isomer 2 of compound 20 | 5 | 0.5 | 10 | Plasma | Intragastrically |
| Vehicle for intravenous administration: 5% DMA+5% Solutol+90% Saline; Vehicle for intragastric administration: 0.5% MC | | | | | | | |

Before and after the administration, 0.15 mL of blood was taken from the orbit of the animals under isoflurane anaesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h, and 24 h; and blood sampling time points for the intragastric administration group: 0, 5 min, 15 min, 30 min, 1 h, 2 h, 4 h, 6 h, 8 h, and 24 h. Before analysis and detection, all samples were stored at -80°C.

Table 3 Pharmacokinetic parameters of test compounds in plasma of rats

| Test compound | Mode of administration | CL (mL/min/kg) | $Vd_{ss}$ (L/kg) | $AUC_{0-t}$ (hr*ng/mL) | F (%) |
|---|---|---|---|---|---|
| Isomer 2 of | i.v. (1 mg/kg) | 1.35 | 0.568 | 11592 | - |
| compound 20 | | | | | |
| Isomer 2 of compound 20 | i.g. (5 mg/kg) | - | - | 64058 | 111 |
| -: not applicable. | | | | | |

Conclusion: isomer 2 of compound 20 has excellent pharmacokinetic parameters in plasma of rats.

**3. Pharmacokinetic experiment in mice**

**3.1 Experimental animals:** male ICR mice, about 22 g, 6-8 weeks old, 18 mice/compound, purchased from Hunan SJA Laboratory Animal Co., Ltd.

**3.2 Experimental design:** On the day of the experiment, 18 ICR mice were randomly grouped according to their body weight. The animals were fasted with water available for 12 to 14 h one day before the administration, and were fed 4 h after the administration.

Table 4 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 9 | Isomer 2 of compound 20 | 1 | 0.2 | 5 | Plasma | Intravenously |
| G2 | 9 | Isomer 2 of compound 20 | 3 | 0.3 | 10 | Plasma | Intragastrically |
| Vehicle for intravenous administration: 5% DMA+5% Solutol+90% Saline; Vehicle for intragastric administration: 0.5% MC | | | | | | | |

Before and after the administration, 0.08 mL of blood was taken from the orbit of the animals under isoflurane anaesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h, and 24 h; and blood sampling time points for the intragastric administration group: 0, 5 min, 15 min, 30 min, 1 h, 2 h, 4 h, 6 h, 8 h, and 24 h. Before analysis and detection, all samples were stored at -80°C.

Table 5 Pharmacokinetic parameters of test compounds in plasma of mice

| Test compound | Mode of administration | CL (mL/min/kg) | $Vd_{ss}$ (L/kg) | $AUC_{0-t}$ (hr*ng/mL) | F (%) |
|---|---|---|---|---|---|
| Isomer 2 of | i.v. (1 mg/kg) | 8.84 | 0.977 | 1838 | - |
| compound 20 | | | | | |
| Isomer 2 of compound 20 | i.g. (3 mg/kg) | - | - | 4807 | 87.2 |
| -: not applicable. | | | | | |

Conclusion: isomer 2 of compound 20 has excellent pharmacokinetic parameters in plasma of mice.

**4. CYP450 enzyme inhibition test**

In this test, after the mixed human liver microsomes were incubated with isomer 2 of compound 20 at different concentrations (0.05-50 μM) and corresponding probe drugs, the changes of CYP enzyme activity were measured, and the $IC_{50}$ value was calculated to evaluate the inhibition potential of isomer 2 of compound 20 on each CYP enzyme.

The test results showed that the $IC_{50}$ values of isomer 2 of compound 20 on CYP1A2, CYP2C9, CYP2C19, CYP2D6 and CYP3A4 enzymes were all more than 50 μM, indicating that there was basically no inhibitory effect.

**5. Test for hERG potassium ion channel**

**Test platform:** electrophysiological manual patch-clamp system

**Cell line:** Chinese hamster ovary (CHO) cell lines stably expressing hERG potassium ion channel

**Test method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition \%} = [1-(I / Io)] \times 100\%$$

Among the formula, Inhibition % represents the percentage of inhibition of hERG potassium current by the compound, and I and Io represent the amplitude of hERG potassium current after and before dosing, respectively.

Compound $IC_{50}$ was calculated using GraphPad Prism 5 software by fitting according to the following equation:

$$Y = \text{Bottom} + (\text{Top-Bottom})/(1+10\char`^((\text{LogIC50-X})*\text{HillSlope}))$$

Among the equation, X represents the Log value of the tested concentration of the test sample, Y represents the inhibition percentage at the corresponding concentration, and Bottom and Top represent the minimum and maximum inhibition percentage, respectively.

**Test results:** the $IC_{50}$ value of isomer 2 of compound 20 for inhibiting the hERG potassium channel current shows that the isomer basically has no inhibitory effect on the hERG potassium channel current.

Table 6

| Compound | Inhibition rate at the highest concentration tested | $IC_{50}$ ($\mu$M) |
|---|---|---|
| Isomer 2 of compound 20 | 20.4 $\pm$ 3.58%@40 $\mu$M | > 40 |

## Claims

**1.** A compound of formula (I), or a stereoisomer, a deuterated compound, a solvate, a prodrug, a metabolite, a pharmaceutically acceptable salt or a eutectic thereof,

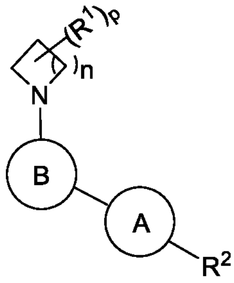
(I)

wherein each $R^1$ is independently selected from deuterium, $C_{1-6}$ alkyl, $C_{2-6}$ alkenyl, $C_{2-6}$ alkynyl, hydroxyl, halogen, amino, nitro, cyano, carboxyl, $C_{1-6}$ alkoxy, $C_{1-6}$ alkylamino, or di($C_{1-6}$ alkyl)amino, wherein the alkyl and alkoxy are optionally substituted with 1 to 5 groups selected from halogen, deuterium, hydroxyl, amino, cyano, or $C_{1-6}$ alkoxy;
p is an integer selected from 1-8;
n is selected from 1, 2, or 3;
ring B is selected from B1, B2, B3, B4, B5, B6, B7, B8 or B9 group, wherein # represents a connection site of ring B to ring A;

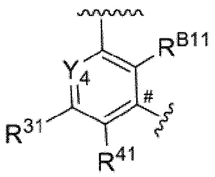
B1
,
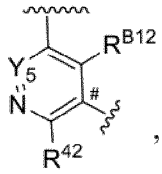
B2
,
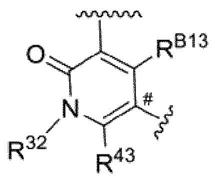
B3
,

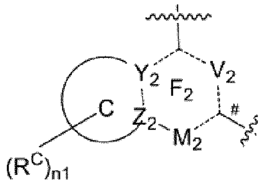
B4
,
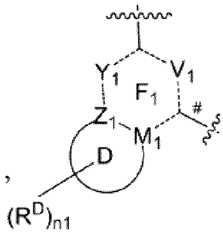
B5
,
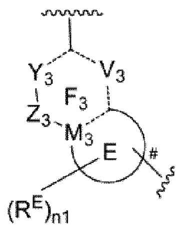
B6
,
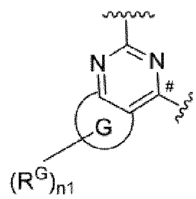
B7

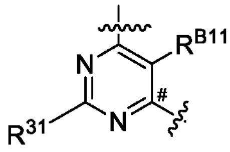
B8

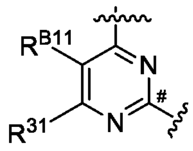
B9

;

----- represents a double bond or a single bond;
rings $F_1$, $F_2$, and $F_3$ are aryl or heteroaryl;
ring C is selected from 3-12-membered cycloalkyl, 3-14-membered heterocycloalkyl, 6-12-membered aryl, or 5-12-membered heteroaryl;
ring D is selected from 3-12-membered cycloalkyl, 3-14-membered heterocycloalkyl, 6-12-membered aryl, or 5-12-membered heteroaryl;
ring E is selected from 3-12-membered cycloalkyl, 3-14-membered heterocycloalkyl, 6-12-membered aryl, or 5-12-membered heteroaryl;
ring G is selected from 6-12-membered aryl, 5-12-membered heteroaryl, 3-14-membered heterocycloalkyl, or 3-12-membered cycloalkyl;
$Y_4$ and $Y_5$ are each independently selected from $-CR^{54}-$ or -N-;
$V_1$ and $Y_1$ are each independently selected from $-CR^{51}-$ or -N-;
$Z_1$ and $M_1$ are each independently selected from -C-, $-CR^{51}-$ or -N-;
$V_2$ and $M_2$ are each independently selected from $-CR^{52}-$ or -N-;
$Y_2$ and $Z_2$ are each independently selected from -C-, $-CR^{52}-$ or -N-;
$V_3$, $Y_3$, and $Z_3$ are each independently selected from $-CR^{53}-$ or -N-;
each $M_3$ is independently selected from -C-, $-CR^{53}-$, or -N-;
provided that when $Z_1$ and $M_1$ are both selected from -C-, $V_1$ and $Y_1$ are not simultaneously -N-;
n1 is selected from 0, 1, 2, or 3;
$R^C$, $R^D$, $R^E$, $R^G$, and $R^{B11}$ are each independently selected from deuterium, halogen, nitro, cyano, amino, hydroxyl, =O, $-SF_5$, di($C_{1-6}$ alkyl)phosphonyl, $C_{1-6}$ alkyl, $C_{1-6}$ alkoxy, $C_{2-6}$ alkenyl, $C_{2-6}$ alkynyl, $-S-C_{1-6}$ alkyl, $-S(O)-C_{1-6}$ alkyl, $-S(O)_2-C_{1-6}$ alkyl, $-(CH_2)_r-C_{3-12}$ cycloalkyl, $-(CH_2)_r-C_{3-12}$ heterocycloalkyl, $-O-C_{3-12}$ cycloalkyl, $-O-C_{3-12}$ heterocycloalkyl, $-NH-C_{3-12}$ cycloalkyl, $-NH-C_{3-12}$ heterocycloalkyl, $-S-C_{3-12}$ cycloalkyl, $-S-C_{3-12}$ heterocycloalkyl, 5- to 12-membered heteroaryl, 6- to 12-membered aryl, $-NHC_{1-6}$ alkyl, $-N(C_{1-6}$ alkyl$)_2$, $-C(=O)NR^{31a}R^{41a}$, $-NR^{31a}C(=O)-R^{41a}$, $-NR^{31a}R^{41a}$, or $-C(=O)-R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-6}$ alkyl, $C_{1-6}$ alkoxy, halo $C_{1-6}$ alkyl, halo $C_{1-6}$ alkoxy, deuterated $C_{1-6}$ alkyl, or deuterated $C_{1-6}$ alkoxy;
$R^{B12}$, $R^{B13}$, $R^{31}$, $R^{32}$, $R^{41}$, $R^{42}$, $R^{43}$, $R^{51}$, $R^{52}$, $R^{53}$, and $R^{54}$ are each independently selected from hydrogen, deuterium, halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $-S-C_{1-6}$ alkyl, $-S(O)-C_{1-6}$ alkyl, $-S(O)_2-C_{1-6}$ alkyl, $-(CH_2)_r-C_{3-12}$ cycloalkyl, $-(CH_2)_r-C_{3-12}$ heterocycloalkyl, $-O-C_{3-12}$ cycloalkyl, $-O-C_{3-12}$ heterocycloalkyl, $-NH-C_{3-12}$ cycloalkyl, $-NH-C_{3-12}$ heterocycloalkyl, $-S-C_{3-12}$ cycloalkyl, $-S-C_{3-12}$ heterocycloalkyl, 5- to 12-membered heteroaryl, 6- to 12-membered aryl, $-NHC_{1-6}$ alkyl, $-N(C_{1-6}$ alkyl$)_2$, $-C(=O)NR^{31a}R^{41a}$, $-NR^{31a}C(=O)-R^{41a}$, $-NR^{31a}R^{41a}$, or $-C(=O)-R^{31a}$, wherein the $CH_2$, alkyl, alkoxy, cycloalkyl, heterocycloalkyl, heteroaryl, and aryl are optionally further substituted with 1-5 groups selected from halogen, deuterium, nitro, cyano, amino, hydroxyl, =O, $C_{1-6}$ alkyl, $C_{1-6}$ alkoxy, halo $C_{1-6}$ alkyl, halo $C_{1-6}$ alkoxy, deuterated $C_{1-6}$ alkyl, or deuterated $C_{1-6}$ alkoxy;
each r is independently selected from 0, 1, 2, 3, or 4;
$R^{31a}$ and $R^{41a}$ are each independently substituted with a group selected from hydrogen, halogen, deuterium, nitro, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, or deuterated $C_{1-6}$ alkoxy;
ring A is selected from the following groups, wherein * represents a connection site of ring A to $R^2$:

(1) 4-7-membered monocyclic heterocycloalkyl and 4-7-membered monocyclic cycloalkyl;
(2) a 5-12-membered spiro ring;
(3)

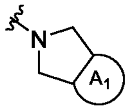, 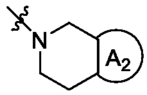, or 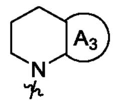,

wherein the connection site of ring A to $R_2$ is $A_1$, $A_2$, or $A_3$ ring atom;

(4)

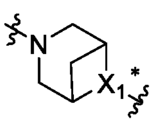, 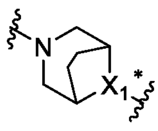, 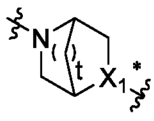, or 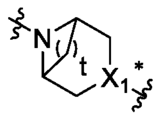;

(5)

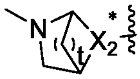 or 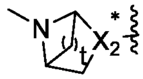;

(6)

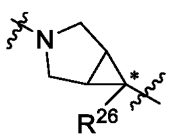 or 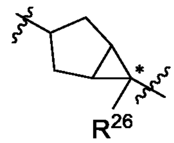;

(7) 7-12-membered aryl;

(8) 5-12-membered heteroaryl;

the ring A is optionally further substituted with 1 to 5 $R^A$;

each $R^A$ is independently selected from deuterium, halogen, cyano, hydroxyl, amino, =O, $C_{1-6}$ alkyl, $C_{1-6}$ alkoxy, halo $C_{1-6}$ alkyl, halo $C_{1-6}$ alkoxy, deuterated $C_{1-6}$ alkyl, or deuterated $C_{1-6}$ alkoxy, or two $R^A$ on the same atom together form 3-5-membered monocyclic cycloalkyl;

each t is independently selected from 1, 2, or 3;

ring $A_1$ is selected from 4-6-membered monocyclic cycloalkyl, 4-6-membered monocyclic heterocycloalkyl, 5-6-membered heteroaryl, or phenyl;

rings $A_2$ and $A_3$ are each independently selected from 3-6-membered monocyclic cycloalkyl, 5-6-membered heteroaryl, or phenyl;

$X_1$ and $X_2$ are each independently selected from -CH-, -$CR^x$-, or -N-;

$R^x$ is selected from deuterium, F, Cl, $C_{1-6}$ alkyl, halo $C_{1-6}$ alkyl, or deuterated $C_{1-6}$ alkyl;

$R^{26}$ is selected from hydrogen, deuterium, F, Cl, or $C_{1-6}$ alkyl, wherein the alkyl is optionally further substituted with 1 to 5 groups selected from deuterium, halogen, cyano, hydroxyl, amino, or $C_{1-6}$ alkoxy;

$R^2$ is selected from $-(CR^{2a}R^{2b})_m-C(O)NR^{21}R^{22}$, $-(CR^{2a}R^{2b})_m-COOR^{23}$, - $(CR^{2a}R^{2b})_m-S(O)R^{24}$, $-(CR^{2a}R^{2b})_m-S(O)_2R^{24}$, $-(CR^{2a}R^{2b})_m-C(O)R^{25}$, $-(CR^{2a}R^{2b})_m-P(O)_2R^{24}$, or $-(CR^{2a}R^{2b})_m$-tetrazol-5-yl;

$R^{2a}$ and $R^{2b}$ are each independently selected from hydrogen, deuterium, F, Cl, $C_{1-6}$ alkyl, or halo $C_{1-6}$ alkyl, or $R^{2a}$ and $R^{2b}$ together with the carbon atom to which they are attached form 3-4-membered cycloalkyl or 4-membered heterocycloalkyl;

$R^{21}$ and $R^{22}$ are each independently selected from hydrogen, deuterium, or $C_{1-6}$ alkyl, wherein the alkyl is optionally further substituted with deuterium;

$R^{23}$ and $R^{25}$ are each selected from hydrogen, deuterium, $C_{1-6}$ alkyl, or halo $C_{1-6}$ alkyl, wherein the alkyl is optionally further substituted with deuterium;

each $R^{24}$ is independently selected from hydrogen, deuterium, hydroxyl, $C_{1-6}$ alkyl, or -$NHC_{1-6}$ alkyl, wherein the alkyl is optionally further substituted with deuterium;

each m is independently selected from 0, 1, 2, 3, or 4;

provided that:

(1) when B is selected from B7 structure, and A is selected from

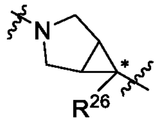

,

$R^2$ is not selected from $-CH_2-COOR^{23}$;

(2) when n is selected from 2, and B is selected from B1, ring A is not selected from

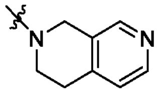

;

(3) when n is selected from 2 and 3, and B is selected from B4, ring A is not selected from piperazinyl; and

(4) when B is selected from B8 or B9 structure, n is only selected from 1, and $R^1$ is not selected from hydroxyl.

**2.** The compound of formula (I), or the stereoisomer, deuterated compound, solvate, prodrug, metabolite, pharmaceutically acceptable salt or eutectic thereof according to claim 1, wherein the compound of formula (I) has a structure of formula (I-a) or (I-b):

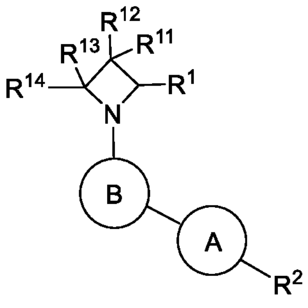

(I-a)

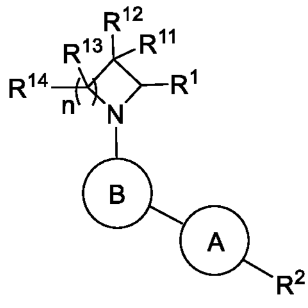

(I-b)

each $R^1$ is independently selected from $C_{1-3}$ alkyl, $C_{2-4}$ alkenyl, or $C_{2-4}$ alkynyl, wherein the alkyl is optionally substituted with 1 to 3 groups selected from halogen, deuterium, hydroxyl, or amino; and

$R^{11}$, $R^{12}$, $R^{13}$, and $R^{14}$ are each independently selected from H, deuterium, $C_{1-3}$ alkyl, F, or Cl, wherein the alkyl is optionally substituted with 1 to 3 groups selected from F, Cl, deuterium, hydroxyl, or amino.

**3.** The compound, or the stereoisomer, deuterated compound, solvate, prodrug, metabolite, pharmaceutically acceptable salt or eutectic thereof according to either claim 1 or claim 2, wherein

ring A is selected from the following groups, wherein * represents a connection site of ring A to $R^2$:

(1) 4-membered monocyclic heterocycloalkyl containing 1 to 3 N heteroatoms and containing 0 to 1 heteroatom selected from O and S, 5-membered monocyclic heterocycloalkyl containing 1 to 3 N heteroatoms and containing 0 to 1 heteroatom selected from O and S, 7-membered monocyclic heterocycloalkyl containing 1 to 3 N heteroatoms and containing 0 to 1 heteroatom selected from O and S, 4-membered monocyclic cycloalkyl, 5-membered monocyclic cycloalkyl, or 6-membered monocyclic cycloalkyl, wherein the N atom is a connection site of ring A to ring B;

(2) azetidinyl spiro 3-membered cycloalkyl, azetidinyl spiro 4-membered cycloalkyl, azetidinyl spiro 5-membered cycloalkyl, azetidinyl spiro 6-membered cycloalkyl, azacyclopentyl spiro 3-membered cycloalkyl, azacyclopentyl spiro 4-membered cycloalkyl, azacyclopentyl spiro 5-membered cycloalkyl, azacyclopentyl spiro 6-membered cycloalkyl, azacyclohexyl spiro 3-membered cycloalkyl, azacyclohexyl spiro 4-membered cycloalkyl, azacyclohexyl spiro 5-membered cycloalkyl, azacyclohexyl spiro 6-membered cycloalkyl, azetidinyl spiro 3-membered heterocycloalkyl, azetidinyl spiro 4-membered heterocycloalkyl, azetidinyl spiro 5-membered heterocycloalkyl, azetidinyl spiro 6-membered heterocycloalkyl, azacyclopentyl spiro 3-membered heterocycloalkyl, azacyclopentyl spiro 4-membered heterocycloalkyl, azacyclopentyl spiro 5-membered heterocycloalkyl, azacyclopentyl spiro 6-membered heterocycloalkyl, azacyclohexyl spiro 3-membered heterocycloalkyl, azacyclohexyl spiro 4-membered heterocycloalkyl, azacyclohexyl spiro 5-membered heterocycloalkyl, or azacyclohexyl spiro 6-membered heterocycloalkyl, wherein the heterocycloalkyl is a saturated monocyclic heterocycloalkyl containing 1 to 2 heteroatoms selected from N, O, and S, the connection site of ring A to $R^2$ is cycloalkyl or heterocycloalkyl, and ring B and $R^2$ are connected in different rings of ring A;

(3)

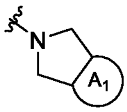, 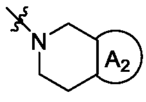, or 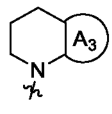,

wherein the connection site of ring A to $R_2$ is $A_1$, $A_2$, or $A_3$ ring atom;

(4)

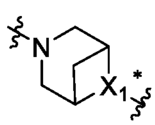, 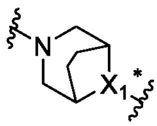, 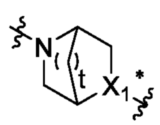, 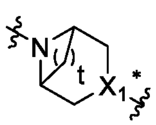, or 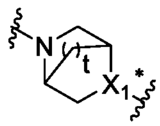;

(5)

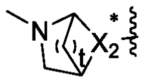 or 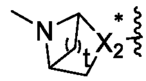;

(6)

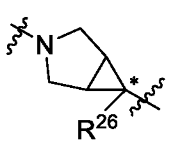 or 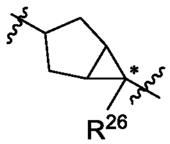;

the ring A is optionally further substituted with 1 to 3 $R^A$;

each $R^A$ is independently selected from deuterium, halogen, cyano, hydroxyl, amino, =O, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy, or two $R^A$ on the same atom together form 3-4-membered monocyclic cycloalkyl;
$R^{26}$ is selected from hydrogen, deuterium, F, Cl, or $C_{1-4}$ alkyl, wherein the alkyl is optionally further substituted with 1 to 3 groups selected from deuterium, halogen, cyano, hydroxyl, amino, or $C_{1-3}$ alkoxy;
each t is independently selected from 1 or 2;
ring $A_1$ is selected from 4-membered monocyclic cycloalkyl, 5-membered monocyclic cycloalkyl, 6-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, or phenyl;
ring $A_2$ is selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, or phenyl;
each ring $A_3$ is independently selected from 3-membered monocyclic cycloalkyl, 4-membered monocyclic heterocycloalkyl, 5-membered monocyclic heterocycloalkyl, 6-membered monocyclic heterocycloalkyl, 5-membered heteroaryl, 6-membered heteroaryl, or phenyl;
$X_1$ and $X_2$ are each independently selected from -CH-, -$CR^x$-, or -N-;
and $R^x$ is selected from deuterium, F, Cl, $C_{1-3}$ alkyl, halo $C_{1-3}$ alkyl, or deuterated $C_{1-3}$ alkyl.

**4.** The compound, or the stereoisomer, deuterated compound, solvate, prodrug, metabolite, pharmaceutically acceptable salt or eutectic thereof according to either claim 1 or claim 2, wherein

ring A is selected from cyclobutyl, cyclopentyl, cyclohexyl,

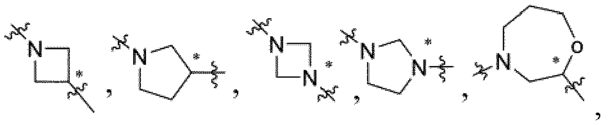

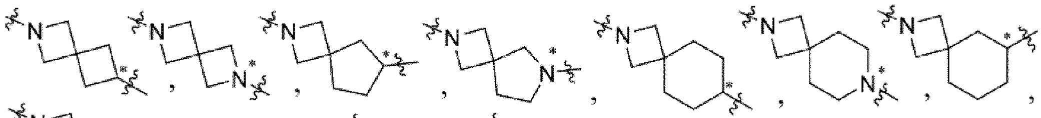

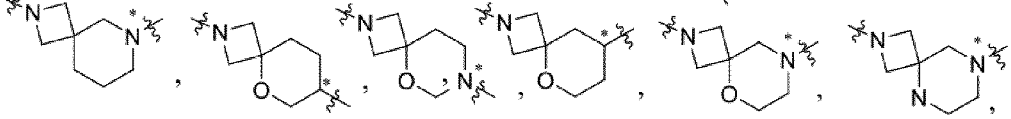

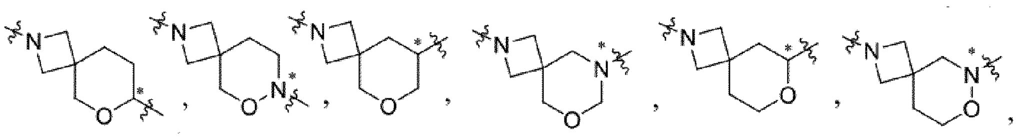

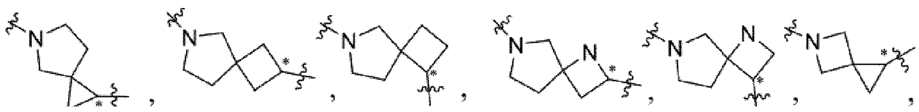

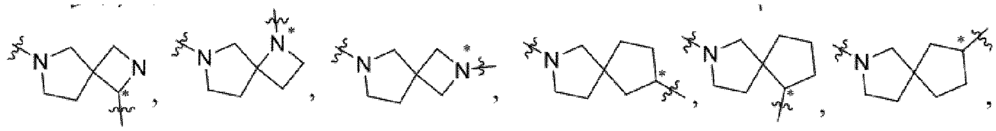

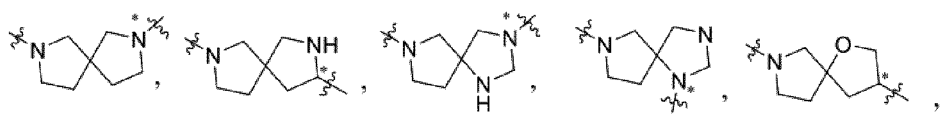

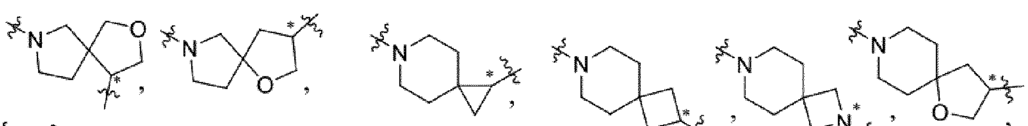

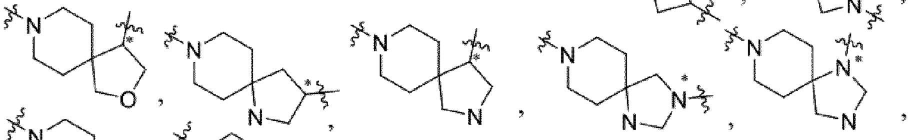

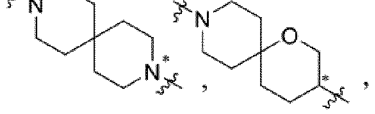

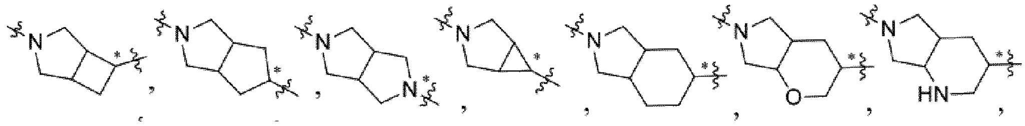

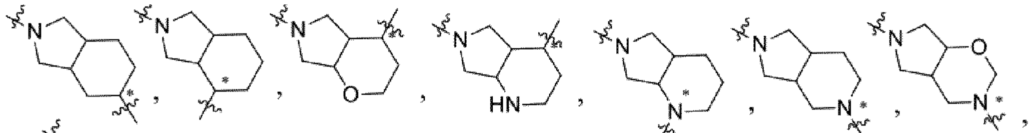

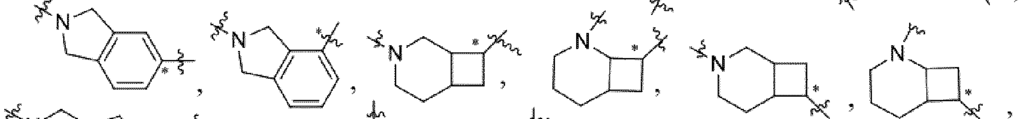

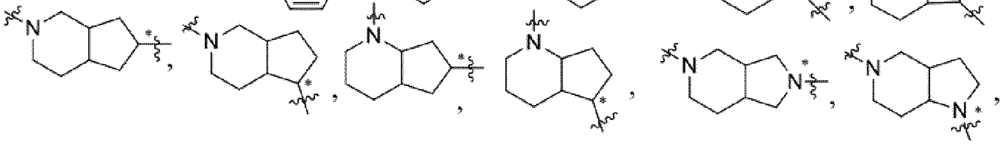

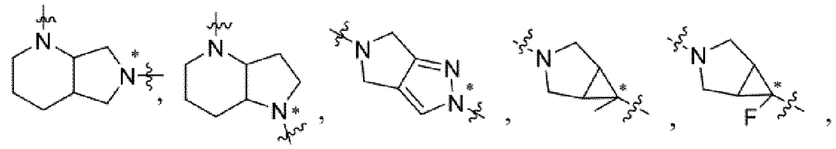

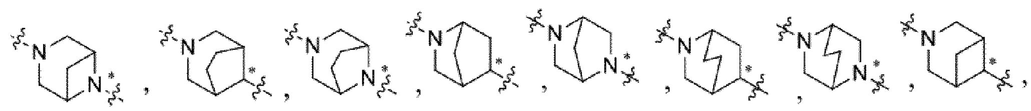

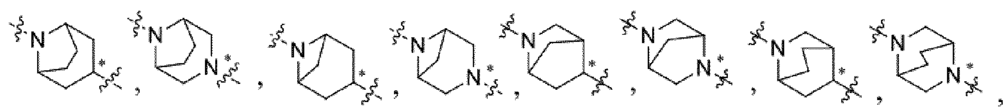

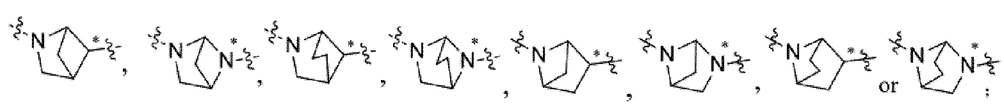

wherein * represents a connection site of ring A to $R^2$.

**5.** The compound of formula (I), or the stereoisomer, deuterated compound, solvate, prodrug, metabolite, pharmaceutically acceptable salt or eutectic thereof according to claim 1, wherein

ring A is selected from 5-membered heteroaryl containing 1 to 2 heteroatoms selected from N, O, and S, 8-membered heteroaryl containing 1 to 2 heteroatoms selected from N, O, and S, 9-membered heteroaryl containing 1 to 2 heteroatoms selected from N, O, and S, or 10-membered heteroaryl containing 1 to 2 heteroatoms selected from N, O, and S; or
ring A is selected from

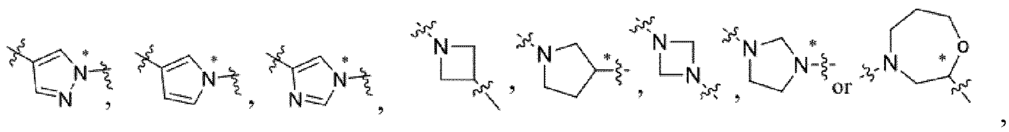

wherein * represents a connection site of ring A to $R^2$.

**6.** The compound, or the stereoisomer, deuterated compound, solvate, prodrug, metabolite, pharmaceutically acceptable salt or eutectic thereof according to either claim 1 or claim 2, wherein

each $R^1$ is independently selected from $C_{1-3}$ alkyl, $C_{2-4}$ alkenyl, or $C_{2-4}$ alkynyl, wherein the alkyl is optionally substituted with 1 to 3 groups selected from F, Cl, deuterium, or hydroxyl; or

each $R^1$ is independently selected from $C_{1-3}$ alkyl or $C_{2-4}$ alkynyl, wherein the alkyl is optionally substituted with 1 to 3 groups selected from F, Cl, deuterium, or hydroxyl; or
each $R^1$ is independently selected from methyl or ethynyl, wherein the methyl is optionally substituted with 1 to 3 groups selected from halogen, deuterium, or hydroxyl.

**7.** The compound, or the stereoisomer, deuterated compound, solvate, prodrug, metabolite, pharmaceutically acceptable salt or eutectic thereof according to either claim 2, wherein

$R^{11}$, $R^{12}$, $R^{13}$, and $R^{14}$ are each independently selected from H, deuterium, $C_{1-3}$ alkyl, F, or Cl, wherein the alkyl is optionally substituted with 1 to 3 groups selected from F, Cl, deuterium, hydroxyl, or amino; or
$R^{11}$, $R^{12}$, $R^{13}$, and $R^{14}$ are each independently selected from H, deuterium, methyl, ethyl, F, or Cl.

**8.** The compound, or the stereoisomer, deuterated compound, solvate, prodrug, metabolite, pharmaceutically acceptable salt or eutectic thereof according to either claim 1 or claim 2, wherein

$R^2$ is selected from $-(CR^{2a}R^{2b})_m-C(O)NR^{21}R^{22}$ or $-(CR^{2a}R^{2b})_m-COOR^{23}$;
$R^{2a}$ and $R^{2b}$ are each independently selected from hydrogen, deuterium, F, Cl, methyl, or ethyl, or $R^{2a}$ and $R^{2b}$ together with the carbon atom to which they are attached form cyclopropyl or cyclobutyl;
$R^{21}$ and $R^{22}$ are each independently selected from hydrogen, deuterium, methyl, ethyl, propyl, or tert-butyl, wherein the methyl, ethyl, propyl, or tert-butyl is optionally further substituted with deuterium;
$R^{23}$ is selected from hydrogen, deuterium, methyl, ethyl, propyl, or tert-butyl, and the $R^{23}$ is optionally further substituted with deuterium;
and m is selected from 0 or 1.

**9.** The compound, or the stereoisomer, deuterated compound, solvate, prodrug, metabolite, pharmaceutically acceptable salt or eutectic thereof according to either claim 1 or claim 2, wherein

$R^C$, $R^D$, $R^E$, $R^G$, and $R^{B11}$ are each independently selected from deuterium, F, Cl, nitro, cyano, amino, hydroxyl, =O, $-SF_5$, di($C_{1-3}$ alkyl)phosphonyl, $C_{1-4}$ alkyl, $C_{1-4}$ alkoxy, $-(CH_2)_r-C_{3-6}$ cycloalkyl, or $-(CH_2)_r-C_{3-6}$ heterocycloalkyl, wherein the $CH_2$, alkyl, alkoxy, cycloalkyl, and heterocycloalkyl are optionally further substituted with 1-3 groups selected from F, Cl, deuterium, cyano, amino, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy;
$R^{B12}$, $R^{B13}$, $R^{31}$, $R^{32}$, $R^{41}$, $R^{42}$ $R^{43}$ $R^{51}$, $R^{52}$ and $R^{53}$ are each independently selected from hydrogen, deuterium, F, Cl, cyano, $-SF_5$, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, or phenyl, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; or
$R^C$, $R^D$, $R^E$, $R^G$, and $R^{B11}$ are each independently selected from deuterium, F, Cl, cyano, hydroxyl, =O, $-SF_5$, di(methyl)phosphonyl, methyl, ethyl, propyl, isopropyl, tert-butyl, 2-methylpropyl, methoxy, ethoxy, propoxy, tert-butoxy, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, $-CH_2$-azacyclohexyl, oxetanyl, oxacyclopentyl, oxacyclohexyl, $-CH_2$-oxetanyl, $-CH_2$-oxacyclopentyl, - $CH_2$-oxacyclohexyl, thietanyl, thiolanyl, thianyl, $-CH_2$-thietanyl, $-CH_2$-thiolanyl, or $-CH_2$-thianyl, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, $-CHF_2$, $-CF_3$, $-OCH_2F$, - $OCHF_2$, $-OCF_3$, $-CH_2D$, $-CHD_2$, $-CD_3$, $-OCH_2D$, $-OCHD_2$, or $-OCD_3$; and
$R^{B12}$, $R^{B13}$, $R^{31}$, $R^{32}$, $R^{41}$, $R^{42}$ $R^{43}$ $R^{51}$, $R^{52}$ and $R^{53}$ are each independently selected from hydrogen, deuterium, F, Cl, cyano, $-SF_5$, di(methyl)phosphonyl, methyl, ethyl, propyl, isopropyl, tert-butyl, 2-methylpropyl, methoxy, ethoxy, propoxy, tert-butoxy, -S-methyl, -S-ethyl, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, azetidinyl, azacyclopentyl, azacyclohexyl, $-CH_2$-azetidinyl, $-CH_2$-azacyclopentyl, $-CH_2$-azacyclohexyl, oxetanyl, oxacyclopentyl, oxacyclohexyl, - $CH_2$-oxetanyl, $-CH_2$-oxacyclopentyl, $-CH_2$-oxacyclohexyl, thietanyl, thiolanyl, thianyl, $-CH_2$-thietanyl, $-CH_2$-thiolanyl, $-CH_2$-thianyl, -O-cyclopropyl, -O-cyclobutyl, -O-cyclopentyl, -O-cyclohexyl, -O-azetidinyl, -O-azacyclopentyl, or - O-azacyclohexyl, wherein the above-mentioned groups are optionally further substituted with 1, 2, and 3 groups selected from F, Cl, deuterium, cyano, amino, hydroxyl, =O, methyl, ethyl, methoxy, ethoxy, $-CH_2F$, $-CHF_2$, $-CF_3$, $-OCH_2F$, - $OCHF_2$, $-OCF_3$, $-CH_2D$, $-CHD_2$, $-CD_3$, $-OCH_2D$, $-OCHD_2$, or $-OCD_3$.

**10.** The compound, or the stereoisomer, deuterated compound, solvate, prodrug, metabolite, pharmaceutically acceptable salt or eutectic thereof according to any one of claims 1-9, wherein

ring B is selected from the following groups, wherein # represents a connection site of ring B to ring A;

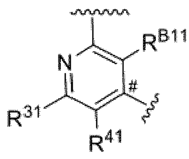
B1-1,
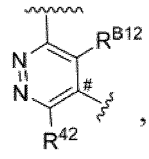
B2-1,
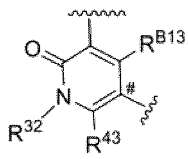
B3-1,

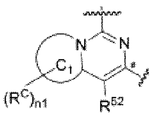
B4-1,
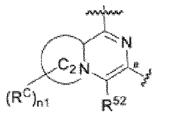
B4-2,
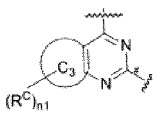
B4-3,
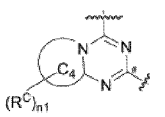
B4-4,
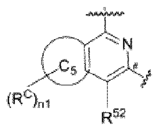
B4-5,

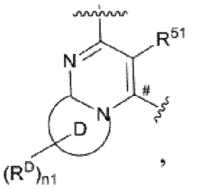
B5-1,
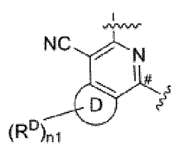
B5-2
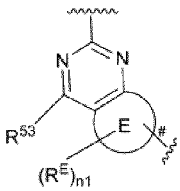
B6-1,
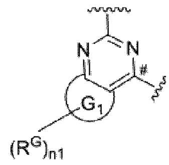
B7-1,
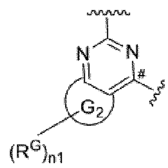
B7-2

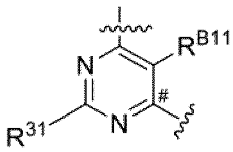
B8
or
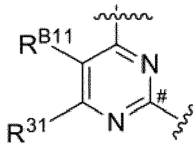
B9
;

rings $C_1$, $C_2$, $C_3$, and $C_4$ are selected from 5-membered heteroaryl or 6-membered heteroaryl;
$C_5$ is selected from 5-membered cycloalkyl, 6-membered cycloalkyl, 5-membered heterocycloalkyl, 6-membered heterocycloalkyl, 5-membered heteroaryl, or 6-membered heteroaryl;
ring D is selected from 5-membered heteroaryl, 6-membered heteroaryl, 5-membered cycloalkyl, or 6-membered cycloalkyl;
ring $G_1$ is selected from phenyl, 5-membered heteroaryl, or 6-membered heteroaryl;
$G_2$ is selected from 5-membered cycloalkyl or 6-membered cycloalkyl;
ring E is selected from 5-membered heterocycloalkyl or 6-membered heterocycloalkyl;
n1 is selected from 0, 1, 2, or 3;
$R^C$, $R^D$, $R^E$, $R^G$, and $R^{B11}$ are each independently selected from deuterium, F, Cl, cyano, $-SF_5$, di($C_{1-2}$ alkyl)phosphonyl, $C_{1-4}$ alkyl, or $C_{1-4}$ alkoxy, wherein the alkyl and alkoxy are optionally further substituted with 1, 2, or 3 groups selected from F, Cl, deuterium, hydroxyl, =O, methyl, ethyl, methoxy, ethoxy, $-CF_3$, $-CHF_2$, $-CH_2F$, $-OCF_3$, $-OCHF_2$, or $-OCH_2F$;
$R^{B12}$, $R^{B13}$, $R^{31}$, $R^{32}$, $R^{41}$, $R^{42}$ $R^{43}$ $R^{51}$, $R^{52}$ and $R^{53}$ are each independently selected from hydrogen, deuterium, F, Cl, cyano, $-SF_5$, di($C_{1-2}$ alkyl)phosphonyl, $C_{1-4}$ alkyl, $C_{1-4}$ alkoxy, $-S-C_{1-2}$ alkyl, $-(CH_2)_r-C_{3-6}$ cycloalkyl, $-(CH_2)_r-C_{4-6}$ heterocycloalkyl, $-O-C_{3-6}$ cycloalkyl, $-O-C_{4-6}$ heterocycloalkyl, 5- to 6-membered heteroaryl, phenyl, $-NHC_{1-3}$ alkyl, or $-N(C_{1-3}$ alkyl$)_2$, wherein the $CH_2$, alkyl, alkoxy, cycloalkyl, heterocycloalkyl, heteroaryl, and aryl are optionally further substituted with 1-5 groups selected from F, Cl, deuterium, hydroxyl, =O, methyl, ethyl, methoxy, ethoxy, $-CF_3$, $-CHF_2$, $-CH_2F$, $-OCF_3$, $-OCHF_2$, or $-OCH_2F$;
provided that: (1) when ring B is selected from B7-1 and B7-2, A-$R^2$ is not

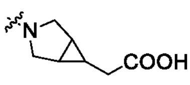 or 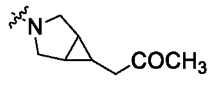;

and

(2) when B is selected from B8 or B9 structure, n is only selected from 1, and $R^1$ is not selected from hydroxyl.

**11.** The compound, or the stereoisomer, deuterated compound, solvate, prodrug, metabolite, pharmaceutically acceptable salt or eutectic thereof according to any one of claims 1-9, wherein

ring B is selected from the following groups:

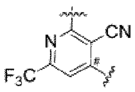, 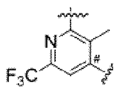, 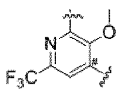, 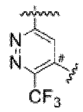, 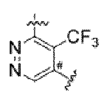, 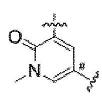, 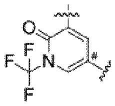, 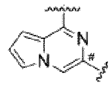,

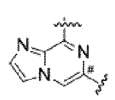, 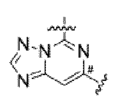, 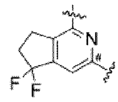, 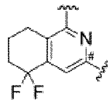, 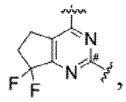, 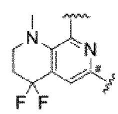, 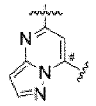, 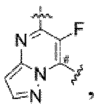,

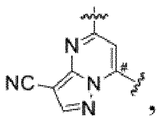, 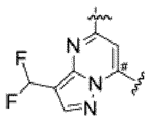, 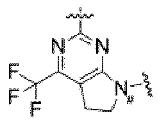 or 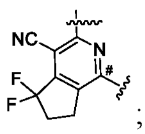;

or

ring B is selected from the following groups:

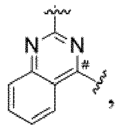, 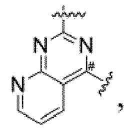, 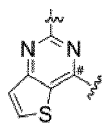, 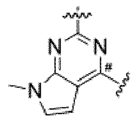, 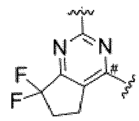, 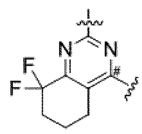,

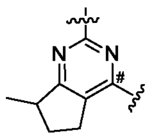, 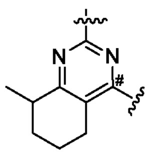, 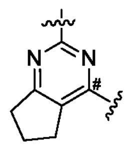, or 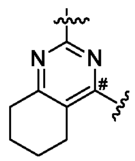,

provided that A-$R^2$ is not

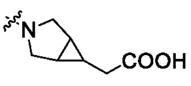 or 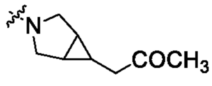;

or

ring B is selected from the following groups:

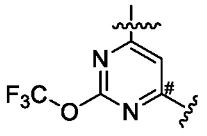 or 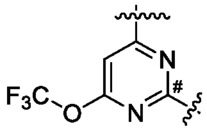 ;

wherein # represents a connection site of ring B to ring A.

**12.** The compound of formula (I-a), or the stereoisomer, deuterated compound, solvate, prodrug, metabolite, pharmaceutically acceptable salt or eutectic thereof according to claim 2,

wherein $R^1$ is D, methyl, ethyl, or propyl, wherein the methyl, ethyl, or propyl is optionally substituted with 1 to 3 groups selected from halogen or deuterium;
$R^{11}$, $R^{12}$, $R^{13}$, and $R^{14}$ are each independently selected from H, deuterium, F, or Cl;
provided that when $R^1$ is methyl, $R^{11}$, $R^{12}$, $R^{13}$, and $R^{14}$ are not simultaneously H;
ring B is selected from the following groups:

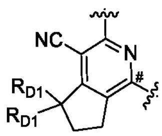 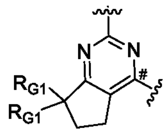 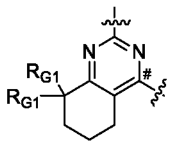 ;

$R_{D1}$ and $R_{G1}$ are each independently selected from D, H, F, Cl, Br, I, methyl, ethyl, or propyl, wherein the methyl, ethyl, or propyl is optionally substituted with 1-3 substituents selected from D, F, Cl, Br, or I;
ring A is selected from the following groups:

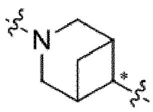 , 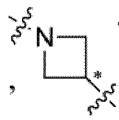 , 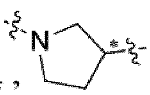 ;

$R^2$ is $-CR^{2a}R^{2b}-COOR^{23}$;
$R^{2a}$ and $R^{2b}$ are each independently selected from hydrogen, deuterium, F, Cl, methyl, or ethyl; and
$R^{23}$ is selected from hydrogen, deuterium, methyl, ethyl, propyl, or tert-butyl.

**13.** The compound, or the stereoisomer, deuterated compound, solvate, prodrug, metabolite, pharmaceutically acceptable salt or eutectic thereof according to claim 12,

wherein $R^1$ is D, methyl, ethyl, or propyl, wherein the methyl, ethyl, or propyl is substituted with 1 to 3 groups selected from halogen or deuterium; or
at least one of $R^{11}$, $R^{12}$, $R^{13}$, and $R^{14}$ is selected from deuterium, F, or Cl.

**14.** The compound, or the stereoisomer, deuterated compound, solvate, prodrug, metabolite, pharmaceutically acceptable salt or eutectic thereof according to claim 1, wherein the compound has a structure selected from:

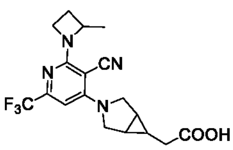 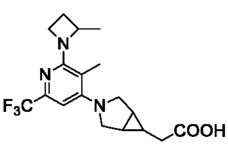 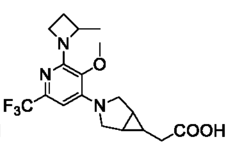 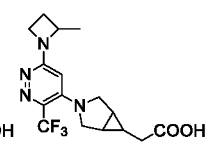 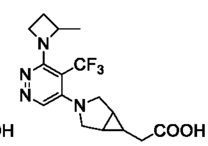

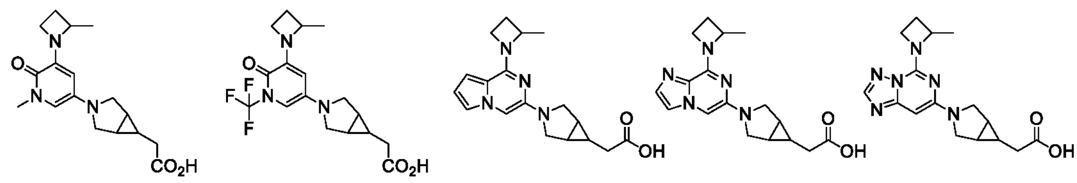

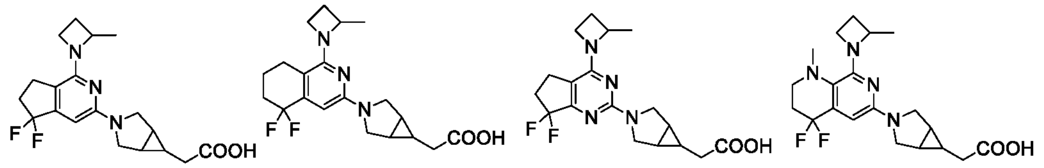

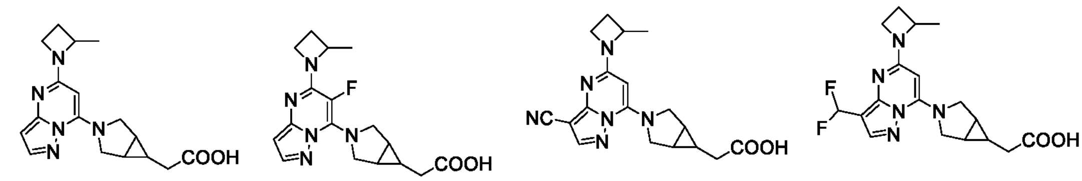

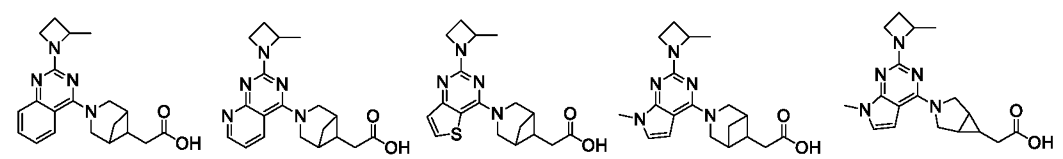

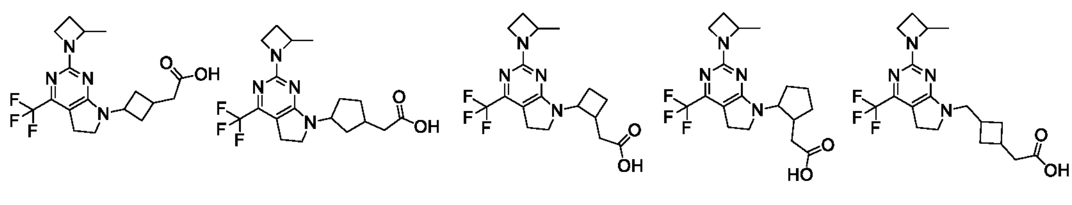

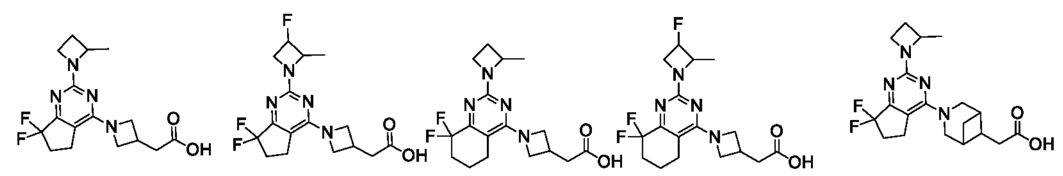

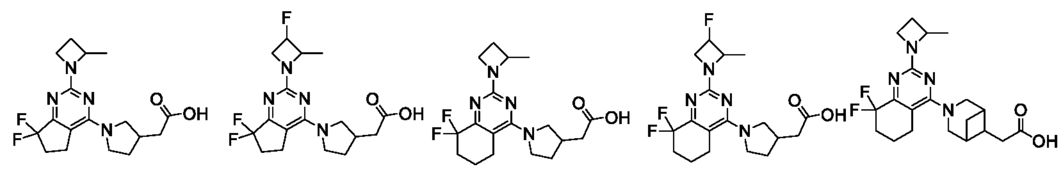

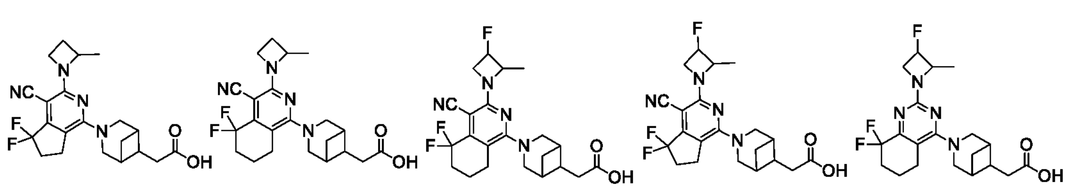

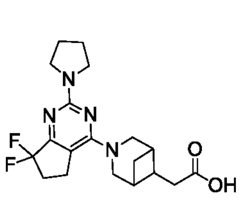 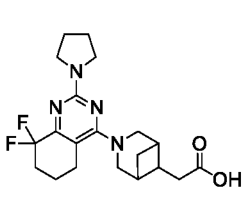 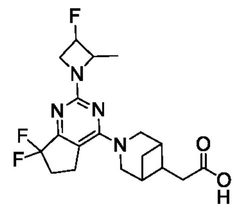

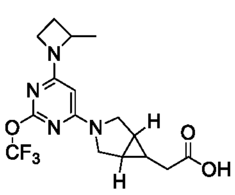 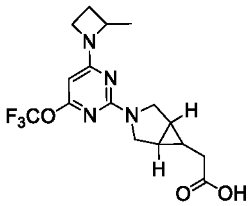 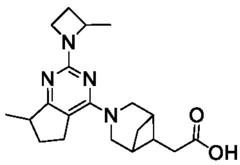 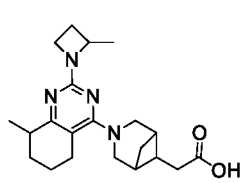

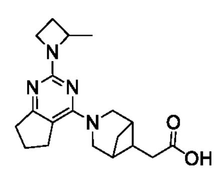 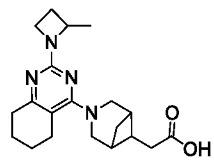 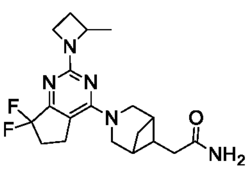 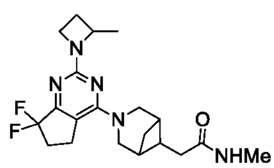

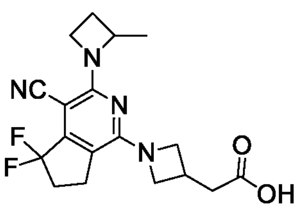 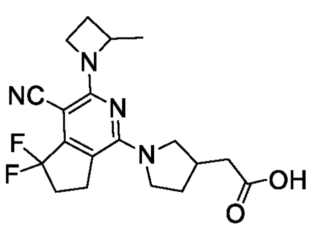 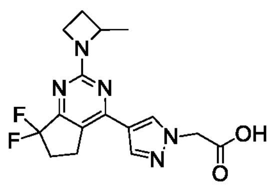

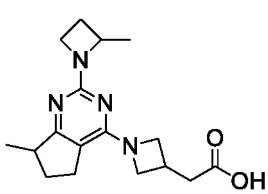 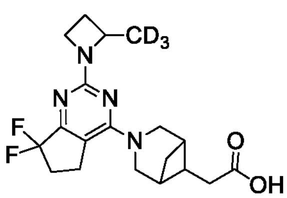 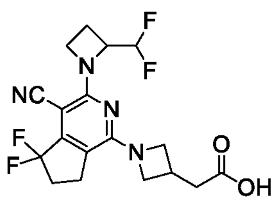

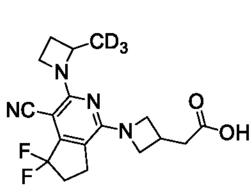 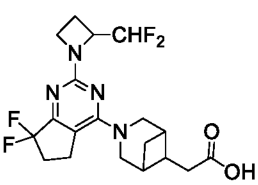 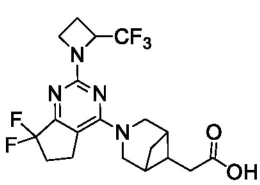 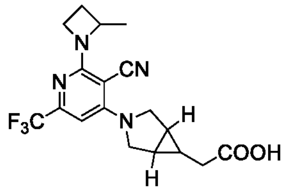

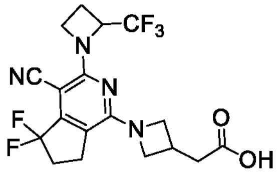 .

**15.** The compound, or the stereoisomer, deuterated compound, solvate, prodrug, metabolite, pharmaceutically acceptable salt or eutectic thereof according to claim 1, wherein the compound has a structure selected from:

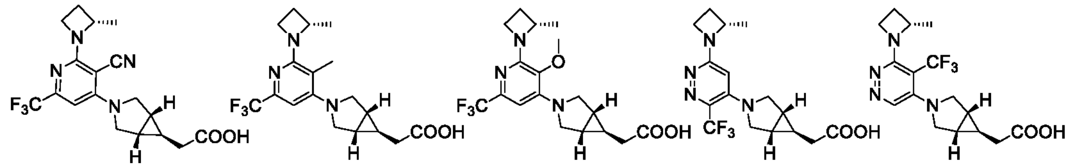

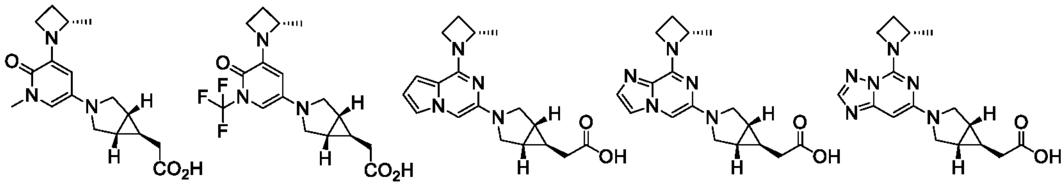

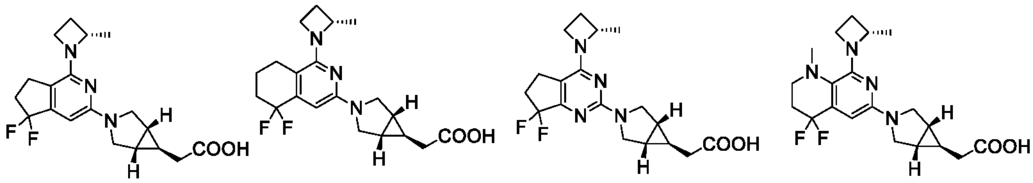

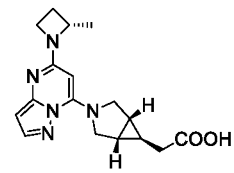

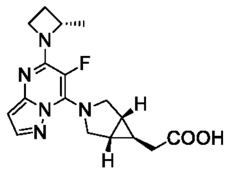

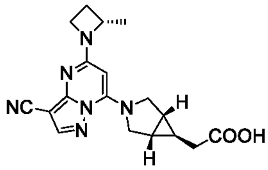

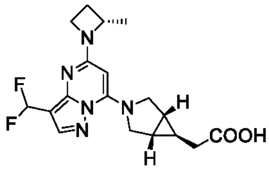

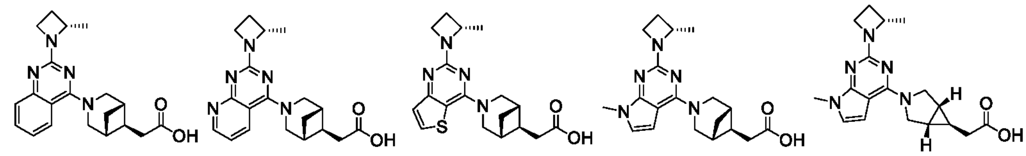

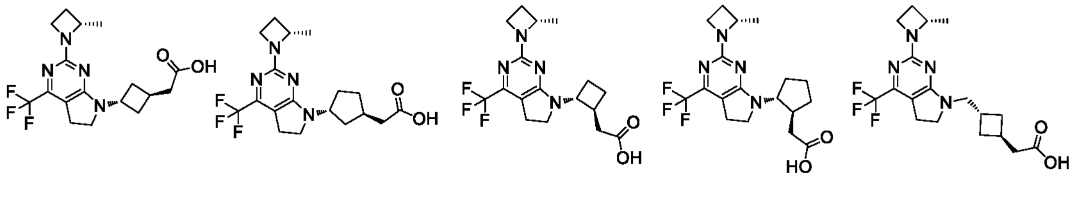

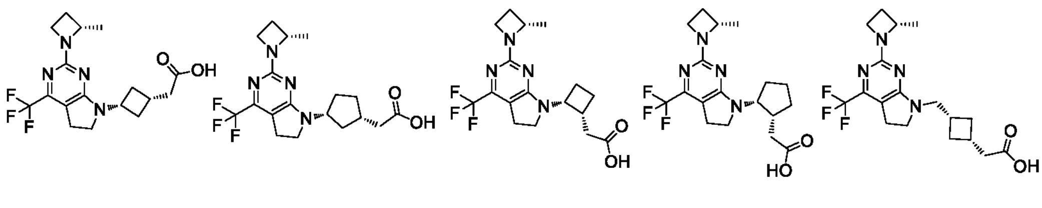

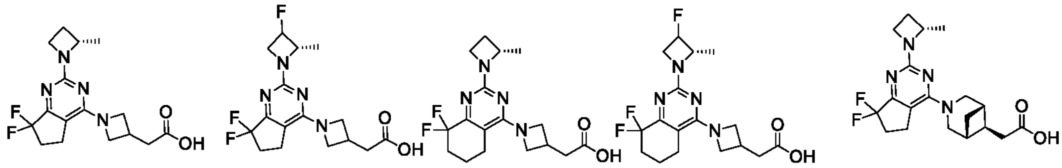

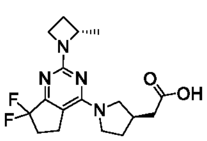
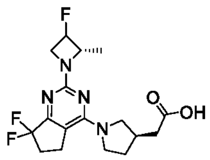
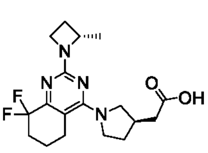
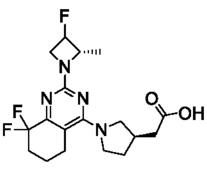
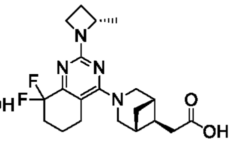

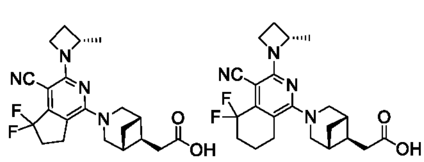
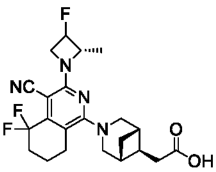
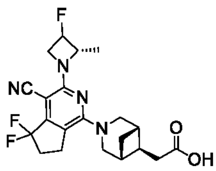
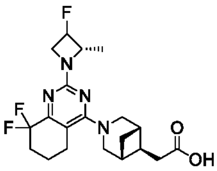

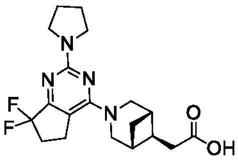
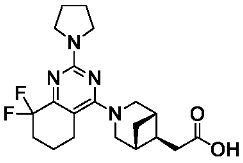
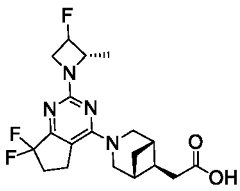

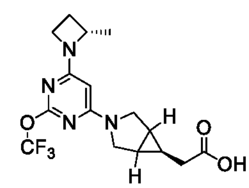
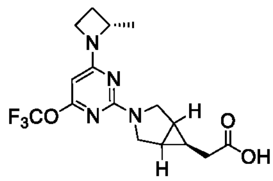

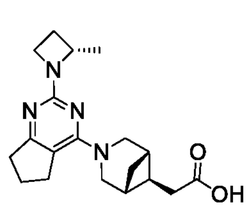
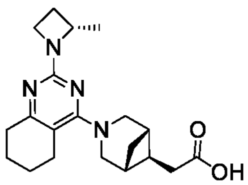
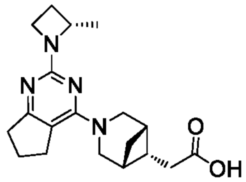
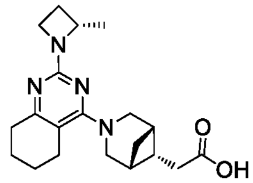

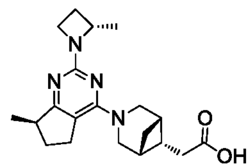
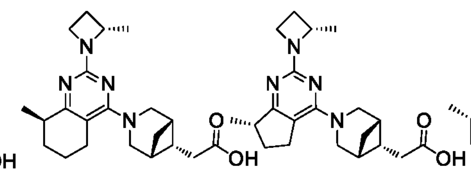
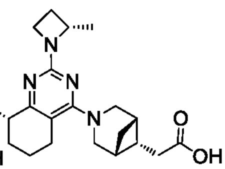

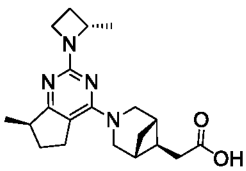
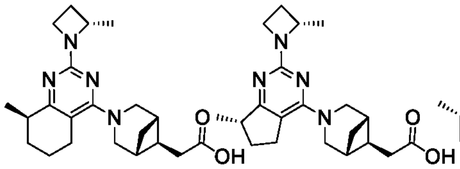
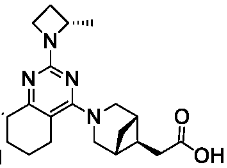

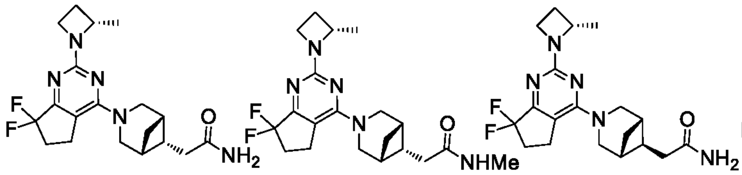
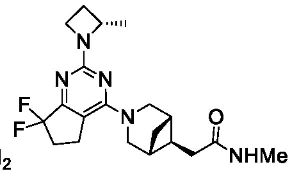

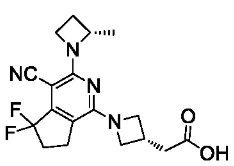 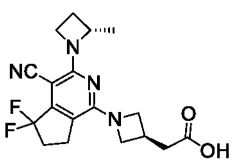 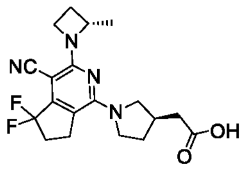 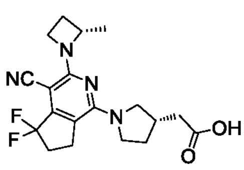

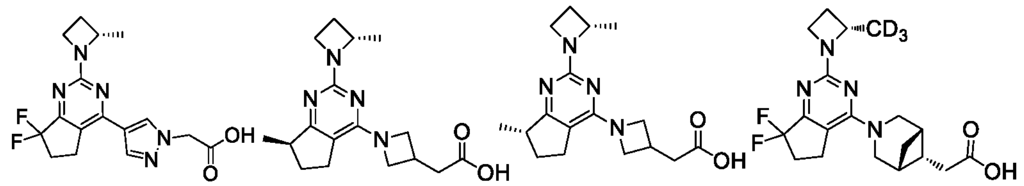

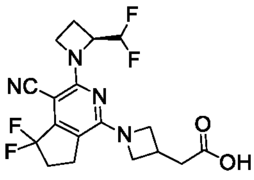 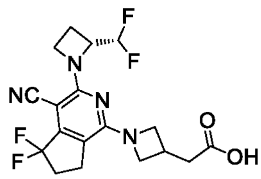 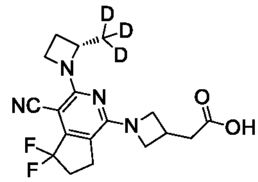 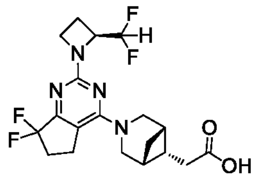

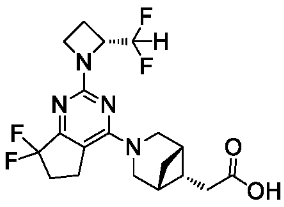 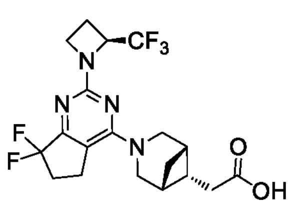 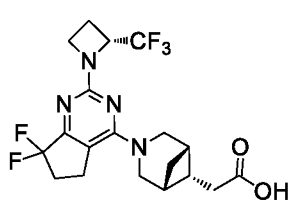

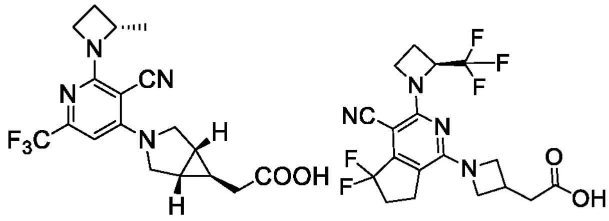 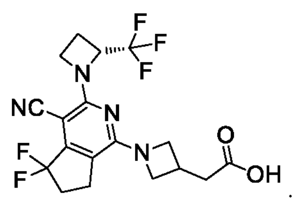.

**16.** A pharmaceutical composition, comprising the compound, or the stereoisomer, deuterated compound, solvate, prodrug, metabolite, pharmaceutically acceptable salt or eutectic thereof according to any one of claims 1-15, and a pharmaceutically acceptable carrier and/or excipient.

**17.** Use of the compound, or the stereoisomer, deuterated compound, solvate, prodrug, metabolite, pharmaceutically acceptable salt or eutectic thereof according to any one of claims 1-15, or the composition according to claim 16 in the preparation of a drug for treating a KHK-mediated disease.

**18.** The use according to claim 17, wherein the KHK-mediated disease is non-alcoholic fatty liver disease.

**INTERNATIONAL SEARCH REPORT**

International application No.
**PCT/CN2021/140067**

**A. CLASSIFICATION OF SUBJECT MATTER**

C07D 401/14(2006.01)i; C07D 401/04(2006.01)i; C07D 403/14(2006.01)i; C07D 403/04(2006.01)i; A61K 31/4439(2006.01)i; A61K 31/506(2006.01)i; A61P 1/16(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

CNTXT, ENTXT, DWPI, CJFD, CNKI, Caplus(STN), Registry(STN): 四川海思科制药有限公司, 李瑶, 王文晶, 陈雷, 张国彪, 张晓波, 胡刚, 王亚军, 王浩东, 唐平明, 余彦, 张晨, 严庞科, 己酮糖激酶, 抑制剂, 杂环, 螺环, 氮, 羧基, ketohexokinase, inhibitor, aza, cyclo, heterocyclic, spiro, nitrogen, carboxyl

**C. DOCUMENTS CONSIDERED TO BE RELEVANT**

| Category* | Citation of document, with indication, where appropriate, of the relevant passages | Relevant to claim No. |
|---|---|---|
| X | CN 108473469 A (PFIZER INC.) 31 August 2018 (2018-08-31)<br>claims 1-24, embodiment 54 | 1-18 |
| X | WO 2020156445 A1 (KBP BIOSCIENCES CO., LTD.) 06 August 2020 (2020-08-06)<br>claims 1-11, embodiments 1-14 | 1-18 |
| X | CN 111978296 A (KBP BIOSCIENCES CO., LTD.) 24 November 2020 (2020-11-24)<br>claims 1-11, and embodiment 1 | 1-18 |
| X | CN 111423420 A (GUANGZHOU BOJI MEDICAL BIOTECHNOLOGY CO., LTD.) 17 July 2020 (2020-07-17)<br>claims 1-10, and embodiment 1 | 1-18 |
| X | WO 2020067735 A1 (LG CHEM, LTD.) 02 April 2020 (2020-04-02)<br>claims 1-10, and embodiments 1-5 | 1-18 |
| X | WO 2020257171 A1 (ELILILLY AND COMPANY) 24 December 2020 (2020-12-24)<br>claims 1-18, embodiments 1-36 | 1-18 |
| A | CN 102939283 A (JANSSEN PHARMACEUTICA NV) 20 February 2013 (2013-02-20)<br>entire document | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2022** | **22 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/140067**

**C. DOCUMENTS CONSIDERED TO BE RELEVANT**

| Category* | Citation of document, with indication, where appropriate, of the relevant passages | Relevant to claim No. |
|---|---|---|
| PX | CN 113754640 A (KBP BIOSCIENCES CO., LTD.) 07 December 2021 (2021-12-07)<br>claims 1-8, embodiments 1-2 | 1-18 |
| PX | WO 2021129817 A1 (MEDSHINE DISCOVERY INC.) 01 July 2021 (2021-07-01)<br>claims 1-15, embodiments 1-41 | 1-18 |
| PX | WO 2021161023 A1 (INORBIT THERAPEUTICS AB) 19 August 2021 (2021-08-19)<br>claims 1-16, embodiments 1-6 | 1-18 |
| PX | WO 2021162943 A1 (ELILILLY AND COMPANY) 19 August 2021 (2021-08-19)<br>claims 1-14, and embodiments 1-3 | 1-18 |
| PX | WO 2021215765 A1 (LG CHEM, LTD.) 28 October 2021 (2021-10-28)<br>claims 1-9, and embodiments 1-12 | 1-18 |

Form PCT/ISA/210 (second sheet) (January 2015)

**INTERNATIONAL SEARCH REPORT**
**Information on patent family members**

International application No.
**PCT/CN2021/140067**

| Patent document cited in search report | Publication date (day/month/year) | Patent family member(s) | Publication date (day/month/year) |
|---|---|---|---|
| CN 108473469 A | 31 August 2018 | EP 3397631 A1 | 07 November 2018 |
| | | EP 3397631 B1 | 07 April 2021 |
| | | DK 3397631 T3 | 25 May 2021 |
| | | PL 3397631 T3 | 20 September 2021 |
| | | SG CN 11201804363U A | 30 July 2018 |
| | | NI 201800072 A | 22 November 2018 |
| | | ZA 201803449 B | 27 February 2019 |
| | | CO 2018006714 A2 | 10 July 2018 |
| | | CU 20180046 A7 | 05 September 2018 |
| | | CU 24540 B1 | 06 August 2021 |
| | | WO 2017115205 A1 | 06 July 2017 |
| | | AU 2016380920 A1 | 14 June 2018 |
| | | AU 2016380920 B2 | 31 October 2019 |
| | | US 2020377482 A1 | 03 December 2020 |
| | | US 10988463 B2 | 27 April 2021 |
| | | US 2021309644 A1 | 07 October 2021 |
| | | MX 2018007755 A | 09 November 2018 |
| | | RS 61896 B1 | 30 June 2021 |
| | | IL 260330 D0 | 30 August 2018 |
| | | IL 260330 A | 29 July 2021 |
| | | CL 2018001667 A1 | 03 August 2018 |
| | | CA 2952466 A1 | 29 June 2017 |
| | | TN 2018000198 A1 | 04 October 2019 |
| | | US 2019106412 A1 | 11 April 2019 |
| | | RU 2696269 C1 | 01 August 2019 |
| | | PT 3397631 T | 28 May 2021 |
| | | UY 37051 A | 31 July 2017 |
| | | SV 2018005709 A | 10 September 2018 |
| | | LT 3397631 T | 10 June 2021 |
| | | EC SP18048517 A | 31 July 2018 |
| | | KR 20180083427 A | 20 July 2018 |
| | | KR 102073048 B1 | 04 February 2020 |
| | | MD 3397631 T2 | 31 August 2021 |
| | | PE 20181289 A1 | 07 August 2018 |
| | | SI 3397631 T1 | 31 January 2022 |
| | | JP 2019500383 A | 10 January 2019 |
| | | JP 6503515 B2 | 17 April 2019 |
| | | HK 1259073 A1 | 22 November 2019 |
| | | GE P20207147 B | 10 September 2020 |
| | | US 2018037575 A1 | 08 February 2018 |
| | | US 10174007 B2 | 08 January 2019 |
| | | HU E054380 T2 | 28 September 2021 |
| | | PH 12018501375 A1 | 11 February 2019 |
| | | BR 112018012047 A2 | 04 December 2018 |
| | | MA 43518 A | 07 November 2018 |
| | | MA 43518 B1 | 31 May 2021 |
| | | US 2017183328 A1 | 29 June 2017 |
| | | US 9809579 B2 | 07 November 2017 |
| | | ES 2871251 T3 | 28 October 2021 |

Form PCT/ISA/210 (patent family annex) (January 2015)

**INTERNATIONAL SEARCH REPORT**
**Information on patent family members**

International application No.
**PCT/CN2021/140067**

| Patent document cited in search report | Publication date (day/month/year) | Patent family member(s) | Publication date (day/month/year) |
|---|---|---|---|
| WO 2020156445 A1 | 06 August 2020 | CA 3127130 A1 | 06 August 2020 |
| | | SG 11202107389 S A | 30 August 2021 |
| | | EP 3919484 A1 | 08 December 2021 |
| | | EP 3919484 A4 | 08 December 2021 |
| | | AU 2020214064 A1 | 05 August 2021 |
| | | KR 20210120080 A | 06 October 2021 |
| | | CN 113412260 A | 17 September 2021 |
| CN 111978296 A | 24 November 2020 | None | |
| CN 111423420 A | 17 July 2020 | WO 2021217734 A1 | 04 November 2021 |
| | | CN 113149968 A | 23 July 2021 |
| WO 2020067735 A1 | 02 April 2020 | CN 112789271 A | 11 May 2021 |
| | | US 2022033381 A1 | 03 February 2022 |
| | | KR 20200035720 A | 06 April 2020 |
| | | JP 2022502444 A | 11 January 2022 |
| | | EP 3842423 A1 | 30 June 2021 |
| | | EP 3842423 A4 | 18 August 2021 |
| WO 2020257171 A1 | 24 December 2020 | BR 112021023153 A2 | 04 January 2022 |
| | | US 2020392118 A1 | 17 December 2020 |
| | | US 11124500 B2 | 21 September 2021 |
| | | IL 288350 D0 | 01 January 2022 |
| | | KR 20220008882 A | 21 January 2022 |
| | | CA 3143490 A1 | 24 December 2020 |
| | | AU 2020295979 A1 | 09 December 2021 |
| | | TW 202112768 A | 01 April 2021 |
| | | US 2022017496 A1 | 20 January 2022 |
| | | JP 2021525793 A | 27 September 2021 |
| | | JP 6986653 B2 | 22 December 2021 |
| CN 102939283 A | 20 February 2013 | CN 102939283 B | 03 June 2015 |
| | | JP 2013525370 A | 20 June 2013 |
| | | WO 2011133750 A1 | 27 October 2011 |
| | | US 2014336170 A1 | 13 November 2014 |
| | | US 9771375 B2 | 26 September 2017 |
| | | US 2011263559 A1 | 27 October 2011 |
| | | US 8822447 B2 | 02 September 2014 |
| | | EP 2560962 A1 | 27 February 2013 |
| | | EP 2560962 B1 | 20 May 2015 |
| | | ES 2545811 T3 | 16 September 2015 |
| | | AU 2011242711 A1 | 08 November 2012 |
| | | AU 2011242711 B2 | 12 November 2015 |
| | | AU 2011242711 C1 | 21 April 2016 |
| CN 113754640 A | 07 December 2021 | None | |
| WO 2021129817 A1 | 01 July 2021 | None | |
| WO 2021161023 A1 | 19 August 2021 | WO 2021161023 A8 | 20 January 2022 |
| | | GB 202001856 D0 | 25 March 2020 |
| WO 2021162943 A1 | 19 August 2021 | None | |
| WO 2021215765 A1 | 28 October 2021 | KR 20210129607 A | 28 October 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017115205 A
- WO 2020156445 A1
- WO 2020156445 A
- WO 2017115205 A1


### Non-patent literature cited in the description


- **ISHIMOTO ; LANASPA et al.** *PNAS,* 2012, vol. 109, 4320-4325
- **DIGGLE CP et al.** *J HistochemCytochem,* 2009, vol. 57, 763-774
- **BOUTELDJA N et al.** *J. Inherit. Metab. Dis.,* April 2010, vol. 33 (2), 105-12
- **TOLAN, DR.** *HumMutat.,* 1995, vol. 6 (3), 210-8, http://www.omim.org/entry/229600
- **ALI M et al.** *J.Med.Genet.,* May 1998, vol. 35 (5), 353-65
- **LANASPA, M.A. et al.** *NatureComm.,* 2013, vol. 4, 2434
- *ACS Med. Chem. Lett.,* 2011, vol. 2 (5), 38-543
- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002
- *Journal of Medicinal Chemistry,* 1994, vol. 37 (24), 4195-4210